(19) Europäisches Patentamt
European Patent Office
Office européen des brevets

(11) EP 1 758 935 B1

(12) **EUROPEAN PATENT SPECIFICATION**

(45) Date of publication and mention of the grant of the patent:
**29.04.2009 Bulletin 2009/18**

(21) Application number: **05718025.9**

(22) Date of filing: **15.03.2005**

(51) Int Cl.:
***C07K 14/725*** *(2006.01)* ***C12N 15/12*** *(2006.01)*
***C12N 15/62*** *(2006.01)* ***A61K 38/17*** *(2006.01)*
***A61P 35/00*** *(2006.01)*

(86) International application number:
**PCT/GB2005/000970**

(87) International publication number:
**WO 2005/116075 (08.12.2005 Gazette 2005/49)**

(54) **HIGH AFFINITY TELOMERASE T CELL RECEPTORS**

HOCHAFFINE TELOMERASE-T-ZELLEN-REZEPTOREN

RECEPTEURS DE LYMPHOCYTES T PRESENTANT UNE GRANDE AFFINITE AVEC LA TELOMERASE

(84) Designated Contracting States:
**AT BE BG CH CY CZ DE DK EE ES FI FR GB GR HU IE IS IT LI LT LU MC NL PL PT RO SE SI SK TR**

(30) Priority: **26.05.2004 GB 0411772**
**03.09.2004 GB 0419644**

(43) Date of publication of application:
**07.03.2007 Bulletin 2007/10**

(73) Proprietor: **Immunocore Ltd.**
**Abingdon**
**Oxford**
**OX14 4RX (GB)**

(72) Inventors:
- **JAKOBSEN, Bent Karsten,**
  **Avidex Ltd.**
  **Abingdon OX14 4RX (GB)**
- **LI, Yi,**
  **Avidex Ltd.**
  **Abingdon OX14 4RX (GB)**

(74) Representative: **Walls, Alan James**
**MediGene Limited**
**57c Milton Park**
**Abingdon**
**Oxfordshire OX14 4RX (GB)**

(56) References cited:
**WO-A-03/020763** **WO-A-03/070752**
**WO-A-20/04044004**

- **HOLLER P D ET AL: "In vitro evolution of a T cell receptor with high affinity for peptide/MHC" PROCEEDINGS OF THE NATIONAL ACADEMY OF SCIENCES OF USA, NATIONAL ACADEMY OF SCIENCE. WASHINGTON, US, vol. 97, no. 10, 9 May 2000 (2000-05-09), pages 5387-5392, XP002217232 ISSN: 0027-8424**
- **WILLEMSEN RALPH A ET AL: "Genetic engineering of T cell specificity for immunotherapy of cancer." HUMAN IMMUNOLOGY. JAN 2003, vol. 64, no. 1, January 2003 (2003-01), pages 56-68, XP002336794 ISSN: 0198-8859**
- **LI YI ET AL: "Directed evolution of human T-cell receptors with picomolar affinities by phage display." NATURE BIOTECHNOLOGY. MAR 2005, vol. 23, no. 3, March 2005 (2005-03), pages 349-354, XP002336795 ISSN: 1087-0156**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 758 935 B1

## Description

The present invention relates to T cell receptors (TCRs) having the property of binding to ILAKFLHWL-HLA-A*0201 and comprising at least one TCR α chain variable domain and/or at least one TCR β chain variable domain CHARACTERISED IN THAT said TCR is mutated relative to the native ILA TCR α chain variable domain shown in SEQ ID No: 1 and/or β chain variable domain shown in SEQ ID NO: 2 in at least one complementarity determining region, and has a $K_D$ for the said ILAKFLHWL-HLA-A*0201 complex of less than or equal to 1μM and/or has an off-rate ($k_{off}$) for the ILAKFLHWL-HLA-A*0201 complex of $1x10^{-3}$ $s^{-1}$ or slower.

## Background to the Invention

The ILAKFLHWL peptide is derived from the catalytic sub-unit of the Telomerase protein. (See Meyerson et al., (1997) Cell 90: 785-795 and Nakamura et al., (1997) Science 277: 955-9) These studies describe the near-simultaneous discovery of the DNA and deduced amino acid sequence of the Telomerase catalytic subunit from database sequences. Both studies note that Telomerase catalytic sub-unit activity is associated with human cancer. The Class I HLA molecules of these cancerous cells present peptides from this protein, including ILAKFLHWL. Therefore, the ILAKFLHWL-HLA-A2 complex provides a cancer marker that the TCRs of the invention can target, for example for the purpose of delivering cytotoxic agents to the cancer cells. However, for that purpose it would be desirable if the TCR had a higher affinity and/or a slower off-rate for the peptide-HLA complex than native TCRs specific for that complex.

A previous study (WO 03/070752) describes the production of a soluble antibody Fab fragment "4A9" which binds to the TLAKFLHWL-HLA-A2 complex with an affinity ($K_D$) of 5nM. Holler et al., (2000) PNAS USA 97 (10): 5387-5392 provides details of a study which utilised yeast cell surface display to increase the affinity of a murine TCR 100-fold.

## Brief Description of the Invention

This invention makes available for the first time TCRs having high affinity ($K_D$) of the interaction less than or equal to 1 μM, and/or a slower off-rate ($k_{off}$) of $1x10^{-3}$ $S^{-1}$ or slower, for the ILAKFLHWL-HLA-A*0201 complex. Such TCRs are useful, either alone or associated with a therapeutic agent, for targeting cancer cells presenting that complex

## Detailed Description of the Invention

The present invention provides T-cell receptors (TCRs) having the property of binding to ILAKFLHWL-HLA-A*0201 and comprising at least one TCR α chain variable domain and/or at least one TCR β chain variable domain CHARACTERISED IN THAT said TCR is mutated relative to the native ILA TCR a chain variable domain shown in SEQ ID No: 1 and/or β chain variable domain shown in SEQ ID NO: 2 in at least one complementarity determining region, and has a $K_D$ for the said ILAKFLHWL-HLA-A*0201 complex of less than or equal to 1μM and/or has an off-rate ($ko_{ff}$) for the ILAKFLHWL-HLA-A*0201 complex of $1x10^{-3}$ $s^{-1}$ or slower.

The $K_D$ and/or ($ko_{ff}$) measurement can be made by any appropriate method. In a preferred embodiment these measurements are made using the Surface Plasmon Resonance (Biacore) method of Example 5.

For comparison a native interaction of a disulfide-linked soluble variant of the native ILA TCR (see SEQ ID NO: 9 for TCR a chain and SEQ ID NO: 10 for TCR β chain) and the ILAKFLHWL-HLA-A*0201 complex has a $K_D$ of approximately 25μM, an off-rate ($k_{off}$) of 1.1 x $10^{-1}$ $S^{-1}$ and a half-life of 0.18 minutes as measured by the Biacore-base method of Example 5.

The native ILA TCR has the following Valpha chain and Vbeta chain gene usage:

| | |
|---|---|
| Alpha chain - | TRAV22 |
| Beta chain: - | TRBV 6.5 |

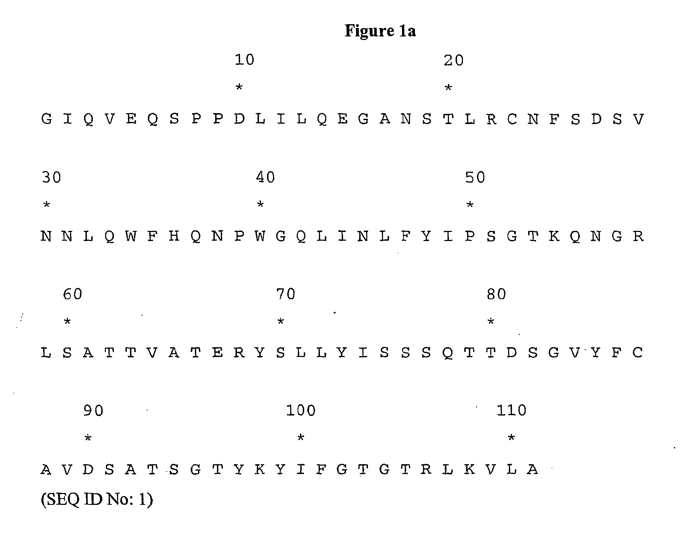

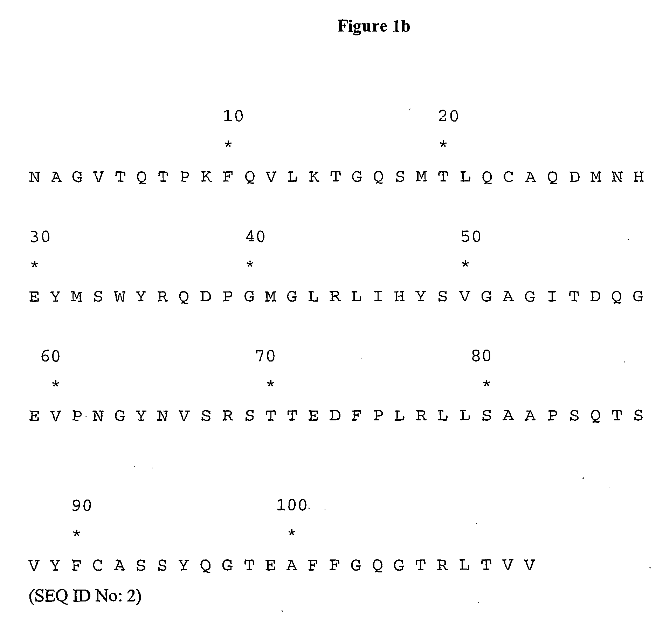

The TLA TCR can be used as a template into which the various mutations that cause high affinity and/or a slow off-rate for the interaction between TCRs of the invention and the ILAKFLHWL-HLA-A*0201 complex can be introduced. Further embodiments of the inventions include TCRs which are mutated relative to the native ILA TCR α chain variable domain (see Figure 1a and SBQ ID No: 1) and/or β chain variable domain (see Figure 1b and SEQ ID NO: 2) in at least one variable domain framework region thereof. It is also contemplated that other hypervariable regions in the variable domains of the TCRs of the invention, such as the hypervariable 4 (HV4) regions, may be mutated so as to produce a high affinity mutant.

Native TCRs exist in heterodimeric αβ or γδ forms. However, recombinant TCRs consisting of a single TCR α or TCR β chain have previously been shown to bind to peptide MHC molecules. In the case where the product of the present invention comprises only a TCR α chain or a TCR β chain, such products are treated as TCRs for the purposes of the invention notwithstanding that the more normal usage of the term TCR relates to heterodimers of α and β chains or single chain constructs comprising α and β portions, especially α and β variable regions. In addition, the term TCR as used herein is to be understood as including αα and ββ homodimers.

In one embodiment the TCR of the invention comprise both an α chain variable domain and an TCR β chain variable domain.

As will be obvious to those skilled in the art the mutation(s) in the TCR α chain sequence and/or TCR β chain sequence may be one or more of substitution(s), deletion(s) or insertion(s). These mutations can be carried out using any appropriate method including, but not limited to, those based on polymerase chain reaction (PCR), restriction enzyme-based cloning, or ligation independent cloning (LIC) procedures. These methods are detailed in many of the standard molecular biology texts. For further details regarding polymerase chain reaction (PCR) mutagenesis and restriction enzyme-based cloning see (Sambrook & Russell, (2001) Molecular Cloning - A Laboratory Manual (3rd Ed.) CSHL Press) Further information on LIC procedures can be found in (Rashtchian, (1995) Curr Opin Biotechnol 6 (1): 30-6)

It should be noted that any αβ TCR that comprises similar Valpha and Vbeta gene usage and therefore amino acid sequence to that of the ILA TCR could make a convenient template TCR. It would then be possible to introduce into the DNA encoding one or both of the variable domains of the template αβ TCR the changes required to produce the mutated high affinity TCRs of the invention. As will be obvious to those skilled in the art, the necessary mutations could be introduced by a number of methods, for example site-directed mutagenesis.

The TCRs of the invention include those in which one or more of the TCR alpha chain variable domain amino acids corresponding to those listed below are mutated relative to the amino acid occurring at these positions in the sequence provided for the native ILA TCR alpha chain variable domain in Figure 1a and SEQ ID No: 1.

Unless stated to the contrary, the TCR amino acid sequences herein are generally provided without an N-terminal methionine (Met or M) residue. As will be known to those skilled in the art this residue is often added during the production of recombinant proteins. As will also be known to those skilled in the art it may be possible to truncate the sequences provided at the C-terminus and/or N-terminus thereof, by 1, 2, 3, 4, 5 or more residues, without substantially affecting the pMHC binding characteristics of the TCR, all such trivial variants are encompassed by the present invention.

As used herein the term "variable domain" is understood to encompass all amino acids of a given TCR which are not included within the constant domain as encoded by the TRAC gene for TCR α chains and either the TRBC1 or TRBC2 for TCR β chains. (T cell receptor Factsbook, (2001) LeFranc and LeFranc, Academic Press, ISBN 0-12-441352-8)

Embodiments of the invention include mutated TCRs which comprise mutation of one or more of alpha chain variable domain amino acids corresponding to 94S, 95G, 96T, 97Y and 98K for example the amino acids:

94A/Q/T/G/R/M/H/K//E/N or L
95L/P/I/V or M
96P or M
97F
98G or A

The numbering used is the same as that shown in Figure 1 a and SEQ ID No: 1.

Embodiments of the invention also include TCRs which comprise mutation of one or more of the TCR beta chain variable domain amino acids corresponding to those listed below, are relative to the amino acid occurring at these positions in the sequence provided for the native ILA TCR alpha chain variable domain of the native ILA TCR beta chain in Figure 1b and SEQ ID No: 2. The amino acids referred to which may be mutated are 19M, 50V, 51G, 52A, 53G, 541, 92A, 93S, 94S and 95Y, for example:

19V
50I/L or R
51H/C or W
52P/W/E or V
53E/S or F
54Y/V/C/E or D
92G
93I/L/ or V
94Q
95P

The lumbering used is the same as that shown in Figure 1b and SEQ ID No: 2.

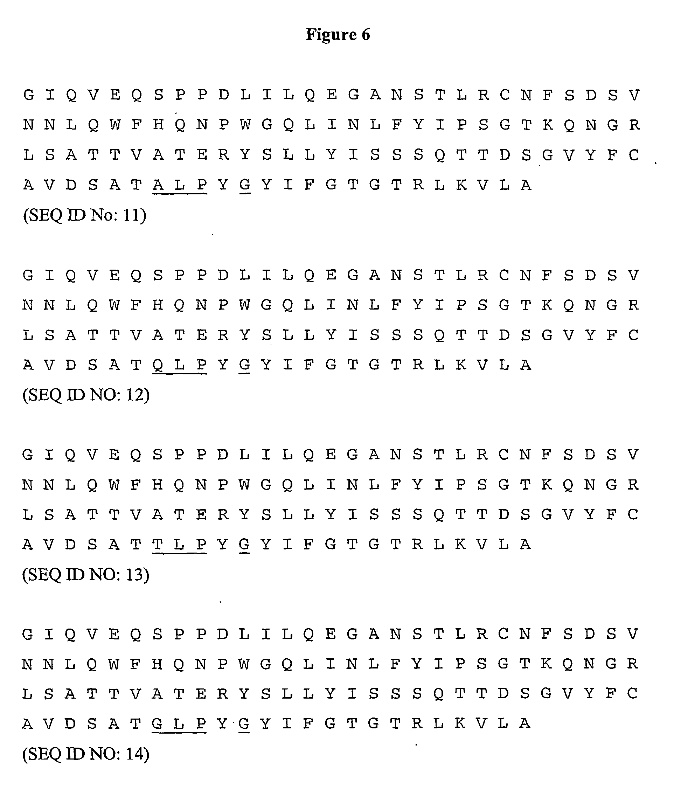

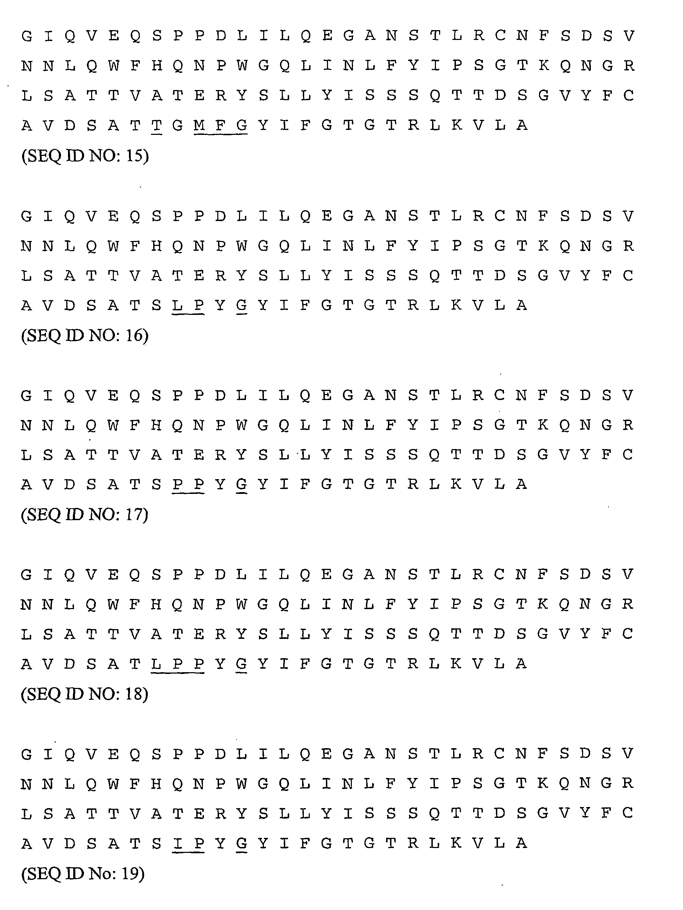

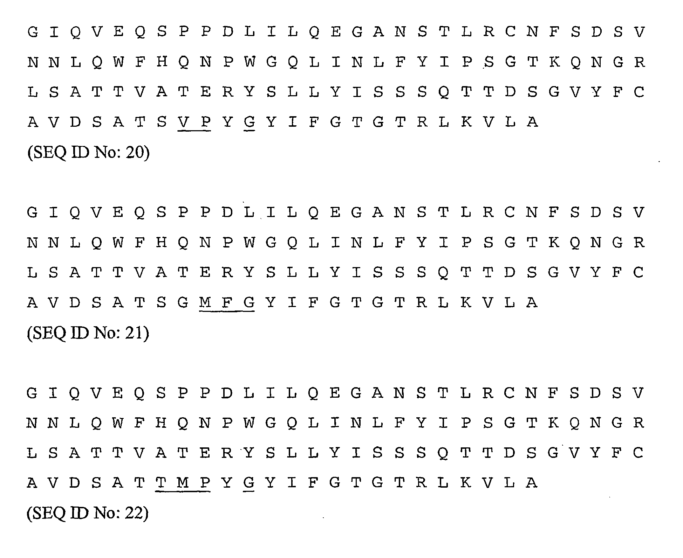

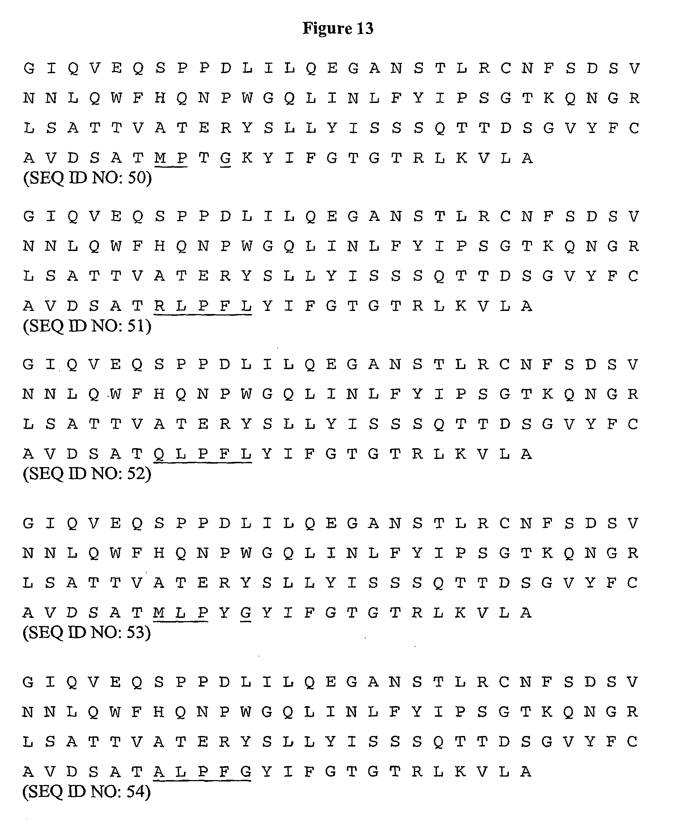

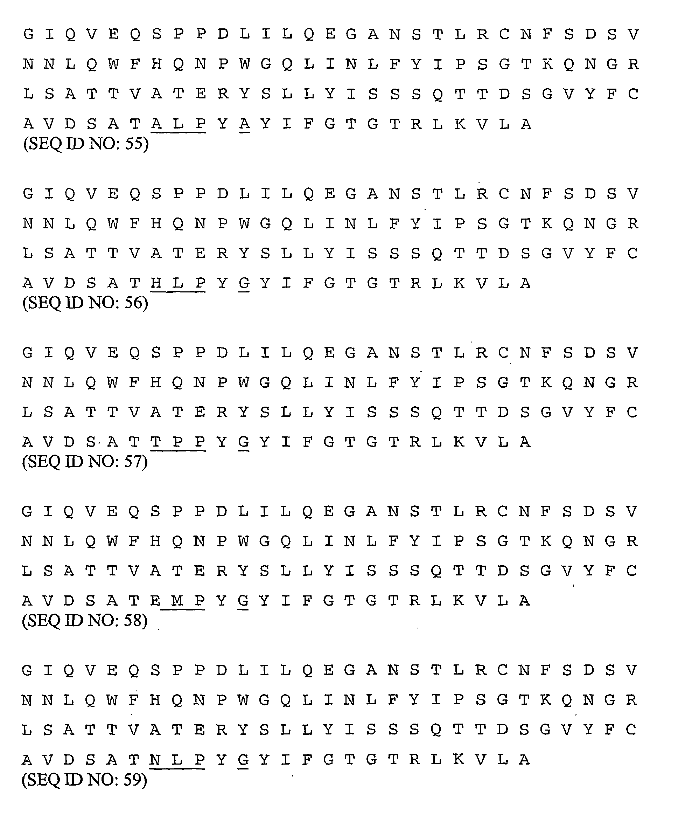

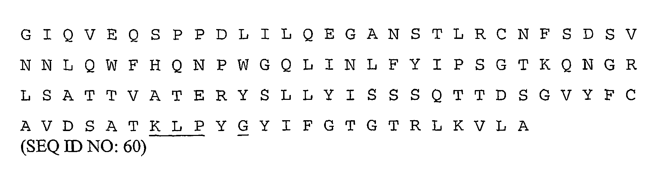

Preferred embodiments of the invention are provided by TCRs comprising one of the mutated alpha chain variable domain amino acid sequences shown in Figures 6 or 13 (SEQ ID Nos: 11 to 22 or 50 to 60). Phenotypically silent variants of such TCRs also form part of this invention.

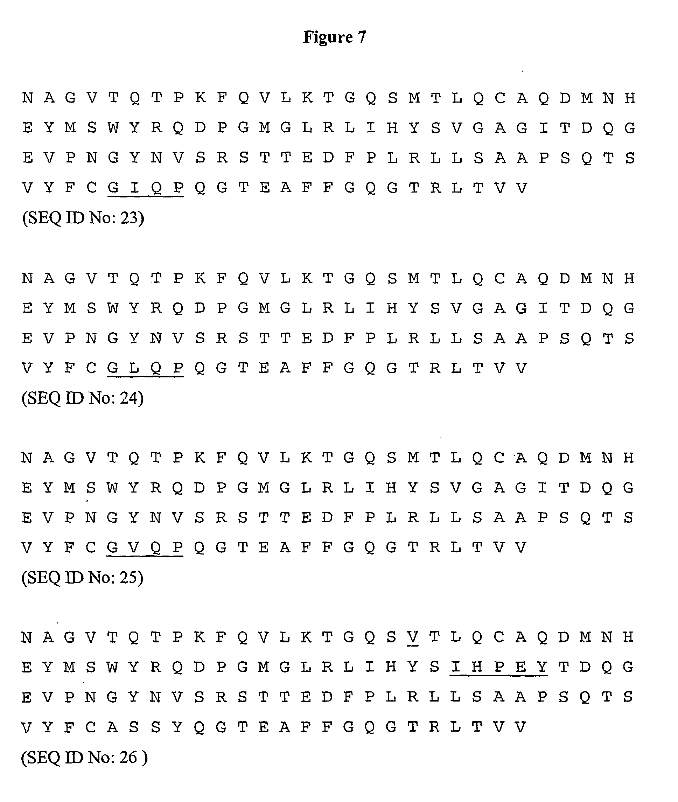

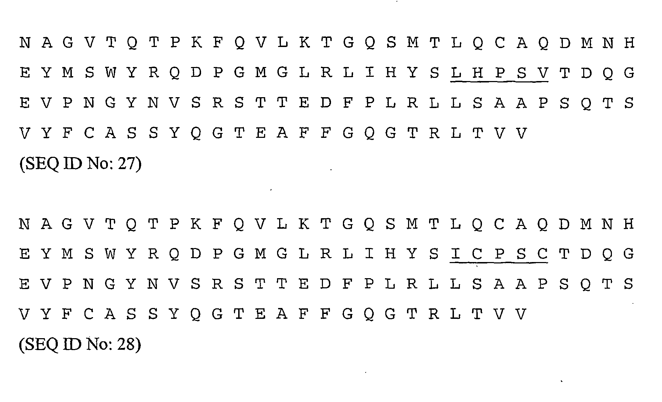

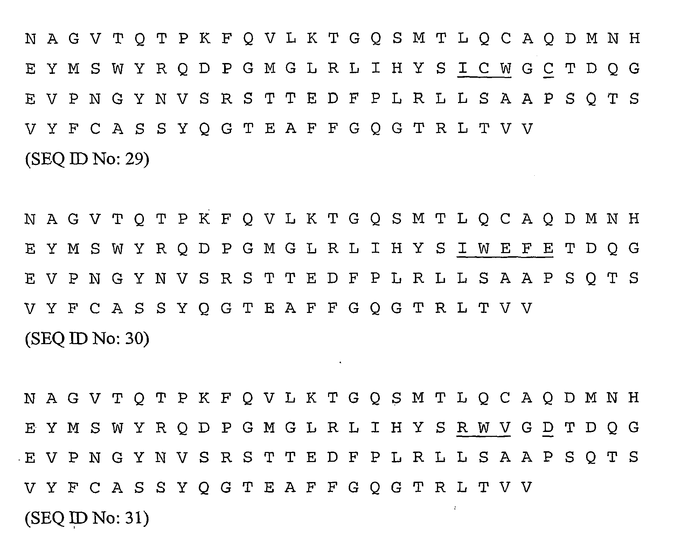

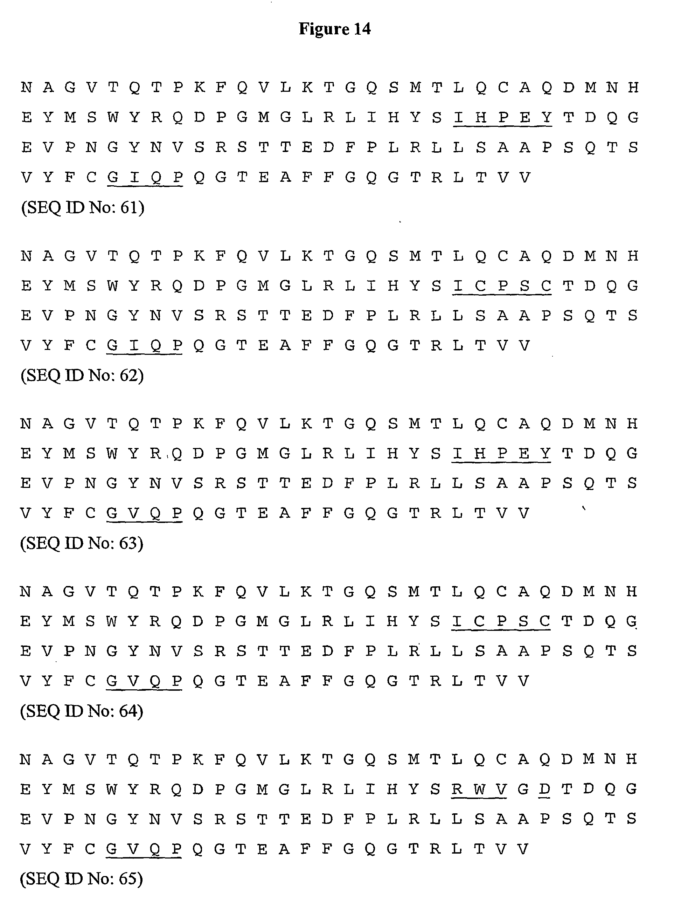

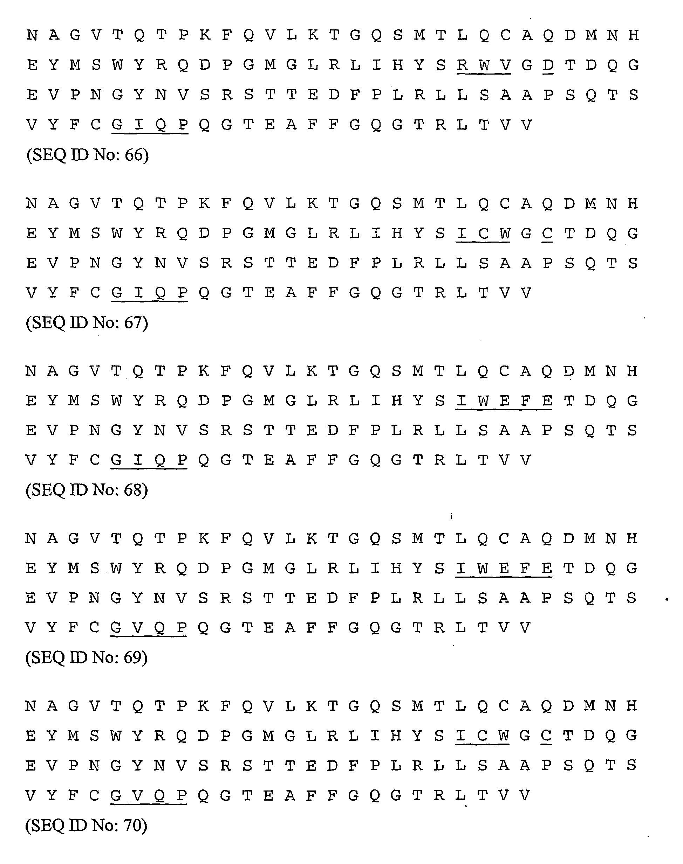

Additional preferred embodiments of the invention are provided by TCRs comprising one of the mutated beta chain variable domain amino acid sequences shown in Figures 7 or 14. (SEQ ID Nos: 23 to 31 or 61 to 70). Phenotypically silent variants of such TCRs also form part of this invention.

Native TCRs exist in heterodimeric αβ or γδ formes. However, recombinant TCRs consisting of αα or ββ homodimers have previously been shown to bind to peptide MHC molecules. Therefore, one embodiment of the invention is provided by TCR αα or TCR ββ homodimers.

Further preferred embodiments are provided by TCRs of the invention comprising the alpha chain variable domain amino acid sequence and the beta chain variable domain amino acid sequence combinations listed below, phenotypically silent variants of such TCRs also form part of this invention:

| Alpha chain variable domain sequence, SEQ ID NO: | Beta chain variable domain sequence, SEQ ID NO: |
|---|---|
| 1 | 23 |
| 1 | 24 |
| 1 | 25 |
| 1 | 26 |
| 1 | 27 |
| 1 | 28 |
| 1 | 29 |
| 1 | 30 |
| 1 | 31 |
| 11 | 2 |
| 12 | 2 |
| 13 | 2 |
| 14 | 2 |
| 15 | 2 |
| 16 | 2 |
| 17 | 2 |
| 18 | 2 |
| 19 | 2 |
| 20 | 2 |
| 21 | 2 |
| 22 | 2 |
| 50 | 2 |
| 51 | 2 |
| 52 | 2 |
| 53 | 2 |
| 54 | 2 |
| 55 | 2 |
| 56 | 2 |
| 57 | 2 |

(continued)

| Alpha chain variable domain sequence, SEQ ID NO: | Beta chain variable domain sequence, SEQ ID NO: |
|---|---|
| 58 | 2 |
| 59 | 2 |
| 60 | 2 |
| 17 | 25 |
| 17 | 23 |
| 12 | 23 |
| 16 | 23 |
| 14 | 23 |
| 12 | 25 |
| 15 | 23 |
| 19 | 23 |
| 20 | 23 |
| 11 | 61 |
| 11 | 62 |
| 11 | 63 |
| 11 | 64 |
| 11 | 65 |
| 14 | 65 |
| 16 | 66 |
| 22 | 65 |
| 20 | 31 |
| 17 | 31 |
| 17 | 66 |
| 18 | 65 |
| 19 | 65 |
| 17 | 65 |
| 21 | 65 |
| 11 | 31 |
| 19 | 31 |
| 16 | 31 |
| 12 | 31 |
| 11 | 66 |
| 17 | 67 |
| 11 | 67 |
| 15 | 66 |
| 15 | 68 |
| 11 | 67 |

(continued)

| Alpha chain variable domain sequence, SEQ ID NO: | Beta chain variable domain sequence, SEQ ID NO: |
|---|---|
| 15 | 8 |
| 11 | 69 |
| 15 | 70 |
| 15 | 69 |
| 17 | 69 |
| 11 | 70 |
| 17 | 70 |
| 11 | 28 |
| 11 | 23 |

Preferred embodiments provide a TCR of the invention comprising:

the alpha chain variable domain shown in the SEQ ID NO: 11 and the beta chain variable domain shown in the SEQ ID NO: 23, or phenotypically silent variants thereof;
the alpha chain variable domain shown in the SEQ ID NO: 11 and the beta chain variable domain shown in the SEQ ID NO: 64, or phenotypically silent variants thereof;
the alpha chain variable domain shown in the SEQ ID NO: 11 and the beta chain variable domain shown in the SEQ ID NO: 65, phenotypically silent variants thereof;

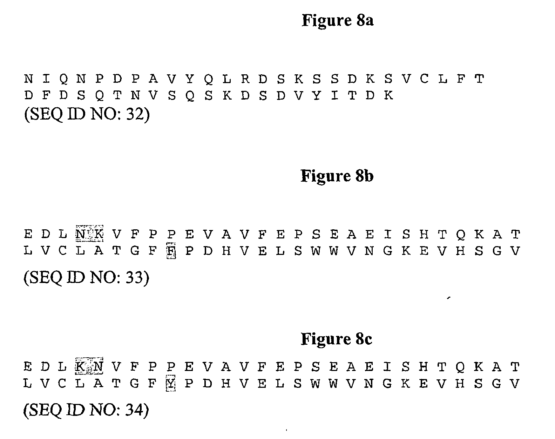

In another preferred embodiment TCRs of the invention comprising the variable domain combinations detailed above further comprise the alpha chain constant region amino acid sequence shown in Figure 8a (SEQ ID NO: 32) and one of the beta chain amino acid constant region sequences shown in Figures 8b and 8c (SEQ ID NOs: 33 and 34) or phenotypically silent variants thereof.

As used herein the term "phenotypically silent variants" is understood to refer to those TCRs which have a $K_D$ for the said ILAKFLHWL (SEQ ID NO: 37) -HLA-A*0201 complex of less than or equal to 1$\mu$M and/or has an off-rate ($k_{off}$) of $1x10^{-3}$ $S^{-1}$ or slower. For example, as is known to those skilled in the art, it may be possible to produce TCRs that incorporate changes in the constant and/or variable domains thereof compared to those detailed above without altering the affinity and/or off-rate for the interaction with the ILAKFLHWL-HLA-A*0201 complex. Such trivial variants are included in the scope of this invention. Those TCRs in which one or more conservative substitutions have been made also form part of this invention.

A suitable scTCR form comprises a first segment constituted by an amino acid sequence corresponding to a TCR $\alpha$ chain variable domain, a second segment constituted by an amino acid sequence corresponding to a TCR $\beta$ chain variable domain sequence fused to the N terminus of an amino acid sequence corresponding to a TCR $\beta$ chain constant domain extracellular sequence, and a linker sequence linking the C terminus of the first segment to the N terminus of the second segment.

Alternatively the first segment may be constituted by an amino acid sequence corresponding to a TCR $\beta$ chain variable domain, the second segment may be constituted by an amino acid sequence corresponding to a TCR $\alpha$ chain variable domain sequence fused to the N terminus of an amino acid sequence corresponding to a TCR $\alpha$ chain constant domain extracellular sequence

The above scTCRs may further comprise a disulfide bond between the first and second chains, said disulfide bond being one which has no equivalent in native $\alpha\beta$T cell receptors, and wherein the length of the linker sequence and the position of the disulfide bond being such that the variable domain sequences of the first and second segments are mutually orientated substantially as in native $\alpha\beta$ T cell receptors.

More specifically the first segment may be constituted by an amino acid sequence corresponding to a TCR $\alpha$ chain variable domain sequence fused to the N terminus of an amino acid sequence corresponding to a TCR $\alpha$ chain constant domain extracellular sequence, the second segment may be constituted by an amino acid sequence corresponding to a TCR $\beta$ chain variable domain fused to the N terminus of an amino acid sequence corresponding to TCR $\beta$ chain constant domain extracellular sequence, and a disulfide bond may be provided between the first and second chains, said disulfide bond being one which has no equivalent in native $\alpha\beta$ T cell receptors.

In the above scTCR forms, the linker sequence may link the C terminus of the first segment to the N terminus of the second segment, and may have the formula -PGGG-(SGGGG)$_n$-P- wherein n is 5 or 6 and P is proline, G is glycine and S is serine:

-PGGG-SGGGGSGGGGSGGGGSGGGGSGGGG-P (SEQ ID NO: 35)
-PGGG-SGGGGSGGGGSGGGGSGGGGSGGGGSGGGG-P (SEQ ID NO: 36)

A suitable dTCR form of the TCRs of the present invention comprises a first polypeptide wherein a sequence corresponding to a TCR α chain variable domain sequence is fused to the N terminus of a sequence corresponding to a TCR α chain constant domain extracellular sequence, and a second polypeptide wherein a sequence corresponding to a TCR β chain variable domain sequence fused to the N terminus a sequence corresponding to a TCR β chain constant domain extracellular sequence, the first and second polypeptides being linked by a disulfide bond which has no equivalent in native αβ T cell receptors.

The first polypeptide may comprise a TCR α chain variable domain sequence is fused to the N terminus of a sequence corresponding to a TCR α chain constant domain extracellular sequence, and a second polypeptide wherein a sequence corresponding to a TCR β chain variable domain sequence is fused to the N terminus a sequence corresponding to a TCR β chain constant domain extracellular sequence, the first and second polypeptides being linked by a disulfide bond between cysteine residues substituted for Thr 48 of exon 1 of TRAC*01 and Ser 57 of exon 1 of TRBC1*01 or TRBC2*01 or the non-human equivalent thereof. ("TRAC" etc. nomenclature herein as per T cell receptor Factsbook, (2001) LeFranc and LeFranc, Academic Press, ISBN 0-12-441352-8)

The dTCR or scTCR form of the TCRs of the invention may have amino acid sequences corresponding to human αβ TCR extracellular constant and variable domain sequences, and a disulfide bond may link amino acid residues of the said constant domain sequences, which disulfide bond has no equivalent in native TCRs. The disulfide bond is between cysteine residues corresponding to amino acid residues whose β carbon atoms are less than 0.6 nm apart in native TCRs, for example between cysteine residues substituted for Thr 48 of exon 1 of TRAC*01 and Ser 57 of exon 1 of TRBC1*01 or TRBC2*01 or the non-human equivalent thereof. Other sites where cysteines can be introduced to form the disulfide bond are the following residues in exon 1 of TRAC*01 for the TCR α chain and TRBC1*01 or TRBC2*01 for the TCR β chain:

| TCR α chain | TCR β chain | Native β carbon separation (nm) |
|---|---|---|
| Thr 45 | Ser 77 | 0.533 |
| Tyr 10 | Ser 17 | 0.359 |
| Thr 45 | Asp 59 | 0.560 |
| Ser 15 | Glu 15 | 0.59 |

In addition to the non-native disulfide bond referred to above, the dTCR or scTCR form of the TCRs of the invention may include a disulfide bond between residues corresponding to those linked by a disulfide bond in native TCRs.

The dTCR or scTCR form of the TCRs of the invention preferably does not contain a sequence corresponding to transmembrane or cytoplasmic sequences of native TCRs.

Preferred embodiments of the invention provide a soluble TCR consisting of:

the alpha chain amino acid sequence of SEQ ID NO: 71 and beta chain amino acid sequence SEQ ID NO: 73:

the alpha chain amino acid sequence of SEQ ID NO: 71 and beta chain amino acid sequence SEQ ID NO: 76;

the alpha chain amino acid sequence of SEQ ID NO: 71 and beta chain amino acid sequence SEQ ID NO: 74;

the alpha chain amino acid sequence of SEQ ID NO: 71 and beta chain amino acid sequence SEQ ID NO: 77;

the alpha chain amino acid sequence of SEQ ID NO: 71 and beta chain amino acid sequence SEQ ID NO: 75;

the alpha chain amino acid sequence of SEQ ID NO: 71 and beta chain amino acid sequence SEQ ID NO: 78;

SEQ ID NOs: 71, and 73-78 have been provided in a form which includes the N-terminal methionine (M).

*PEGylated TCR Monomers*

In one particular embodiment a TCR of the invention is associated with at least one polyalkylene glycol chain (s). This association may be cause in a number of ways known to those skilled in the art. In a preferred embodiment the polyalkylene chain(s) is/are covalently linked to the TCR. In a further embodiment the polyethylene glycol chains of the present aspect of the invention comprise at least two polyethylene repeating units.

*Multivalent TCR Complexes*

One aspect of the invention provides a multivalent TCR complex comprising at least two TCRs of the invention. In one embodiment of this aspect, at least two TCR molecules are linked via linker moieties to form multivalent complexes. Preferably the complexes are water soluble, so the linker moiety should be selected accordingly. Furthermore, it is preferable that the linker moiety should be capable of attachment to defined positions on the TCR molecules, so that the structural diversity of the complexes formed is minimised. One embodiment of the present aspect is provided by a TCR complex of the invention wherein the polymer chain or peptidic linker sequence extends between amino acid residues of each TCR which are not located in a variable region sequence of the TCR.

Since the complexes of the invention may be for use in medicine, the linker moieties should be chosen with due regard to their pharmaceutical suitability, for example their immunogenicity.

Examples of linker moieties which fulfil the above desirable criteria are known in the art, for example the art of linking antibody fragments.

There are two classes of linker that are preferred for use in the production of multivalent TCR molecules of the present invention. A TCR complex of the invention in which the TCRs are linked by a polyalkylene glycol chain provides one embodiment of the present aspect.

The first are hydrophilic polymers such as polyalkylene glycols. The most commonly used of this class are based on polyethylene glycol or PEG, the structure of which is shown below.

$$HOCH_2CH_2O\ (CH_2CH_2O)_n\text{-}CH_2CH_2OH$$

Wherein n is greater than two. However, others are based on other suitable, optionally substituted, polyalkylene glycols include polypropylene glycol, and copolymers of ethylene glycol and propylene glycol.

Such polymers may be used to treat or conjugate therapeutic agents, particularly polypeptide or protein therapeutics, to achieve beneficial changes to the PK profile of the therapeutic, for example reduced renal clearance, improved plasma half-life, reduced immunogenicity, and improved solubility. Such improvements in the PK profile of the PEG-therapeutic conjugate are believe to result from the PEG molecule or molecules forming a 'shell' around the therapeutic which sterically hinders the reaction with the immune system and reduces proteolytic degradation. (Casey et al, (2000) Tumor Targetting 4 235-244) The size of the hydrophilic polymer used my in particular be selected on the basis of the intended therapeutic use of the TCR complex. Thus for example, where the product is intended to leave the circulation and penetrate tissue, for example for use in the treatment of a tumour, it may be advantageous to use low molecular weight polymers in the order of 5 KDa. There are numerous review papers and books that detail the use of PEG and similar molecules in pharmaceutical formulations. For example, see Harris (1992) Polyethylene Glycol Chemistry - Biotechnical and Biomedical Applications, Plenum, New York, NY. or Harris & Zalipsky (1997) Chemistry and Biological Applications of Polyethylene Glycol ACS Books, Washington, D.C.

The polymer used can have a linear or branched conformation. Branched PEG molecules, or derivatives thereof, can be induced by the addition of branching moieties including glycerol and glycerol oligomers, pentaerythritol, sorbitol and lysine.

Usually, the polymer will have a chemically reactive group or groups in its structure, for example at one or both termini, and/or on branches from the backbone, to enable the polymer to link to target sites in the TCR. This chemically reactive group or groups may be attached directly to the hydrophilic polymer, or there may be a spacer group/moiety between the hydrophilic polymer and the reactive chemistry as shown below:

Reactive chemistry-Hydrophilic polymer-Reactive chemistry

Reactive chemistry-Spacer-Hydrophilic polymer-Spacer-Reactive chemistry

The spacer used in the formation of constructs of the type outlined above may be any organic moiety that is a non-reactive, chemically stable, chain, Such spacers include, by are not limited to the following:

$-(CH_2)_n-$ wherein n = 2 to 5

$-(CH_2)_3NHCO(CH_2)_2$

A TCR complex of the invention in which a divalent alkylene spacer radical is located between the polyalkylene glycol chain and its point of attachment to a TCR of the complex provides a further embodiment of the present aspect.

A TCR complex of the invention in which the polyalkylene glycol chain comprises at least two polyethylene glycol repeating units provides a further embodiment of the present aspect.

There are a number of commercial suppliers of hydrophilic polymers linked, directly or via a spacer, to reactive chemistries that may be of use in the present invention. These suppliers include Nektar Therapeutics (CA, USA), NOF Corporation (Japan), Sunbio (South Korea) and Enzon Pharmaceuticals (NJ, USA).

Commercially available hydrophilic polymers linked, directly or via a spacer, to reactive chemistries that may be of use in the present invention include, but are not limited to, the following:

| PEG linker Description | Source of PEG | Catalogue Number |
|---|---|---|
| **TCR Monomer attachment** | | |
| 5K linear (Maleimide) | Nektar | 2D2MOHO1 |
| 20K linear (Maleimide) | Nektar | 2D2MOPO1 |
| 20K linear (Maleimide) | NOF Corporation | SUNBRIGHT ME-200MA |
| 20K branched (Maleimide) | NOF Corporation | SUNBRIGHT GL2-200MA |
| 30K linear(Maleimide) | NOF Corporation | SUNBRIGHT ME-300MA |
| 40K branched PEG (Maleimide) | Nektar | 2D3XOTO1 |
| 5K-NP linear (for Lys attachment) | NOF Corporation | SUNBRIGHT MENP-50H |
| 10K-NP linear (for Lys attachment) | NOF Corporation | SUNBRIGHT MENP-10T |
| 20K-NP linear (for Lys attachment) | NOF Corporation | SUNBRIGHT MENP-20T |
| **TCR dimer linkers** | | |
| 3.4K linear (Maleimide) | Nektar | 2D2DOFO2 |
| 5K forked (Maleimide) | Nektar | 2D2DOHOF |
| 10K linear (with orthopyridyl ds-linkers in place of Maleimide) | Sunbio | |
| 20K forked (Maleimide) | Nektar | 2D2DOPOF |
| 20K linear (Maleimide) | NOF Corporation | |
| 40K forked (Maleimide) | Nektar | 2D3KOTOF |
| **Higher order TCR multimers** | | |
| 15K, 3 arms, $Mal_3$ (for trimer) | Nektar | OJOONO3 |
| 20K, 4 arms, $Mal_4$ (for tetramer) | Nektar | OJOOPO4 |
| 40 K, 8 arms, $Mal_8$ (for octamer) | Nektar | OJOOTO8 |

A wide variety of coupling chemistries can be used to couple polymer molecules to protein and peptide therapeutics. The choice of the most appropriate coupling chemistry is largely dependant on the desired coupling site. For example, the following coupling chemistries have been used attached to one or more of the termini of PEG molecules (Source: Nektar Molecular Engineering Catalogue 2003):

N-maleimide
Vinyl sulfone
Benzotriazole carbonate
Succinimidyl proprionate
Succinimidyl butanoate
Thio-ester

Acetaldehydes
Acrylates
Biotin
Primary amines

As stated above non-PEG based polymers also provide suitable linkers for multimerising the TCRs of the present invention. For example, moieties containing maleimide termini linked by aliphatic chains such as BMH and BMOE (Pierce, products Nos. 22330 and 22323) can be used.

Peptidic linkers are the other class of TCR linkers. These linkers are comprised of chains of amino acids, and function to produce simple linkers or multimerisation domains onto which TCR molecules can be attached. The biotin / streptavidin system has previously been used to produce TCR tetramers (see WO/99/60119) for in-vitro binding studies. However, streptavidin is a microbially-derived polypeptide and as such not ideally suited to use in a therapeutic.

A TCR complex of the invention in which the TCRs are linked by a peptidic linker derived from a human multimerisation domain provides a further embodiment of the present aspect.

There are a number of human proteins that contain a multimerisation domain that could be used in the production of multivalent TCR complexes. For example the tetramerisation domain of p53 which has been utilised to produce tetramers of scFv antibody fragments which exhibited increased serum persistence and significantly reduced off-rate compared to the monomeric scFV fragment. (Willuda et al. (2001) J. Biol. Chem. 276 (17) 14385-14392) Haemoglobin also has a tetramerisation domain that could potentially be used for this kind of application.

A multivalent TCR complex of the invention comprising at least two TCRs provides a final embodiment of this aspect, wherein at least one of said TCRs is associated with a therapeutic agent.

In one aspect a TCR (or multivalent complex thereof) of the present invention may alternatively or additionally comprise a reactive cysteine at the C-terminal or N-terminal of the alpha or beta chains thereof.

*Diagnostic and therapeutic Use*

In one aspect the TCR of the invention may be associated with a therapeutic agent or detectable moiety. For example, said therapeutic agent or detectable moiety may be covalently linked to the TCR.

In one embodiment of the invention said therapeutic agent or detectable moiety is covalently linked to the C-terminus of one or both TCR chains.

In one aspect the scTCR or one or both of the dTCR chains of TCRs of the present invention may be labelled with an detectable moiety, for example a label that is suitable for diagnostic purposes. Such labelled TCRs are useful in a method for detecting a ILAKFLHWL-HLA-A*0201 complex which method comprises contacting the TCR ligand with a TCR (or a multimeric high affinity TCR complex) which is specific for the TCR ligand; and detecting binding to the TCR ligand. In tetrameric TCR complexes formed for example, using biotinylated heterodimers, fluorescent streptavidin can be used to provide a detectable label. Such a fluorescently-labelled TCR tetramer is suitable for use in FACS analysis, for example to detect antigen presenting cells carrying the ILAKFLHWL-HLA-A*0201 complex for which these high affinity TCRs are specific.

Another manner in which the soluble TCRs of the present invention may be detected is by the use of TCR-specific antibodies, in particular monoclonal antibodies. There are many commercially available anti-TCR antibodies, such as $\alpha$F1 and $\beta$F1, which recognise the constant domains of the $\alpha$ and $\beta$ chains, respectively.

In a further aspect a TCR (or multivalent complex thereof) of the present invention may alternatively or additionally be associated with (e.g. covalently or otherwise linked to) a therapeutic agent which may be, for example, a toxic moiety for use in cell killing, or an immune effector molecule such as an interleukin or a cytokine. A multivalent TCR complex of the invention may have enhanced binding capability for a TCR ligand compared to a non-multimeric wild-type or T cell receptor heterodimer of the invention. Thus, the multivalent TCR complexes according to the invention are particularly useful for tracking or targeting cells presenting particular antigens *in vitro* or *in vivo,* and are also useful as intermediates for the production of further multivalent TCR complexes having such uses. These TCRs or multivalent TCR complexes may therefore be provided in a pharmaceutically acceptable formulation for use *in vivo.*

Also disclosed is a method for delivering a therapeutic agent to a target cell, which method comprises contacting potential target cells with a TCR or multivalent TCR complex in accordance with the invention under conditions to allow attachment of the TCR or multivalent TCR complex to the target cell, said TCR or multivalent TCR complex being specific for the ILAKFLHWL-HLA-A*0201 complex and having the therapeutic agent associated therewith.

In particular, the soluble TCR or multivalent TCR complex of the present invention can be used to deliver therapeutic agents to the location of cells presenting a particular antigen. This would be useful in many situations and, in particular, against tumours. A therapeutic agent could be delivered such that it would exercise its effect locally but not only on the cell it binds to. Thus, one particular strategy envisages anti-tumour molecules linked to TCRs or multivalent TCR complexes according to the invention specific for tumour antigens.

Many therapeutic agents could be employed for this use, for instance radioactive compounds, enzymes (perforin for example) or chemotherapeutic agents (cis-platin for example). To ensure that toxic effects are exercised in the desired location the toxin could be inside a liposome linked to streptavidin so that the compound is released slowly. This will prevent damaging effects during the transport in the body and ensure that the toxin has maximum effect after binding of the TCR to the relevant antigen presenting cells.

Other suitable therapeutic agents include:

- small molecule cytotoxic agents, i.e. compounds with the ability to kill mammalian cells having a molecular weight of less than 700 daltons. Such compounds could also contain toxic metals capable of having a cytotoxic effect. Furthermore, it is to be understood that these small molecule cytotoxic agents also include pro-drugs, i.e. compounds that decay or are converted under physiological conditions to release cytotoxic agents. Examples of such agents include cis-platin, maytansine derivatives, rachelmycin, calicheamicin, docetaxel, etoposide, gemcitabine, ifosfamide, irinotecan, melphalan, mitoxantrone, sorfimer sodiumphotofrin II, temozolmide, topotecan, trimetreate glucuronate, auristatin E vincristine and doxorubicin;
- peptide cytotoxins, i.e. proteins or fragments thereof with the ability to kill mammalian cells. Including but not limited to, ricin, diphtheria toxin, pseudomonas bacterial exotoxin A, DNAase and RNAase;
- radio-nuclides, i.e. unstable isotopes of elements which decay with the concurrent emission of one or more of $\alpha$ or $\beta$ particles, or $\gamma$ rays. including but not limited to, iodine 131, rhenium 186, indium 111, yttrium 90, bismuth 210 and 213, actinium 225 and astatine 213; chelating agents may be used to facilitate the association of these radio-nuclides to the high affinity TCRs, or multimers thereof;
- prodrugs, including but not limited to, antibody directed enzyme pro-drugs;
- immuno-stimulants, i.e. moieties which stimulate immune response. Including but not limited to, cytokines such as IL-2 and IFN, Superantigens and mutants thereof, TCR-HLA fusions and chemokines such as IL-8, platelet factor 4, melanoma growth stimulatory protein, etc, antibodies or fragments thereof, complement activators, xenogeneic protein domains, allogeneic protein domains, viral/bacterial protein domains, viral/bacterial peptides and anti-T cell determinant antibodies (e.g. anti-CD3 or anti-CD28).

Soluble TCRs or multivalent TCR complexes of the invention may be linked to an enzyme capable of converting a prodrug to a drug. This allows the prodrug to be converted to the drug only at the site where it is required (i.e. targeted by the sTCR).

It is expected that the high affinity ILAKFLHWL (SEQ ID NO: 37)-HLA-A*0201 specific TCRs disclosed herein may be used in methods for the diagnosis and treatment of cancer.

For cancer treatment, the localisation in the vicinity of tumours or metastasis would enhance the effect of toxins or immunostimulants. For vaccine delivery, the vaccine antigen could be localised in the vicinity of antigen presenting cells, thus enhancing the efficacy of the antigen. The method can also be applied for imaging purposes.

One embodiment is provided by an isolated cell presenting a TCR of the invention. For example, said cell may be a T cell.

Further embodiments of the invention are provided by a pharmaceutical composition comprising:

a TCR or a multivalent TCR complex of the invention (optionally associated with a therapeutic agent), or a plurality of cells presenting at least one TCR of the invention, together with a pharmaceutically acceptable carrier;

Also disclosed is a method of treatment of cancer comprising administering to a subject suffering such cancer disease an effective amount of a TCR or a multivalent TCR complex of the invention (optionally associated with a therapeutic agent), or a plurality of cells presenting at least one TCR of the invention. In a related embodiment the invention provides for the use of a TCR or a multivalent TCR complex of the invention (optionally associated with a therapeutic agent), or a plurality of cells presenting at least one TCR of the invention, in the preparation of a composition for the treatment of cancer.

Therapeutic or imaging TCRs in accordance with the invention will usually be supplied as part of a sterile, pharmaceutical composition which will normally include a pharmaceutically acceptable carrier. This pharmaceutical composition may be in any suitable form, (depending upon the desired method of administering it to a patient). It may be provided in unit dosage form, will generally be provided in a sealed container and may be provided as part of a kit. Such a kit would normally (although not necessarily) include instructions for use. It may include a plurality of said unit dosage forms.

The pharmaceutical composition may be adapted for administration by any appropriate route, for example parenteral, transdermal or via inhalation, preferably a parenteral (including subcutaneous, intramuscular, or, most preferably intravenous) route. Such compositions may be prepared by any method known in the art of pharmacy, for example by admixing the active ingredient with the carrier(s) or excipient(s) under sterile conditions.

Dosages of the substances of the present invention can vary between wide limits, depending upon the disease or disorder to be treated, the age and condition of the individual to be treated, etc. and a physician will ultimately determine appropriate dosages to be used.

*Additional Aspects*

A scTCR or dTCR (which preferably is constituted by constant and variable sequences corresponding to human sequences) of the present invention may be provided in substantially pure form, or as a purified or isolated preparation. For example, it may be provided in a form which is substantially free of other proteins.

The invention also provides a method of producing a high affinity TCR having the property of binding to ILAKFLHWL-HLA-A*0201.CHARACTERISED IN THAT the TCR (i) comprises at least one TCR α chain variable domain and/or at least one TCR β chain variable domain and (ii) has a $K_D$ for the said ILAKFLHWL-HLA-A*0201 complex of less than or equal to 1μM and/or an off-rate ($k_{off}$) for the ILAKFLHWL-HLA-A*0201 complex of $1x10^{-3}$ $S^{-1}$ or slower, wherein the method comprises:

(a) the production of a TCR comprising the α and β chain variable domains of the ILA TCR wherein one or both of the α and β chain variable domains comprise a mutation(s) in one or more of the amino acids identified in claims 7 and 8;

(b) contacting said mutated TCR with ILAKFLHWL-HLA-A*0201 under conditions suitable to allow the binding of the TCR to ILAKFLHWL-HLA-A*0201;

and measuring the $K_D$ and/or $k_{off}$ of the interaction.

Preferred features of each aspect of the invention are as for each of the other aspect *mutatis mutandis.*

## Examples

The invention is further described in the following examples, which do not limit the scope of the invention in any way.

Reference is made in the following to the accompanying drawings in which:

Figure 1a and 1b details the alpha chain variable domain amino acid and beta chain variable domain amino acid sequences of the native ILA TCR respectively.

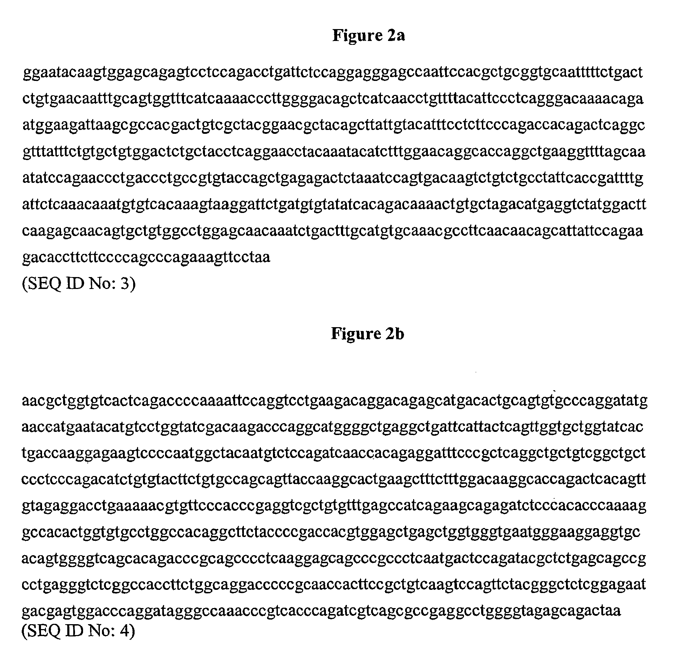

Figures 2a and 2b show respectively the DNA sequence of soluble versions of the native ILA TCR α and β chains.

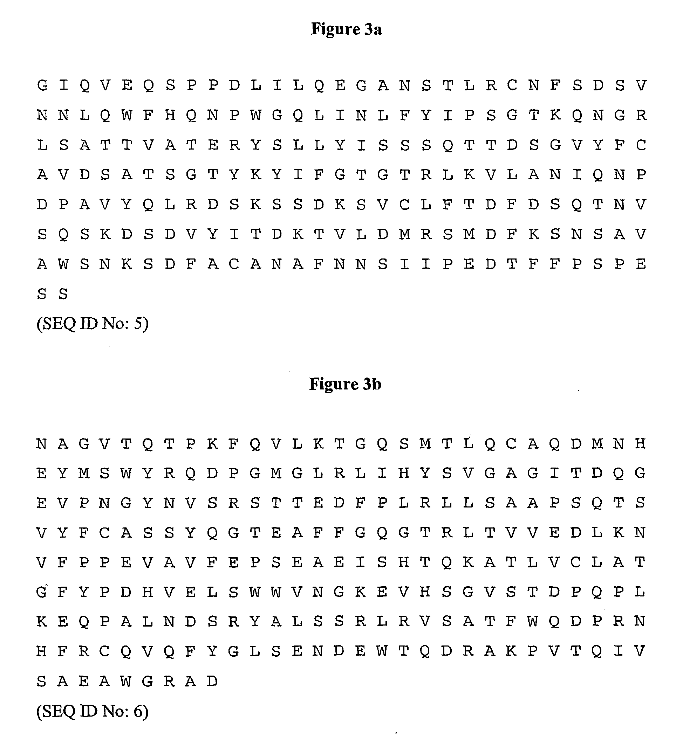

Figures 3a and 3b show respectively the ILA TCR α and β chain extracellular amino acid sequences produced from the DNA sequences of Figures 2a and 2b.

Figures 4a and 4b show respectively the DNA sequence of soluble versions of the ILA TCR α and β chains mutated to include additional cysteine residues to form a non-native disulphide bond. The mutated codon is indicated by shading.

Figures 5a and 5b show respectively the ILA TCR α and β chain extracellular amino acid sequences produced from the DNA sequences of Figures 4a and 4b. The introduced cysteine is indicated by shading.

Figure 6 details the alpha chain variable domain amino acid sequences of the high affinity ILA TCR variants.

Figure 7 details the beta chain variable domain amino acid sequences of the high affinity ILA TCR variants.

Figure 8a details the amino acid sequence of a soluble form of TRAC.

Figure 8b details the amino acid sequence of a soluble form of TRBC1. The amino acids which differ from those in TRBC2 are highlighted.

Figure 8c details the amino acid sequence of a soluble form of TRBC2. The amino acids which differ from those in TRBC1 are highlighted.

Figure 9 details the DNA sequence of the pEX954 plasmid.

Figure 10 details the DNA sequence of the per821 plasmid.

Figure 11 details the DNA sequence of the pEX202 plasmid.

Figure 12 details the DNA sequence of the pEX205 plasmid.

Figure 13 details the alpha chain variable domain amino acid sequences of additional high affinity ILA TCR variants.

Figure 14 details the beta chain variable domain amino acid sequences of additional high affinity ILA TCR variants.

Figure 15a details the amino acid sequence of the alpha chain of the c13c1 ILA TCR.

Figure 15b details the amino acid sequence of the beta chain of the c13c1 ILA TCR fused via a peptide linker to wild-type human IL-2. The mutations conferring high pMHC affinity to the TCR are underlined. The introduced cysteine residue in the TCR constant domain is highlighted and the linker and IL-2 sequence is in italics.

Figure 16 shows FACs staining of T2 cell pulsed with a range of ILAKFLHWL peptide concentrations.

Figure 17 shows FACs staining of J82 cells transfected with an ILAKFLHWL-producing ubiquitin minigene (± proteosome inhibitors)

Figure 18 shows inhibition of ILA-1 T cell clone-mediated killing of J82 cells transfected with an ILAKFLHWL-producing ubiquitin minigene by soluble high affinity c13c1 ILA TCR-IL-2 fusion protein.

Figure 19 shows ELISPOT data demonstrating the inhibition of T cell activation against peptide-pulsed LNCaP and MCF-7 target cells by soluble high affinity c13c1 ILA TCR-IL-2 fusion protein.

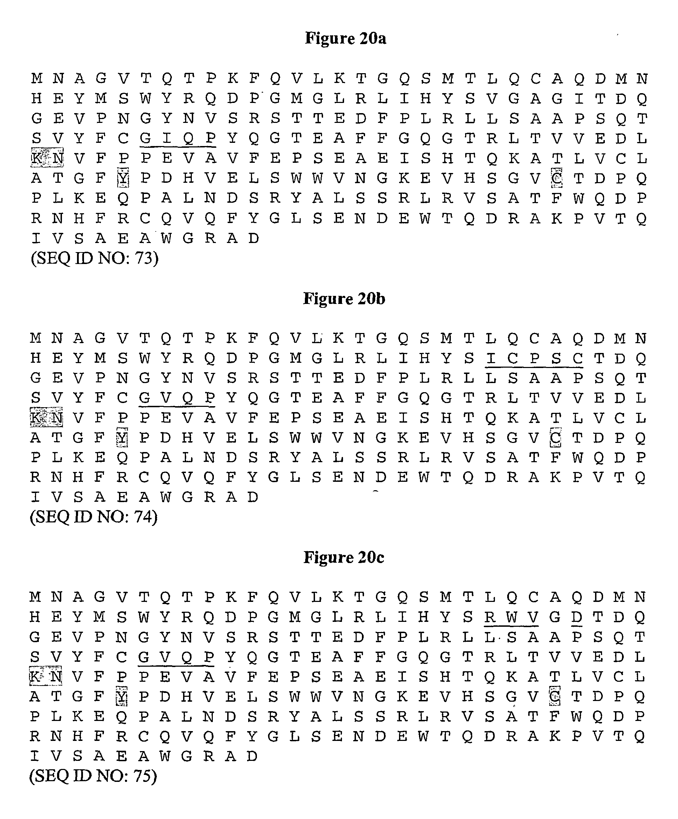

Figure 20a shows the TRBC2 form of the beta chain of the high affinity ILA TCR c13c1. (SEQ ID NO: 73) The amino acids which differ from those in TRBC1 are highlighted.

Figure 20b shows the TRBC2 form of the beta chain of the high affinity ILA TCR c13c1. (SEQ ID NO: 74) The amino acids which differ from those in TRBC1 are highlighted.

Figure 20c shows the TRBC2 form of the beta chain of the high affinity ILA TCR c13c1. (SEQ ID NO: 75) The amino acids which differ from those in TRBC1 are highlighted.

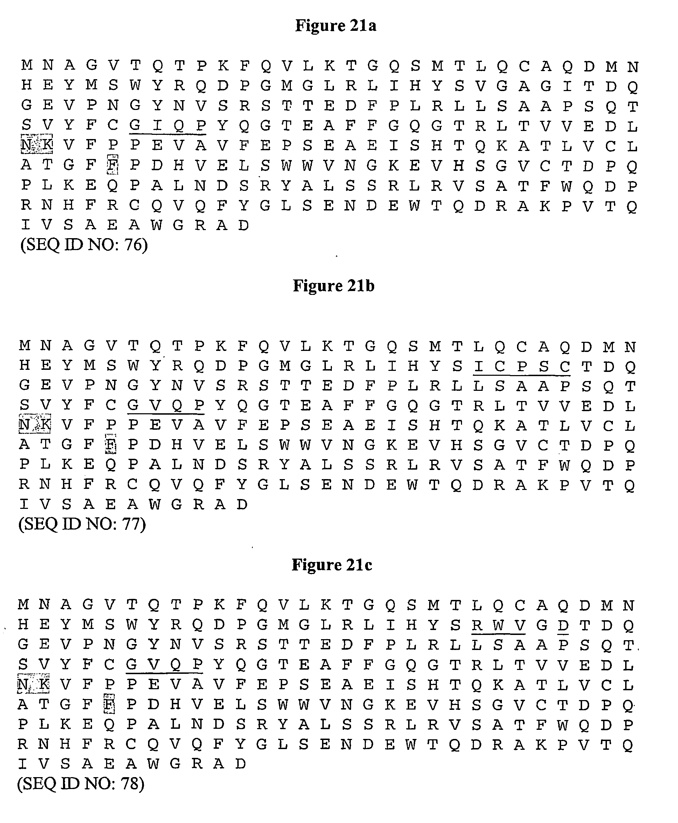

Figure 21 a shows the TRBC1 form of the beta chain of the high affinity ILA TCR c13c1. (SEQ ID NO: 76) The amino acids which differ from those in TRBC2 are highlighted.

Figure 21b shows the TRBC1 form of the beta chain of the high affinity ILA TCR c13c1. (SEQ ID NO: 77) The amino acids which differ from those in TRBC2 are highlighted.

Figure 21c shows the TRBC1 form of the beta chain of the high affinity ILA TCR c13c1. (SEQ ID NO: 78) The amino acids which differ from those in TRBC2 are highlighted.

*Examples 1 - Production of a soluble disulfide-linked TCR comprising the native ILA TCR variable domain*

*RNA isolation*

Total RNA was isolated from 10000 clonal T cells by re-suspension in 100μl tri-reagent (Sigma) and processing of the lysate according to the manufacturer's instructions. After the final precipitation the RNA was re-dissolved in 12.5 μl RNAse free water.

cDNA Production

To the above sample of RNA, 2.5μl of 10 mM oligo-$dT^{15}$ (Promega) was added and the sample incubated at 60°C for 2 minutes then placed on ice. Reverse transcription was carried out using OmniscriptRT kit (Qiagen) by addition of 2μl RT buffer (10 x), 2μl 5 mM dNTP, 1 μl Omniscript reverse transcriptase. The sample was mixed and incubated for 1 hour at 37°C. cDNA was then stored at -80°C.

The above cDNA was used as template. A panel of forward primers covering all possible alpha and beta variable chains was used to screen for, and amplify by PCR, alpha and beta chains genes. Primer sequences used for TCR chain gene amplification were designed from the NCBI website (http://www.ncbi.nlm.nih.gov/Entrez/) using accession numbers obtained from the T cell receptor Factsbook, (2001) LeFranc and LeFranc, Academic Press, ISBN 0-12-441352-8. Alpha -chain forward primers were designed to contain a *ClaI* restriction site and the universal alpha chain reverse primer a *SalI* restriction site. Beta-chain forward primers were designed to contain a *AseI* restriction site and universal beta reverse primer an *AgeI* restriction site.

Recipient vectors for the TCR gene fragments were based on a pGMT7 parent plasmid, which contains the T7 promoter for high level expression in E.coli strain BL21-DE3(pLysS) (Pan et al., Biotechniques (2000) 29 (6): 1234-8)

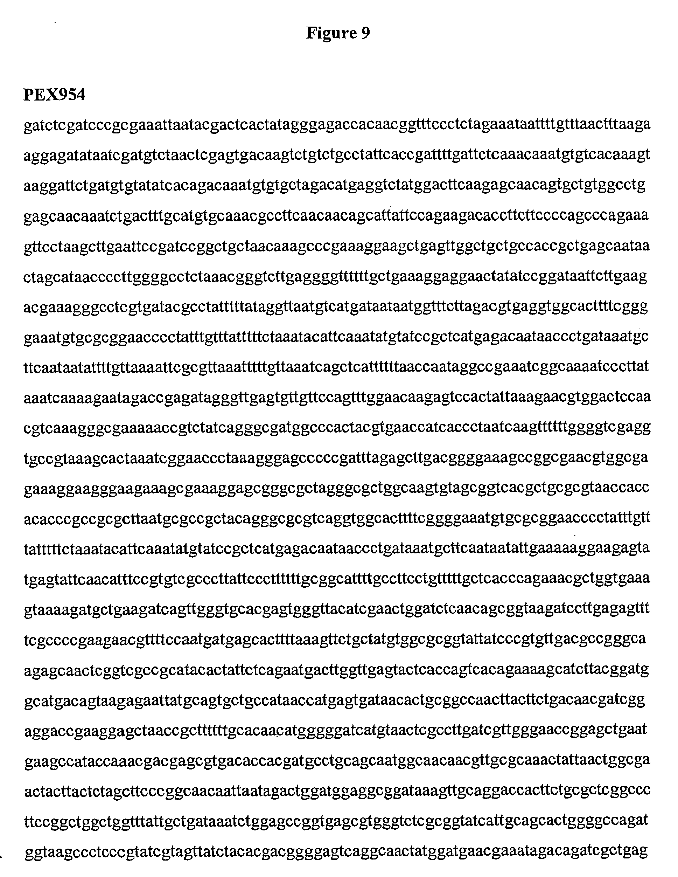

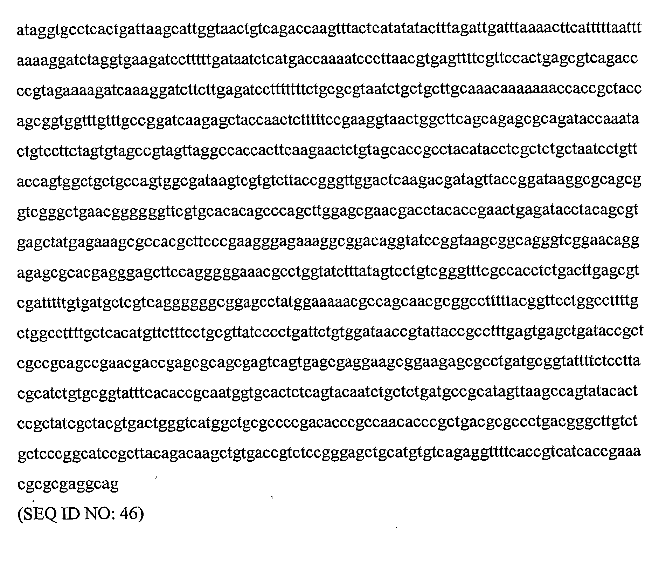

Alpha chain purified PCR products were digested with *ClaI* and *SalII* and ligated into pEX954 (see figure 9) cut with *ClaI* and *XhoI.*

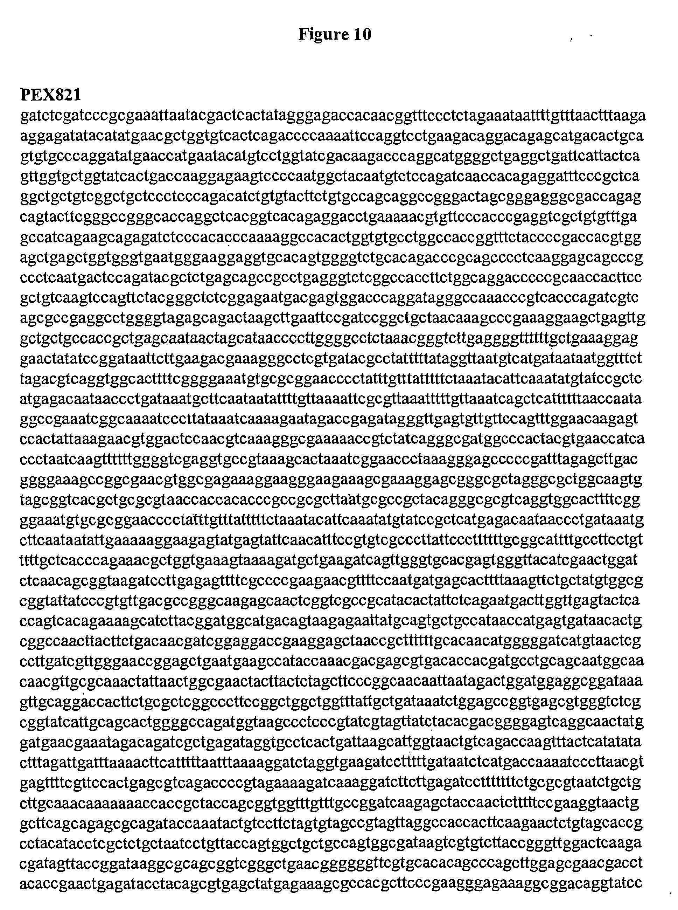

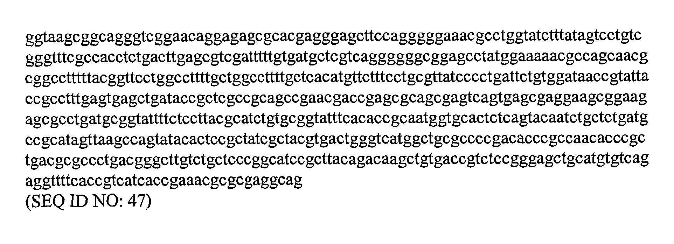

Beta chain purified PCR products were digested with *AseI* and *AgeI* and ligated into pEX821 (See Figure 10) cut with *NdeI/AgeI.*

*Ligation*

The cut PCR product and cut vector were ligated using a rapid DNA ligation kit (Roche) following the manufacturers instructions.

Ligated plasmids were transformed into competent E.coli strain XL1-blue cells and plated out on LB/agar plates containing 100mg/ml ampicillin. Following incubation overnight at 37°C, single colonies were picked and grown in 10 ml LB containing 100mg/ml ampicillin overnight at 37°C with shaking. Cloned plasmids were purified using a Miniprep kit (Qiagen) and the insert was sequenced using an automated DNA sequencer (Lark Technologies).

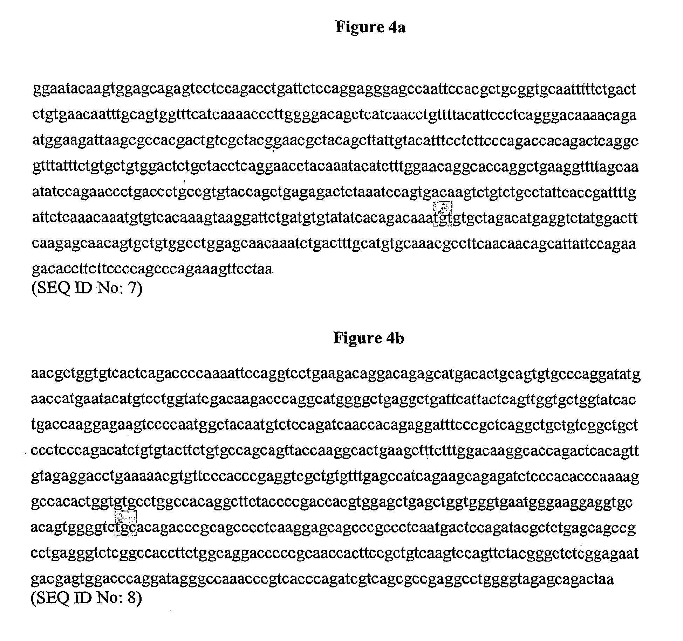

Figures 4a and 4b show respectively the DNA sequence of soluble versions of the ILA TCR α and β chains mutated to include additional cysteine residues to form a non-native disulphide bond.

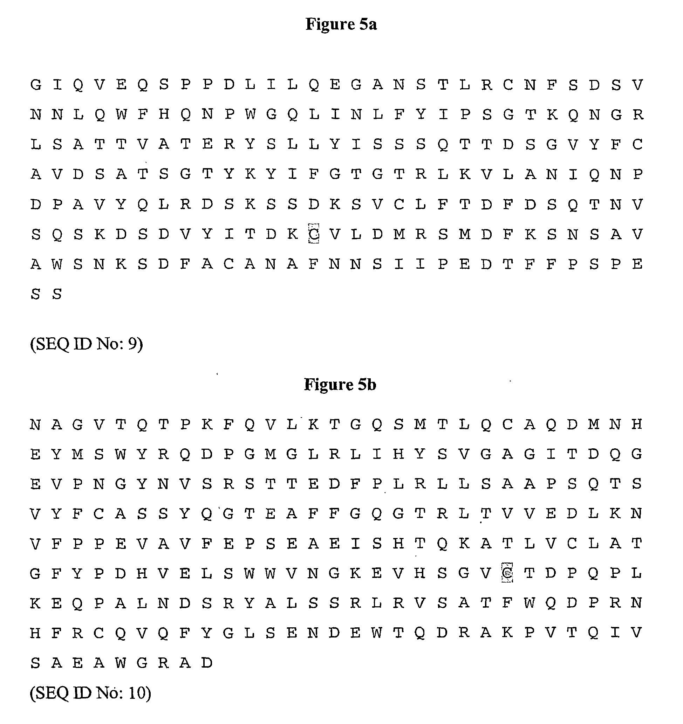

Figures 5a and 5b show respectively the ILA TCR α and β chain extracellular amino acid sequences produced from the DNA sequences of Figures 4a and 4b

*Examples 2- Production of high affinity variants of the soluble disulfide linked ILA TCR*

The soluble disulfide-linked native ILA TCR produced as described in Example 1 can be used a template from which to produce the TCRs of the invention which have an increased affinity for the ILAKFLHWL (SEQ ID NO: 35) -HLA-A*0201 complex.

The amino sequences of the mutated TCR alpha and beta chain variable domains which demonstrate high affinity for the ILAKFLHWL-HLA-A*0201 complex are listed in Figures 6 and 7 respectively. (SEQ ID Nos: 11-22 and 23-31 respectively) As is known to those skilled in the art the necessary codon changes required to produce these mutated chains can be introduced into the DNA encoding these chains by site-directed mutagenesis. (QuickChange™ Site-Directed Mutagenesis Kit from Stratagene)

Briefly, this is achieved by using primers that incorporate the desired codon change(s) and the plasmids containing the relevant TCR chain as a template for the mutagenesis:

> Mutagenesis was carried out using the following conditions : 50ng plasmid template, 1μl of 10mM dNTP, 5 μl of 10x Pfu DNA polymerase buffer as supplied by the manufacturer, 25 pmol of fwd primer, 25 pmol of rev primer, 1μl pfu DNA polymerase in total volume 50 μl. After an initial denaturation step of 2 mins at 95C, the reaction was subjected to 25 cycles of denaturation (95C, 10 secs), annealing (55C 10 secs), and elongation (72C, 8 mins). The resulting product was digested with DpnI restriction enzyme to remove the template plasmid and transformed into E. coli strain XL1-blue. Mutagenesis was verified by sequencing.

*Examples 3 - Production of soluble "zippered " high affinity TCRs*

*Alpha chains - c-jun leucine zipper*

The construct was made by PCR stitching.

For the 5'-end of the gene the plasmid coding for the high affinity TCR alpha chains and containing the code for the introduced inter-chain di-sulfide bridge was used as template. PCR with the following two primer pairs generated the desired variable domain.

| | |
|---|---|
| 5'-TRAV22 fwd (SEQ ID NO: 38) | tctctcattaatgGGAATACAAGTGGAGCAGAGTCCT |
| C-alpha rev (SEQ ID NO: 39) | CGGCAGGGTCAGGGTTCTGG |

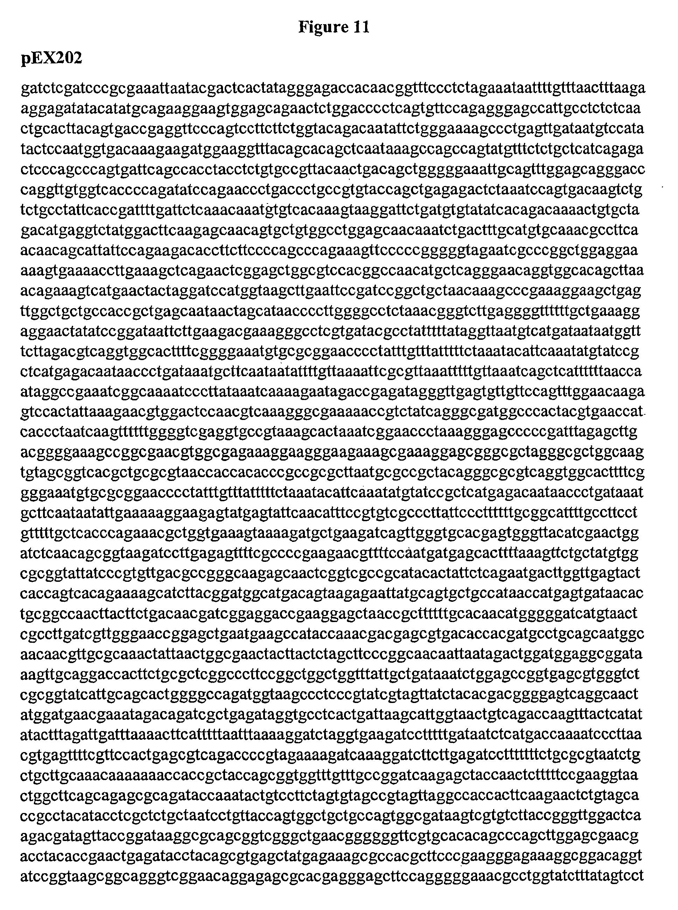

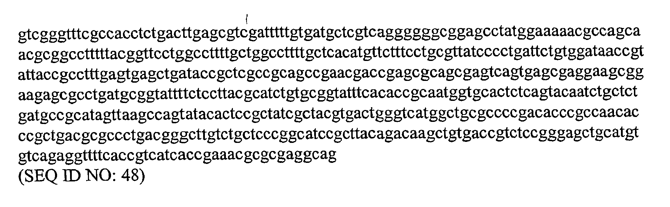

For the 3'-end of the gene the plasmid pEX202 (see Figure 11), coding for a wild type affinity TCR alpha chain fused to human c-jun leucine zipper domain and not containing the code for the introduced inter-chain di-sulfide bridge, was used as template. PCR with the following primer pair generated the desired constant domain.

| | |
|---|---|
| C-alpha fwd (SEQ ID NO:40) | CCAGAACCCTGACCCTGCCG |
| 3'-alpha rev (SEQ ID NO: 41.) | aagcttcccgggggaactttctgggctggg |

The two products were mixed and diluted 1000 fold and 1 μl was used as template in a 50 μl PCR with 5'-TRAV21 fwd and 3'-alpha rev primers.

The resulting PCR product was digested using restriction enzymes AseI and XmaI and ligated into pEX202 cut with NdeI and XmaI.

PCRs were carried out using the following conditions : 50 pg plasmid template, 1 μl of 10 mM dNTP, 5 μl of 10x Pfu DNA polymerase buffer as supplied by the manufacturer, 25 pmol of fwd primer, 25 pmol of rev primer, 1μl Pfu DNA polymerase in total volume 50 μl. After an initial denaturation step of 2 mins at 95C, the reaction was subjected to 30 cycles of denaturation (95C, 10 secs), annealing (55C 10 secs), and elongation (72C, 2 mins).

*Beta chain - c-fos leucine zipper*

The construct was made by PCR stitching.

For the 5'-end of the gene plasmids coding for the high affinity TCR beta chains and containing the introduced inter-chain di-sulfide bridge were used as template. PCR with the following two primers generated the desired variable domain gene fragment.

| | |
|---|---|
| TRBV6-5 fwd (SEQ ID NO: 42) | tctctcattaatgaatgctggtgtcactcagacccc |
| C-beta rev (SEQ ID NO: 43) | CTTCTGATGGCTCAAACACAGC |

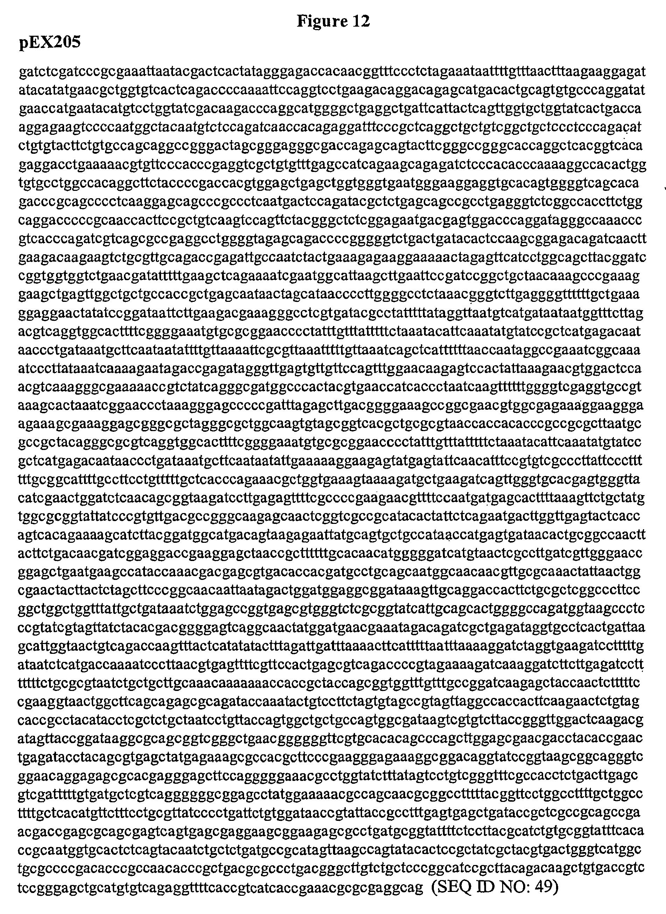

For the 3'-end of the gene the plasmid pEX205 (see Figure 12), coding for a wild type affinity TCR beta chain fused to the human c-fos leucine zipper domain and not containing the code for the introduced inter-chain di-sulfide bridge, was used as template. PCR with the following two primers generated the desired constant domain gene fragment.

| | |
|---|---|
| C-beta fwd (SEQ ID NO: 44) | GCTGTGTTTGAGCCATCAGAAG |
| TRBC rev (SEQ ID NO: 45) | aagcttcccggggtctgctctaccccaggc |

The two products were mixed and diluted 1000 fold and 1 μl was used as template in a 50 μl PCR with TRBV6-5 fwd and TRBC rev primers. PCRs were carried out as described above.

The resulting PCR product was digested using restriction enzymes AseI and XmaI and ligated into pEX205 (Figure 12) cut with NdeI and XmaI.

*Example 4 - Expression, refolding and purification of soluble TCR*

The expression plasmids containing the mutated α-chain, and β-chain respectively as prepared in Examples 1, 2 or 3 were transformed separately into *E.coli* strain BL21pLysS, and single ampicillin-resistant colonies were grown at 37°C in TYP (ampicillin 100μg/ml) medium to $OD_{600}$ of 0.4 before inducing protein expression with 0.5mM IPTG. Cells were harvested three hours post-induction by centrifugation for 30 minutes at 4000rpm in a Beckman J-6B. Cell pellets were re-suspended in a buffer containing 50mM Tris-HCl, 25% (w/v) sucrose, 1mM NaEDTA, 0.1% (w/v) NaAzide, 10mM DTT, pH 8.0. After an overnight freeze-thaw step, re-suspended cells were sonicated in 1 minute bursts for a total of around 10 minutes in a Milsonix XL2020 sonicator using a standard 12mm diameter probe. Inclusion body pellets were recovered by centrifugation for 30 minutes at 13000rpm in a Beckman J2-21 centrifuge. Three detergent washes were then carried out to remove cell debris and membrane components. Each time the inclusion body pellet was homogenised in a Triton buffer (50mM Tris-HCl, 0.5% Triton-X100, 200mM NaCl, 10mM NaEDTA, 0.1% (w/v) NaAzide, 2mM DTT, pH 8.0) before being pelleted by centrifugation for 15 minutes at 13000rpm in a Beckman J2-21. Detergent and salt was then removed by a similar wash in the following buffer: 50mM Tris-HCl, 1mM NaEDTA, 0.1% (w/v) NaAzide, 2mM DTT, pH 8.0. Finally, the inclusion bodies were divided into 30 mg aliquots and frozen at -70°C. Inclusion body protein yield was quantitated by solubilising with 6M guanidine-HCl and measurement with a Bradford dye-binding assay (PerBio).

Approximately 30mg of TCR β chain and 60mg of TCR α chain solubilised inclusion bodies were thawed from frozen stocks, samples were then mixed and the mixture diluted into 15ml of a guanidine solution (6 M Guanidine-hydrochloride, 10mM Sodium Acetate, 10mM EDTA), to ensure complete chain de-naturation. The guanidine solution containing fully reduced and denatured TCR chains was then injected into 1 litre of the following refolding buffer: 100mM Tris pH 8.5, 400mM L-Arginine, 2mM EDTA, 5mM reduced Glutathione, 0.5mM oxidised Glutathione, 5M urea, 0.2mM PMSF. The redox couple (2-mercaptoethylamine and cystamine (to final concentrations of 6.6mM and 3.7mM, respectively) were added approximately 5 minutes before addition of the denatured TCR chains. The solution was left for 5 hrs ± 15minutes. The refolded TCR was dialysed in Spectrapor 1 membrane (Spectrum; Product No. 132670) against 10 L 10 mM Tris pH 8.1 at 5°C ± 3°C for 18-20 hours. After this time, the dialysis buffer was changed to fresh 10 mM Tris pH 8.1 (10 L) and dialysis was continued at 5°C ± 3°C for another 20-22 hours.

sTCR was separated from degradation products and impurities by loading the dialysed refold onto a POROS 50HQ anion exchange column and eluting bound protein with a gradient of 0-500mM NaCl over 50 column volumes using an Akta purifier (Pharmacia). Peak fractions were stored at 4°C and analysed by Coomassie-stained SDS-PAGE before being pooled and concentrated. Finally, the sTCR was purified and characterised using a Superdex 200HR gel filtration column pre-equilibrated in HBS-EP buffer (10 mM HEPES pH 7.4, 150 mM NaCl, 3.5 mM EDTA, 0.05% nonidet p40). The peak eluting at a relative molecular weight of approximately 50 kDa was pooled and concentrated prior to characterisation by BIAcore surface plasmon resonance analysis.

*Example 5 - BIAcore surface plasmon resonance characterisation of sTCR binding to specific pMHC*

A surface plasmon resonance biosensor (BIAcore 3000™) was used to analyse the binding of a sTCR to its peptide-MHC ligand. This was facilitated by producing single pMHC complexes (described below) which were immobilised to a streptavidin-coated binding surface in a semi-oriented fashion, allowing efficient testing of the binding of a soluble T-cell receptor to up to four different pMHC (immobilised on separate flow cells) simultaneously. Manual injection of HLA complex allows the precise level of immobilised class I molecules to be manipulated easily.

Biotinylated class I HLA-A*0201 molecules were refolded *in vitro* from bacterially-expressed inclusion bodies containing the constituent subunit proteins and synthetic peptide, followed by purification and *in vitro* enzymatic biotinylation (O'Callaghan et al. (1999) Anal. Biochem. 266: 9-15). HLA-A*0201-heavy chain was expressed with a C-terminal biotinylation tag which replaces the transmembrane and cytoplasmic domains of the protein in an appropriate construct. Inclusion body expression levels of ~75 mg/litre bacterial culture were obtained. The MHC light-chain or β2-microglobulin was also expressed as inclusion bodies in *E.coli* from an appropriate construct, at a level of ~500 mg/litre bacterial culture.

*E. coli* cells were lysed and inclusion bodies are purified to approximately 80% purity. Protein from inclusion bodies was denatured in 6 M guanidine-HCl, 50 mM Tris pH 8.1, 100 mM NaCl, 10 mM DTT, 10 mM EDTA, and was refolded at a concentration of 30 mg/litre heavy chain, 30 mg/litre β2m into 0.4 M L-Arginine-HCl, 100 mM Tris pH 8.1, 3.7 mM cystamine, mM cysteamine, 4 mg/ml of the ILAKFLHWL peptide required to be loaded by the HLA-A*0201 molecule, by addition of a single pulse of denatured protein into refold buffer at < 5°C. Refolding was allowed to reach completion at 4°C for at least 1 hour.

Buffer was exchanged by dialysis in 10 volumes of 10 mM Tris pH 8.1. Two changes of buffer were necessary to reduce the ionic strength of the solution sufficiently. The protein solution was then filtered through a 1.5μm cellulose acetate filter and loaded onto a POROS 50HQ anion exchange column (8 ml bed volume). Protein was eluted with a linear 0-500 mM NaCl gradient. HLA-A*0201-peptide complex eluted at approximately 250 mM NaCl, and peak fractions were collected, a cocktail of protease inhibitors (Calbiochem) was added and the fractions were chilled on ice.

Biotinylation tagged pMHC molecules were buffer exchanged into 10 mM Tris pH 8.1, 5 mM NaCl using a Pharmacia fast desalting column equilibrated in the same buffer. Immediately upon elution, the protein-containing fractions were chilled on ice and protease inhibitor cocktail (Calbiochem) was added. Biotinylation reagents were then added:

1 mM biotin, 5 mM ATP (buffered to pH 8), 7.5 mM MgCl2, and 5 μg/ml BirA enzyme (purified according to O'Callaghan et al. (1999) Anal. Biochem. 266: 9-15). The mixture was then allowed to incubate at room temperature overnight.

The biotinylated pHLA-A*0201 molecules were purified using gel filtration chromatography. A Pharmacia Superdex 75 HR 10/30 column was pre-equilibrated with filtered PBS and 1 ml of the biotinylation reaction mixture was loaded and the column was developed with PBS at 0.5 ml/min. Biotinylated pHLA-A*0201 molecules eluted as a single peak at approximately 15 ml. Fractions containing protein were pooled, chilled on ice, and protease inhibitor cocktail was added. Protein concentration was determined using a Coomassie-binding assay (PerBio) and aliquots of biotinylated pHLA-A*0201 molecules were stored frozen at -20°C. Streptavidin was immobilised by standard amine coupling methods.

Such immobilised complexes are capable of binding both T-cell receptors and the coreceptor CD8αα, both of which may be injected in the soluble phase. Specific binding of TCR is obtained even at low concentrations (at least 40μg/ml), implying the TCR is relatively stable. The pMHC binding properties of sTCR are observed to be qualitatively and quantitatively similar if sTCR is used either in the soluble or immobilised phase. This is an important control for partial activity of soluble species and also suggests that biotinylated pMHC complexes are biologically as active as non-biotinylated complexes.

The interactions between ILA sTCR containing a novel inter-chain bond and its ligand/ MHC complex or an irrelevant HLA-peptide combination, the production of which is described above, were analysed on a BIAcore 3000™ surface plasmon resonance (SPR) biosensor. SPR measures changes in refractive index expressed in response units (RU) near a sensor surface within a small flow cell, a principle that can be used to detect receptor ligand interactions and to analyse their affinity and kinetic parameters. The probe flow cells were prepared by immobilising the individual HLA-peptide complexes in separate flow cells via binding between the biotin cross linked onto β2m and streptavidin which have been chemically cross linked to the activated surface of the flow cells. The assay was then performed by passing sTCR over the surfaces of the different flow cells at a constant flow rate, measuring the SPR response in doing so.

*To measure Equilibrium binding constant*

Serial dilutions of WT ILA sTCR were prepared and injected at constant flow rate of 5 μl min-1 over two different flow cells; one coated with ~1000 RU of specific ILAKFLHWL-HLA-A*0201 complex, the second coated with ~1000 RU of non-specific HLA-A2 -peptide complex. Response was normalised for each concentration using the measurement from the control cell. Normalised data response was plotted versus concentration of TCR sample and fitted to a hyperbola in order to calculate the equilibrium binding constant, $K_D$. (Price & Dwek, Principles and Problems in Physical Chemistry for Biochemists (2nd Edition) 1979, Clarendon Press, Oxford).

*To measure Kinetic Parameters*

For high affinity TCRs $K_D$ was determined by experimentally measuring the dissociation rate constant, kd, and the association rate constant, ka. The equilibrium constant $K_D$ was calculated as kd/ka.
TCR was injected over two different cells one coated with -300 RU of specific ILAKFLHWL-HLA-A*0201 complex, the second coated witch ~300 RU of non-specific HLA-A2 -peptide complex. Flow rate was set at 50 μl/min. Typically 250 μl of TCR at -3 μM concentration was injected. Buffer was then flowed over until the response had returned to baseline. Kinetic parameters were calculated using Biaevaluation software. The dissociation phase was also fitted to a single exponential decay equation enabling calculation of half-life.

*Results*

The interaction between a soluble disulfide-linked native ILA TCR and the ILAKFLHWL-HLA-A*0201 complex was analysed using the above methods and demonstrated a $K_D$ of approximately 25μM, an off-rate ($k_{off}$) of 1.1 x $10^{-1}$ $S^{-1}$ and a half-life of 0.18 minutes.

The TCRs specified in the following table have a $K_D$ of less than or equal to 1 μM and/or a $k_{off}$ of 1 x $10^{-3}$ $S^{-1}$ or slower.

| Alpha chain variable domain sequence, SEQ ID NO: | Beta chain variable domain sequence, SEQ ID NO: |
|---|---|
| 1 | 23 |
| 1 | 24 |
| 1 | 25 |

(continued)

| Alpha chain variable domain sequence, SEQ ID NO: | Beta chain variable domain sequence, SEQ ID NO: |
|---|---|
| 1 | 26 |
| 1 | 27 |
| 1 | 28 |
| 1 | 29 |
| 1 | 30 |
| 1 | 31 |
| 11 | 2 |
| 12 | 2 |
| 13 | 2 |
| 14 | 2 |
| 15 | 2 |
| 16 | 2 |
| 17 | 2 |
| 18 | 2 |
| 19 | 2 |
| 20 | 2 |
| 21 | 2 |
| 22 | 2 |
| 50 | 2 |
| 51 | 2 |
| 52 | 2 |
| 53 | 2 |
| 54 | 2 |
| 55 | 2 |
| 56 | 2 |
| 57 | 2 |
| 58 | 2 |
| 59 | 2 |
| 60 | 2 |
| 17 | 25 |
| 17 | 23 |
| 12 | 23 |
| 16 | 23 |
| 14 | 23 |
| 12 | 25 |
| 15 | 23 |
| 19 | 23 |

(continued)

| Alpha chain variable domain sequence, SEQ ID NO: | Beta chain variable domain sequence, SEQ ID NO: |
|---|---|
| 20 | 23 |
| 11 | 61 |
| 11 | 62 |
| 11 | 63 |
| 11 | 64 |
| 11 | 65 |
| 14 | 65 |
| 16 | 66 |
| 22 | 65 |
| 20 | 31 |
| 17 | 31 |
| 17 | 66 |
| 18 | 65 |
| 19 | 65 |
| 17 | 65 |
| 21 | 65 |
| 11 | 31 |
| 19 | 31 |
| 16 | 31 |
| 12 | 31 |
| 11 | 66 |
| 17 | 67 |
| 11 | 67 |
| 15 | 66 |
| 15 | 68 |
| 11 | 67 |
| 15 | 8 |
| 11 | 69 |
| 15 | 70 |
| 15 | 69 |
| 17 | 69 |
| 11 | 70 |
| 17 | 70 |
| 11 | 28 |
| 11 | 23 |

*Example 6 - Competition ELISA analysis of the binding of native and mutated disulphide-linked ILA TCRS displayed on phage particles.*

The following competition ELISA assay using native and mutated disulfide-linked ILA TCRs displayed on the phage particles was used to assess the affinity of these molecules for the ILAKFLHWL-HLA-A*0201 complex:

Binding of the ILA TCR-displaying phage particles to immobilised ILAKFLHWL-HLA-A*0201 complex in ELISA was detected with primary rabbit anti-fd antisera (Sigma) followed by alkaline phosphatase (AP) conjugated anti-Rabbit mAb (Sigma) in the presence of varying amounts of soluble ILAKFLHWL-HLA-A*0201 complex. Non-specific protein binding sites in the plates can be blocked with 2% MPBS or 3% BSA-PBS.

*Materials and reagents*

1. Coating buffer, PBS

2. PBS: 138mM NaCl, 2.7mM KCl, 10mM $Na_2HPO_4$, 2mM $KH_2PO_4$

3. MPBS , 3% marvel-PBS

4. PBS-Tween: PBS, 0.1% Tween-20

5. Substrate solution, Sigma FAST pNPP, Cat# N2770

*Method*

1. Rinse NeutrAvidin coated wells twice with PBS.

2. Add 25μl of biotin-HLA-A2 Tax or biotin-HLA-A2 NYESO in PBS at concentration of 10 μg/ml, and incubate at room temperature for 30 to 60 min.

3. Rinse the wells twice with PBS

4. Add 300 μl of 3% Marvel-PBS, and incubate at room temperature for 1hr. Mix the TCR-phage suspension with 1 volume of 3% Marvel-PBS and incubate at room temperature.

5. Rinse the wells twice with PBS

6. Add 25 μl of the mixture of phage-A6 TCR/Marvel-PBS and the required amount (20nM or 200nM) of soluble ILAKFLHWL-HLA-A*0201 complex, incubate on ice for 1hr

7. Rinse the wells three times with ice-cold PBStween, and three times with ice-cold PBS.

8. Add 25 μl of ice cold rabbit anti-fd antibody diluted 1:1000 in Marvel-PBS, and incubate on ice for 1hr

9. Rinse the wells three times with ice-cold PBStween, and three times with ice-cold PBS.

10. Add 25 μl of ice cold anti-rabbit mAb-Ap conjugate diluted 1:50,000 in Marvel-PBS, and incubate on ice for 1hr

11. Rinse the wells three times with ice-cold PBStween, and three times with ice-cold PBS.

12. Add 150 μl of Alkaline phosphatase yellow to each well and read the signal at 405nm.

The degree of inhibition of binding that occurs for a given addition of soluble ILAKFLHWL-HLA-A*0201 is proportional to the affinity of the TCR for the ILAKFLHWL-HLA-A*0201 complex.

*Results*

| α **Variable Domain** | β **Variable Domain** | **% Binding Inhibition 20nM Sol. pMHC** | **% Binding Inhibition 200nM Sol. pMHC** |
|---|---|---|---|
| SEQ ID NO: 1 | SEQ ID NO: 2 | 0 | 0 |
| SEQ ID NO: 1 | SEQ ID NO: 23 | 70 | 95 |
| SEQ ID NO: 1 | SEQ ID NO: 24 | 64 | 89 |
| SEQ ID NO: 1 | SEQ ID NO: 25 | 88 | 93 |
| SEQ ID NO: 1 | SEQ ID NO: 26 | 84 | 96 |
| SEQ ID NO: 1 | SEQ ID NO: 27 | 68 | 96 |
| SEQ ID NO: 1 | SEQ ID NO: 28 | 88 | 94 |
| SEQ ID NO: 1 | SEQ ID NO: 29 | 79 | 92 |
| SEQ ID NO: 1 | SEQ ID NO: 30 | 79 | 95 |
| SEQ ID NO: 1 | SEQ ID NO: 31 | 33/80 | 95 |
| SEQ ID NO: 11 | SEQ ID NO: 2 | 82 | 98 |
| SEQ ID NO: 12 | SEQ ID NO: 2 | 87 | 98 |
| SEQ ID NO: 13 | SEQ ID NO: 2 | 77 | 97 |
| SEQ ID NO: 14 | SEQ ID NO: 2 | 88 | 98 |
| SEQ ID NO: 15 | SEQ ID NO: 2 | 47 | 92 |
| SEQ ID NO: 16 | SEQ ID NO: 2 | 67 | 95 |
| SEQ ID NO: 17 | SEQ ID NO: 2 | 63 | 95 |
| SEQ ID NO: 18 | SEQ ID NO: 2 | 47 | 94 |
| SEQ ID NO: 19 | SEQ ID NO: 2 | 61 | 96 |
| SEQ ID NO: 20 | SEQ ID NO: 2 | 80 | 95 |
| SEQ ID NO: 21 | SEQ ID NO: 2 | 71 | 97 |
| SEQ ID NO: 22 | SEQ ID NO: 2 | 79 | 95 |

*Example 7 - In-vitro cell staining using high affinity clone cl3cl ILA TCR- IL-2 fusion protein.*

T2 lymphoblastoid cells were pulsed with a telomerase-derived ILAKFLHWL (540-548) peptide at a range of concentrations ($10^{-5}$ -$10^{-11}$M) for 180 minutes at 37∘C. After pulsing, cells were washed in serum-free RPMI and 5 x $10^{5}$ cells were incubated with high affinity clone c13c1 ILA TCR/IL-2 fusion protein for 10min at room temperature, followed by secondary anti-IL-2 mAb conjugated with PE (Serotec) for 15min at room temperature. After washing, bound TCR-IL-2 was quantified by flow cytometry using a FACSVantage SE (Becton Dickinson).Controls, also using peptide-pulsed T2 cells were included where TCR-IL-2 was omitted.

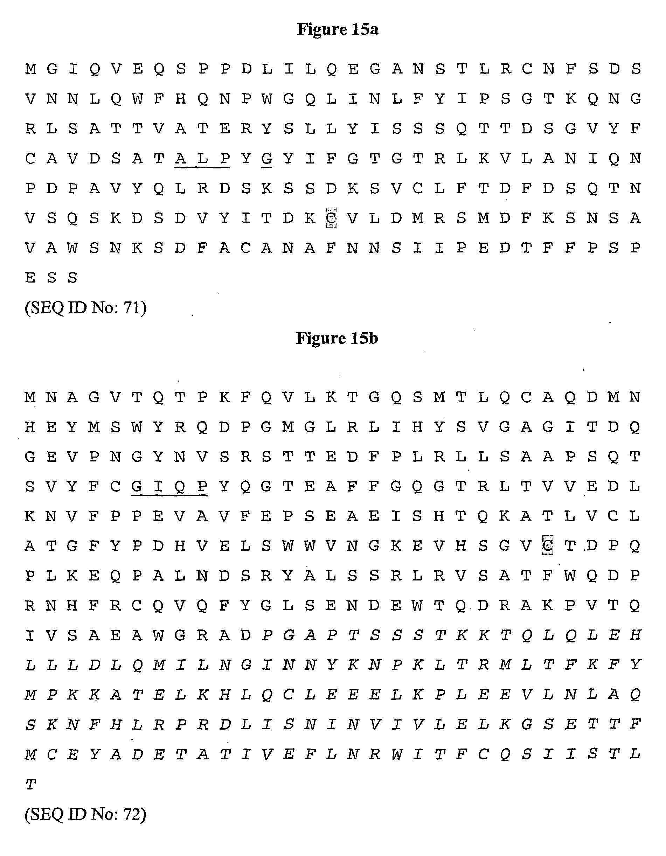

Figure 15a details the amino acid sequence of the alpha chain of the c13c1 ILA TCR. (SEQ ID NO: 71) Figure 15b details the amino acid sequence of the beta chain of the c13c1 ILA TCR fused via a peptide linker to wild-type human IL-2. (SEQ ID NO: 72) The alpha and beta chain variable domain mutations contained within the soluble c13c1 ILA TCR - IL-2 fusion protein correspond to those detailed in SEQ ID NO: 11 and SEQ ID NO: 23 respectively. Note that SEQ ID NOs: 71 and 72 have been provided in a form which includes the N-terminal methionine (M).

In similar experiments, J82 bladder cancer cells transfected with a telomerase-deived ILAKFLHWL (540-548) peptide expressing ubiquitin minigene construct were used. The J82 cells were transfected using substantially the methods described in (Rimoldi et al., (2000) J. Immunol. 165 7253-7261). Cells were labelled as described above.

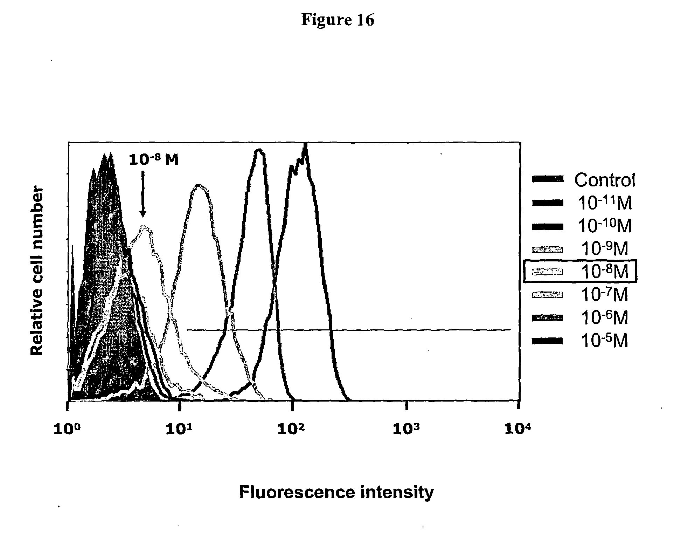

Figure 16 shows FACs staining of T2 cell pulsed with a range of ILAKFLHWL peptide concentrations.

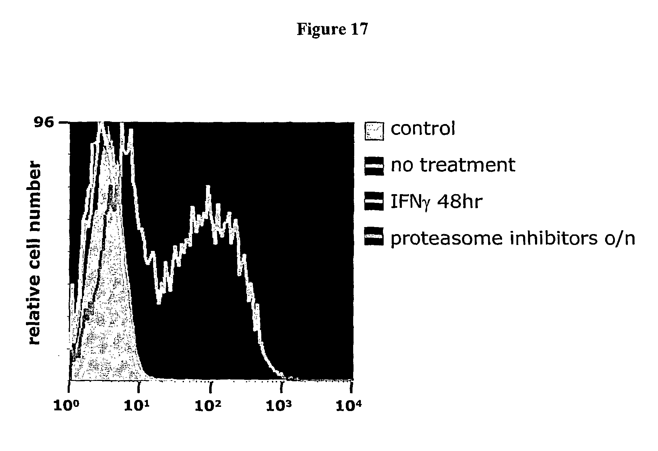

Figure 17 shows FACs staining of J82 cells transfected with an ILAKFLHWL-producing ubiquitin minigene (± proteosome inhibitors).

*Examples 8 - Soluble TCR-mediated inhibition of CTL activation using high affinity c13c1 ILA TCR -IL-2 fusion protein.*

The following assay was carried out to demonstrate that the soluble high affinity c1c13 ILA-IL-2 TCR was capable of inhibiting cell lysis mediated by an ILAKFLHWL-HLA-A*0201 specific CTL clone (ILA-1).

*In-vitro cell lysis assay*

J82 cells transfected with an ILAKFLHWL encoding mini-gene construct were treated with, or without, proteosomes inhibitors (1.5μM epoxomicin and 20μM calpain inhibitor I) for 16 hours before the CTL assay.
These target cells were treated with trypsin for 5 minutes at 37 °C. The cells are then washed and re-suspended in R10 media with 3μl BATDA reagent / 1 x $10^6$ cells as directed by the instructions supplied with the Europium/DELFIA assay kit. (Perkin Elmer) These cells are then incubated for 30 minutes at 37 °C, prior to washing three times in R10 media supplemented with 1mM sulfinpyrazone.
5000 target cells were then plated out per well in 50μl of R10 media in a 96 well plate (Nunc).
The following was then added to the above target cell cultures:
1 x $10^{-7}$ M high affinity c13c1 ILA TCR-IL-2 fusion protein in 50μl of R10 media.
ILAKFLHWL-HLA-A*0201 specific T cell clone (ILA-1) at a range of cell numbers in 50μl of R10 media. (To give Effector: Target ratios of 100:1, 30:1, 10:1 and 3:1) These cultures were then incubated for 2 hours at 37°C, 5%$CO_2$. 20μl of supernatant was then removed from each well and placed into a black opaque 96 well plate (Nunc). 180μl of Europium solution from the Europium/DELFIA assay kit was then added to each well and the level of target cell lysis that had occurred was assayed by time-resolved fluorescence in a Wallac Victor 2. (Perkin Elmer)

*Calculations:*

$$\% \text{ cell lysis } = 100 \text{ x } (RFU_{Exp} - RFU_{Spont}) / (RFU_{Max} - RFU_{Spont})$$

Wherein:

RFU - is relative fluorescence units

$RFU_{Exp}$ - is the RFU measured in the sample wells - cell free background RFU.

$RFUS_{pont}$ - is the RFU measured in the sample wells not containing any Effector cells - cell free background RFU.

$RFU_{Max}$ - is the RFU measured in the sample wells to which triton x-100 was added - cell free background RFU.

*Results*

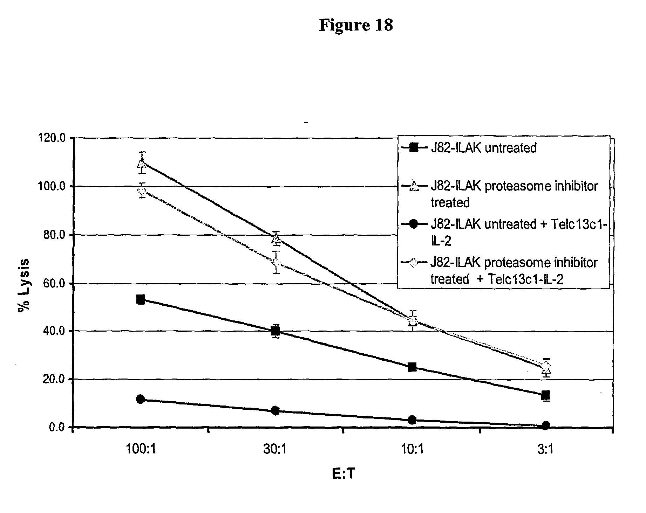

The soluble high affinity c13c1 ILA TCR-IL-2 fusion protein caused a significant inhibition of ILA-1T cell clone-mediated killing of J82 target cells in the absence of proteosomes inhibitor treatment. (See Figure 18)

*Example 9 - CTL activation ELISPOT assay*

The following assay was carried out to demonstrate that the soluble high affinity c1c13 ILA-IL-2 TCR was capable of inhibiting activation of an ILAKFLHWL-HLA-A*0201 specific CTL clone (ILA-1). IFN-γ production was used as the read-out for CTL activation.

*Reagents*

R10 Assay media: 10% FCS (heat-inactivated, Gibco, cat# 10108-165), 88% RPMI 1640 (Gibco, cat# 42401-018), 1% glutamine (Gibco, cat# 25030-024) and 1% penicillin/streptomycin (Gibco, cat# 15070-063).

Peptide: (obtained from various sources) initially dissolved in DMSO (Sigma, cat# D2650) at 4mg/ml and frozen, then made up to 200 μM in assay media and frozen in small aliquots. One aliquot of 200 μM peptide is thawed for each experiment so that the peptide has only gone through two freeze-thaw cycles.

Wash buffer: 0.01M PBS/0.05% Tween 20 (1 sachet of Phosphate buffered saline with Tween 20, pH7.4 from Sigma, Cat. # P-3563 dissolved in 1 litre distilled water gives final composition 0.01M PBS, 0.138M NaCl, 0.0027M KCl, 0.05% Tween 20).
PBS (Gibco, cat#10010-015).

The EliSpot kit contains all other reagents required i.e. capture and detection antibodies, skimmed milk powder, BSA, streptavidin-alkaline phosphatase, BCIP/NBT solution (Human IFN-g PVDF Eli-spot 20 x 96 wells with plates (IDS cat# Doc-856.051.020, DC-856.000.000 - use both codes on an order). The following method is based on the instructions supplied with each kit but contains some alterations.

*Method*

ELISPOT plates were prepared according to the manufacturers instructions. (Diaclone kit, Immunodiagnostic systems, UK

LNCaP or MCF-7 cancer cell line target cells were treated with trypsin for 5 minutes at 37 °C. The cells are then washed and re-suspended in R10 media with or without 1uM ILAKFLHWL peptide, then incubated for 1 hour at 37°C, 5%$CO_2$.

50,000 target cells per well were then plated out into a 96 well ELISPOT plate.

To these plates the following was added to the relevant well::

1 x 10-7 M of the high affinity c13c1 ILA TCR, or an irrelevant high affinity TCR in 50 μl of R10 media.

500 ILA-1 effector cells in 50 μl of R10 media.

The plates were then incubated for 5 hours at 37°C, 5%$CO_2$. The ELISPOT was then processed according to manufacturer's instructions.

*Results*

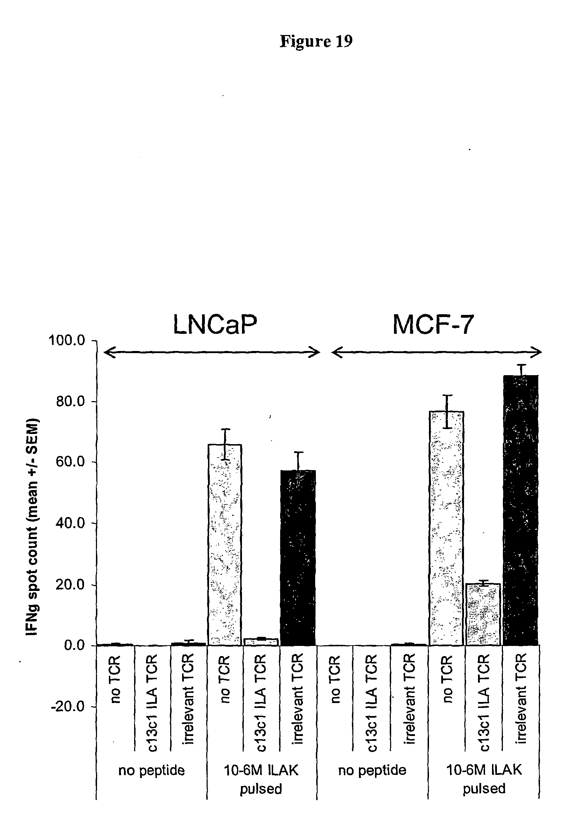

The soluble high affinity c1c13 ILA TCR strongly inhibits the activation of ILA-1 T cell clones against both MCF-7 and LNCaP cancer cells, as measured by IFN-γ production. Whereas the irrelevant soluble high affinity TCR had no effect on the activation of this T cell clone. (See Figure 19)

## Claims

1. A T-cell receptor (TCR) having the property of binding to ILAKFLHWL-HLA-A*0201 and comprising at least one TCR α chain variable domain and/or at least one TCR β chain variable domain **CHARACTERISED IN THAT** said TCR

(i) is mutated relative to the native ILA TCR α chain variable domain shown in SEQ ID No: 1 and/or β chain variable domain shown in SEQ ID NO: 2 in at least one complementarity determining region, and
(ii) has a $K_D$ for the said ILAKFLHWL-HLA-A*0201 complex of less than or equal to 1μM and/or has an off-rate ($k_{off}$) for the ILAKFLHWL-HLA-A*0201 complex of $1x10^{-3}$ $s^{-1}$ or slower.

**2.** A TCR as claimed in claim 1, which is mutated relative to the native ILA TCR α chain variable domain shown in SEQ ID No: 1 and/or β chain variable domain shown in SEQ ID NO: 2 in at least one variable domain framework region.

**3.** A TCR as claimed in claim 1 or claim 2 comprising one or more of alpha chain variable domain amino acids 94A, 94L, 94Q, 94T, 94G, 94R, 94M, 94H, 94K, 94E, 94N, 95L, 95P, 95I, 95V, 95M, 96P, 96M, 97F, 98G or 98A using the numbering shown in SEQ ID NO: 1.

**4.** A TCR as claimed in any of the preceding claims comprising one or more of beta chain variable domain amino acids 19V, 50I, 50L, 50R, 51H, 51C, 51 W, 52P, 52W, 52E, 52V, 53E, 53S, 53F, 54Y, 54V, 54C, 54E, 54D, 92G, 93T, 93L, 93V, 94Q and 95P using the numbering shown in SEQ ID NO: 2.

**5.** A TCR as claimed in any of the preceding claims comprising one of the alpha chain variable domain amino acid sequences shown in SEQ ID Nos: 11 to 22 or 50 to 60 , optionally comprising one or more phenotypically silent substitutions.

**6.** A TCR as claimed in any of the preceding claims comprising one of the beta chain variable domain amino acid sequences shown in SEQ ID Nos: 23 to 31 or 61 to 70 optionally comprising one or more phenotypically silent substitutions.

**7.** A TCR as claimed in any of the preceding claims comprising the alpha and beta chain variable domain pairings shown in the following table, optionally comprising one or more phenotypically silent substitutions:

| Alpha chain variable domain sequence, SEQ ID NO: | Beta chain variable domain sequence, SEQ ID NO: |
|---|---|
| 1 | 23 |
| 1 | 24 |
| 1 | 25 |
| 1 | 26 |
| 1 | 27 |
| 1 | 28 |
| 1 | 29 |
| 1 | 30 |
| 1 | 31 |
| 11 | 2 |
| 12 | 2 |
| 13 | 2 |
| 14 | 2 |
| 15 | 2 |
| 16 | 2 |
| 17 | 2 |
| 18 | 2 |

(continued)

| Alpha chain variable domain sequence, SEQ ID NO: | Beta chain variable domain sequence, SEQ ID NO: |
|---|---|
| 19 | 2 |
| 20 | 2 |
| 21 | 2 |
| 22 | 2 |
| 50 | 2 |
| 51 | 2 |
| 52 | 2 |
| 53 | 2 |
| 54 | 2 |
| 55 | 2 |
| 56 | 2 |
| 57 | 2 |
| 58 | 2 |
| 59 | 2 |
| 60 | 2 |
| 17 | 25 |
| 17 | 23 |
| 12 | 23 |
| 16 | 23 |
| 14 | 23 |
| 12 | 25 |
| 15 | 23 |
| 19 | 23 |
| 20 | 23 |
| 11 | 61 |
| 11 | 62 |
| 11 | 63 |
| 11 | 64 |
| 11 | 65 |
| 14 | 65 |
| 16 | 66 |
| 22 | 65 |
| 20 | 31 |
| 17 | 31 |
| 17 | 66 |
| 18 | 65 |
| 19 | 65 |

(continued)

| Alpha chain variable domain sequence, SEQ ID NO: | Beta chain variable domain sequence, SEQ ID NO: |
|---|---|
| 17 | 65 |
| 21 | 65 |
| 11 | 31 |
| 19 | 31 |
| 16 | 31 |
| 12 | 31 |
| 11 | 66 |
| 17 | 67 |
| 11 | 67 |
| 15 | 66 |
| 15 | 68 |
| 11 | 67 |
| 15 | 8 |
| 11 | 69 |
| 15 | 70 |
| 15 | 69 |
| 17 | 69 |
| 11 | 70 |
| 17 | 70 |
| 11 | 28 |
| 11 | 23 |

**8.** A TCR as claimed in any of claims 1 to 4 comprising the alpha chain variable domain shown in the SEQ ID NO: 11 and the beta chain variable domain shown in the SEQ ID NO: 23, optionally comprising one or more phenotypically silent substitutions.

**9.** A TCR as claimed in any of the preceding claims further comprising the alpha chain constant region amino acid sequence shown in SEQ ID NO: 32, and/or one of the beta chain amino acid constant region sequences shown in SEQ ID NOs: 33 and 34, optionally comprising one or more phenotypically silent substitutions.

**10.** A TCR as claimed in any of the preceding claims which is a dimeric TCR (dTCR) comprising
a first polypeptide wherein a sequence corresponding to a TCR α chain variable domain sequence is fused to the N terminus of a sequence corresponding to a TCR α chain constant domain extracellular sequence, and
a second polypeptide wherein a sequence corresponding to a TCR βchain variable domain sequence fused to the N terminus a sequence corresponding to a TCR β chain constant domain extracellular sequence,
the said constant domain sequences of the first and second polypeptides being linked by a disulfide bond which has no equivalent in native αβ T cell receptors between cysteine residues substituted for Thr 48 of exon 1 of TRAC*01 and Ser 57 of exon 1 of TRBC1*01 or TRBC2*01 or the non-human equivalent thereof.

**11.** A TCR as claimed in claim 1 consisting of the alpha chain amino acid sequence of SEQ ID NO: 71 and beta chain amino acid sequence SEQ ID NO: 73.

**12.** A TCR as claimed in any preceding claim wherein the TCR is associated with at least one polyalkylene glycol chain(s).

**13.** A TCR as claimed in any preceding claim wherein a therapeutic agent or detectable moiety is covalently linked to the C terminus of one or both TCR chains.

**14.** A TCR as claimed in claim 13 wherein the therapeutic agent is selected from:

(i) an immune effector molecule;
(ii) a cytotoxic agent;
and
(iii) a radionuclide.

**15.** A TCR as claimed in claim 13 or claim 14 wherein the therapeutic agent is IL-2.

**16.** A multivalent TCR complex comprising at least two TCRs as claimed in any of the preceding claims.

**17.** An isolated cell presenting a TCR as defined in any of claims 1 to 10.

**18.** A pharmaceutical composition comprising a TCR or a multivalent TCR complex as claimed in any of claims 1 to 16, or a plurality of cells as claimed in claim 17, together with a pharmaceutically acceptable carrier.

**19.** The use of a TCR or a multivalent TCR complex as claimed in any of claims 1 to 16, or a plurality of cells as claimed in claim 17, in the preparation of a composition for the treatment of cancer.

## Patentansprüche

**1.** T-Zell-Rezeptor (TCR) mit der Eigenschaft, ILAKFLHWL-HLA-A*0201 zu binden, und der mindestens eine variable Domäne der TCR α-Kette und/oder mindestens eine variable Domäne der TCR β-Kette umfasst, **dadurch gekennzeichnet, dass** dieser TCR

(i) hinsichtlich der nativen variablen Domäne der ILA TCR α-Kette, die in SEQ ID NO: 1 gezeigt ist, und/oder der variablen Domäne der β-Kette, die in SEQ ID NO: 2 gezeigt ist, in mindestens einer komplementaritätsbestimmenden Region mutiert ist, und
(ii) eine $K_D$ für diesen ILAKFLHWL-HLA-A*0201-Komplex von weniger als oder gleich 1 μM und/oder eine Off-Rate ($k_{off}$) für den ILAKFLHWL-HLA-A*0201-Komplex von $1 \times 10^{-3}$ $s^{-1}$ oder langsamer hat.

**2.** TCR wie in Anspruch 1 beansprucht, der, bezogen auf die variable Domäne der α-Kette des nativen IL TCRs, die in SEQ ID NO: 1 gezeigt ist, und/oder die variable Domäne der β-Kette, die in SEQ ID NO: 2 gezeigt ist, in mindestens einem Rahmenbereich der variablen Domäne mutiert ist.

**3.** TCR, wie in Anspruch 1 oder Anspruch 2 beansprucht, umfassend eine oder mehrere der Aminosäuren 94A, 94L, 94Q, 94T, 94G, 94R, 94M, 94H, 94K, 94E, 94N, 95L, 95P, 95I, 95V, 95M, 96P, 96M, 97F, 98G oder 98A der variablen Domäne der α-Kette, unter Verwendung der Numerierung, die in SEQ ID NO: 1 gezeigt ist.

**4.** TCR, wie in irgendeinem der vorhergehenden Ansprüche beansprucht, umfassend eine oder mehrere der Aminosäuren 19V, 50I, 50L, 50R, 51H, 51C, 51W, 52P, 52W, 52E, 52V, 53E, 53S, 53F, 54Y, 54V, 54C, 54E, 54D, 92G, 931, 93L, 93V, 94Q und 95P der variablen Domäne der β-Kette, unter Verwendung der Numerierung, die in SEQ ID NO: 2 gezeigt ist.

**5.** TCR, wie in irgendeinem der vorhergehenden Ansprüche beansprucht, umfassend eine der Aminosäuresequenzen der variablen Domänen der α-Kette, die in SEQ ID NO: 11 bis 22 oder 50 bis 60 gezeigt sind, gegebenenfalls umfassend eine oder mehrere phänotypisch stumme Substitutionen.

**6.** TCR, wie in irgendeinem der vorhergehenden Ansprüche beansprucht, umfassend eine der Aminosäuresequenzen der variablen Domänen der β-Kette, die in SEQ ID NO: 23 bis 31 oder 61 bis 70 gezeigt sind, gegebenenfalls umfassend eine oder mehrere phänotypisch stumme Substitutionen.

**7.** TCR, wie in irgendeinem der vorhergehenden Ansprüche beansprucht, umfassend die Paarungen der variablen Domäne der α- und β-Kette, die in der folgenden Tabelle gezeigt sind, gegebenenfalls umfassend eine oder mehrere phänotypisch stumme Substitutionen:

| Sequenz der variablen Domäne der α-Kette SEQ ID NO: | Sequenz der variablen Domäne der β-Kette SEQ ID NO: |
|---|---|
| 1 | 23 |
| 1 | 24 |
| 1 | 25 |
| 1 | 26 |
| 1 | 27 |
| 1 | 28 |
| 1 | 29 |
| 1 | 30 |
| 1 | 31 |
| 11 | 2 |
| 12 | 2 |
| 13 | 2 |
| 14 | 2 |
| 15 | 2 |
| 16 | 2 |
| 17 | 2 |
| 18 | 2 |
| 19 | 2 |
| 20 | 2 |
| 21 | 2 |
| 22 | 2 |
| 50 | 2 |
| 51 | 2 |
| 52 | 2 |
| 53 | 2 |
| 54 | 2 |
| 55 | 2 |
| 56 | 2 |
| 57 | 2 |
| 58 | 2 |
| 59 | 2 |
| 60 | 2 |
| 17 | 25 |
| 17 | 23 |
| 12 | 23 |

(fortgesetzt)

| Sequenz der variablen Domäne der α-Kette SEQ ID NO: | Sequenz der variablen Domäne der β-Kette SEQ ID NO: |
|---|---|
| 16 | 23 |
| 14 | 23 |
| 12 | 25 |
| 15 | 23 |
| 19 | 23 |
| 20 | 23 |
| 11 | 61 |
| 11 | 62 |
| 11 | 63 |
| 11 | 64 |
| 11 | 65 |
| 14 | 65 |
| 16 | 66 |
| 22 | 65 |
| 20 | 31 |
| 17 | 31 |
| 17 | 66 |
| 18 | 65 |
| 19 | 65 |
| 17 | 65 |
| 21 | 65 |
| 11 | 31 |
| 19 | 31 |
| 16 | 31 |
| 12 | 31 |
| 11 | 66 |
| 17 | 67 |
| 11 | 67 |
| 15 | 66 |
| 15 | 68 |
| 11 | 67 |
| 15 | 8 |
| 11 | 69 |
| 15 | 70 |
| 15 | 69 |
| 17 | 69 |

(fortgesetzt)

| Sequenz der variablen Domäne der α-Kette SEQ ID NO: | Sequenz der variablen Domäne der β-Kette SEQ ID NO: |
|---|---|
| 11 | 70 |
| 17 | 70 |
| 11 | 28 |
| 11 | 23 |

**8.** TCR, wie in irgendeinem der Ansprüche 1 bis 4 beansprucht, umfassend die variable Domäne der α-Kette, die in SEQ ID NO: 11 gezeigt ist, und die variable Domäne der β-Kette, die in SEQ ID NO: 23 gezeigt ist, gegebenenfalls umfassend eine oder mehrere phänotypisch stumme Substitutionen.

**9.** TCR, wie in irgendeinem der vorhergehenden Ansprüche beansprucht, ferner umfassend die Aminosäuresequenz der konstanten Region der α-Kette, die in SEQ ID NO: 32 gezeigt ist, und/oder eine der Sequenzen der konstanten Region der Aminosäure der β-Kette, die in SEQ ID NO: 33 und 34 gezeigt ist, gegebenenfalls umfassend eine oder mehrere phänotypisch stumme Substitutionen.

**10.** TCR, wie in irgendeinem der vorhergehenden Ansprüche beansprucht, der ein dimerer TCR (dTCR) ist, umfassend:

ein erstes Polypeptid, wobei eine Sequenz, die einer Sequenz einer variablen Domäne der TCR α-Kette entspricht, mit dem N-Terminus einer Sequenz fusioniert ist, die der extrazellulären Sequenz der konstanten Domäne der TCR α-Kette entspricht, und
ein zweites Polypeptid, wobei eine Sequenz, die einer Sequenz einer variablen Domäne der TCR β-Kette entspricht, mit dem N-Terminus einer Sequenz fusioniert ist, die der extrazellulären Sequenz der konstanten Domäne der TCR β-Kette entspricht,

wobei die Sequenzen dieser konstanten Domänen der ersten und zweiten Polypeptide durch eine Disulfidbindung verbunden sind, die kein Äquivalent in nativen αβ-T-Zell-Rezeptoren zwischen den Cysteinresten hat, die Thr 48 des exon 1 von TRAC*01 und Ser 57 des exon 1 von TRBC1*01 oder TRBC2*01 oder das nicht-humane Äquivalent davon substituieren.

**11.** TCR, wie in Anspruch 1 beansprucht, bestehend aus der α-Kette der Aminosäuresequenz SEQ ID NO: 71 und der β-Kette der Aminosäuresequenz SEQ ID NO: 73.

**12.** TCR, wie in irgendeinem der vorhergehenden Ansprüche beansprucht, wobei der TCR mit mindestens einer Polyalkylenglykolkette assoziiert ist.

**13.** TCR, wie in irgendeinem der vorhergehenden Ansprüche beansprucht, wobei ein therapeutisches Mittel oder ein nachweisbarer Rest kovalent mit dem C-Terminus einer der beiden TCR-Ketten verbunden ist.

**14.** TCR, wie in Anspruch 13 beansprucht, wobei das therapeutische Mittel ausgewählt ist aus:

(i) einem Immuneffektormolekül;
(ii) einem cytotoxischen Mittel; und
(iii) Radionuklid.

**15.** TCR, wie in Anspruch 13 oder 14 beansprucht, wobei das therapeutische Mittel IL-2 ist.

**16.** Multivalenter TCR-Komplex, umfassend mindestens zwei TCRs, die in irgendeinem der vorhergehenden Ansprüche beansprucht wurden.

**17.** Isolierte Zelle, die einen TCR, der in irgendeinem der Ansprüche 1 bis 10 definiert ist, präsentiert.

**18.** Pharmazeutische Zusammensetzung, umfassend einen TCR oder einen multivalenten TCR-Komplex, wie in irgendeinem der Ansprüche 1 bis 16 beansprucht, oder eine Vielzahl von Zellen, wie in Anspruch 17 beansprucht, zusammen mit einem pharmazeutisch annehmbaren Träger.

**19.** Verwendung eines TCRs oder eines multivalenten TCR-Komplexes, wie in irgendeinem der Ansprüche 1 bis 16 beansprucht, oder einer Vielzahl von Zellen, wie in Anspruch 17 beansprucht, bei der Herstellung einer Zusammensetzung zur Behandlung von Krebs.

**Revendications**

**1.** Récepteur des cellules T (TCR) ayant la propriété de se lier à ILAKFLHWL-HLA-A*0201 et comprenant au moins un domaine variable de la chaîne $\alpha$ du TCR et/ou au moins un domaine variable de la chaîne $\beta$ du TCR, **caractérisé en ce que** ledit TCR :

(i) est muté par rapport au domaine variable de la chaîne $\alpha$ du TCR ILA natif présenté dans la SEQ ID n° : 1 et/ou au domaine variable de la chaîne $\beta$ présenté dans la SEQ ID n° : 2 dans au moins une région déterminant la complémentarité, et
(ii) a un $K_D$ pour ledit complexe ILAKFLHWL-HLA-A*0201 inférieur ou égal à 1 $\mu$M et/ou a une constante de vitesse de dissociation ($k_{off}$) pour le complexe ILAKFLHWL-HLA-A*0201 de 1 x $10^{-3}$ $s^{-1}$ ou moins.

**2.** TCR selon la revendication 1, qui est muté par rapport au domaine variable de la chaîne $\alpha$ du TCR ILA natif présenté dans la SEQ ID n° : 1 et/ou au domaine variable de la chaîne $\beta$ présenté dans la SEQ ID n° : 2 dans au moins une région charpente de domaine variable.

**3.** TCR selon la revendication 1 ou 2, comprenant un ou plusieurs des acides aminés du domaine variable de la chaîne alpha 94A, 94L, 94Q, 94T, 94G, 94R, 94M, 94H, 94K, 94E, 94N, 95L, 95P, 95I, 95V, 95M, 96P, 96M, 97F, 98G et 98A, en utilisant la numérotation présentée dans la SEQ ID n° : 1.

**4.** TCR selon l'une quelconque des revendications précédentes, comprenant un ou plusieurs des acides aminés du domaine variable de la chaîne bêta 19V, 50I, 50L, 50R, 51H, 51C, 51W, 52P, 52W, 52E, 52V, 53E, 53S, 53F, 54Y, 54V, 54C, 54E, 54D, 92G, 93I, 93L, 93V, 94Q et 95P, en utilisant la numérotation présentée dans la SEQ ID n° : 2.

**5.** TCR selon l'une quelconque des revendications précédentes, comprenant une des séquences d'acides aminés du domaine variable de la chaîne alpha présentées dans les SEQ ID n° : 11 à 22 ou 50 à 60, et comprenant éventuellement une ou plusieurs substitutions phénotypiquement silencieuses.

**6.** TCR selon l'une quelconque des revendications précédentes, comprenant une des séquences d'acides aminés du domaine variable de la chaîne bêta présentées dans les SEQ ID n° : 23 à 31 ou 61 à 70, et comprenant éventuellement une ou plusieurs substitutions phénotypiquement silencieuses.

**7.** TCR selon l'une quelconque des revendications précédentes, comprenant les appariements de domaines variables des chaînes alpha et bêta présentés dans le tableau suivant, et comprenant éventuellement une ou plusieurs substitutions phénotypiquement silencieuses :

| Séquence du domaine variable de la chaîne alpha, SEQ ID n° : | Séquence du domaine variable de la chaîne bêta, SEQ ID n° : |
|---|---|
| 1 | 23 |
| 1 | 24 |
| 1 | 25 |
| 1 | 26 |
| 1 | 27 |
| 1 | 28 |
| 1 | 29 |

(suite)

| Séquence du domaine variable de la chaîne alpha, SEQ ID n° : | Séquence du domaine variable de la chaîne bêta, SEQ ID n° : |
|---|---|
| 1 | 30 |
| 1 | 31 |
| 11 | 2 |
| 12 | 2 |
| 13 | 2 |
| 14 | 2 |
| 15 | 2 |
| 16 | 2 |
| 17 | 2 |
| 18 | 2 |
| 19 | 2 |
| 20 | 2 |
| 21 | 2 |
| 22 | 2 |
| 50 | 2 |
| 51 | 2 |
| 52 | 2 |
| 53 | 2 |
| 54 | 2 |
| 55 | 2 |
| 56 | 2 |
| 57 | 2 |
| 58 | 2 |
| 59 | 2 |
| 60 | 2 |
| 17 | 25 |
| 17 | 23 |
| 12 | 23 |
| 16 | 23 |
| 14 | 23 |
| 12 | 25 |
| 15 | 23 |
| 19 | 23 |
| 20 | 23 |
| 11 | 61 |
| 11 | 62 |
| 11 | 63 |

(suite)

| Séquence du domaine variable de la chaîne alpha, SEQ ID n° : | Séquence du domaine variable de la chaîne bêta, SEQ ID n° : |
|---|---|
| 11 | 64 |
| 11 | 65 |
| 14 | 65 |
| 16 | 66 |
| 22 | 65 |
| 20 | 31 |
| 17 | 31 |
| 17 | 66 |
| 18 | 65 |
| 19 | 65 |
| 17 | 65 |
| 21 | 65 |
| 11 | 31 |
| 19 | 31 |
| 16 | 31 |
| 12 | 31 |
| 11 | 66 |
| 17 | 67 |
| 11 | 67 |
| 15 | 66 |
| 15 | 68 |
| 11 | 67 |
| 15 | 8 |
| 11 | 69 |
| 15 | 70 |
| 15 | 69 |
| 17 | 69 |
| 11 | 70 |
| 17 | 70 |
| 11 | 28 |
| 11 | 23 |

**8.** TCR selon l'une quelconque des revendications 1 à 4, comprenant le domaine variable de la chaîne alpha présenté dans la SEQ ID n° : 11 et le domaine variable de la chaîne bêta présenté dans la SEQ ID n° : 23, et comprenant éventuellement une ou plusieurs substitutions phénotypiquement silencieuses.

**9.** TCR selon l'une quelconque des revendications précédentes, comprenant en outre la séquence d'acides aminés de la région constante de la chaîne alpha présentée dans la SEQ ID n° : 32 et/ou une des séquences d'acides aminés de la région constante de la chaîne bêta présentées dans les SEQ ID n° : 33 et 34, et comprenant éventuellement une ou plusieurs substitutions phénotypiquement silencieuses.

**10.** TCR selon l'une quelconque des revendications précédentes, qui est un TCR dimère (dTCR) comprenant :

- un premier polypeptide dans lequel une séquence correspondant à une séquence du domaine variable de la chaîne α du TCR est condensée avec l'extrémité N-terminale d'une séquence correspondant à une séquence extracellulaire du domaine constant de la chaîne α du TCR, et
- un second polypeptide dans lequel une séquence correspondant à une séquence du domaine variable de la chaîne β du TCR est condensée avec l'extrémité N-terminale d'une séquence correspondant à une séquence extracellulaire du domaine constant de la chaîne β du TCR,

lesdites séquences de domaines constants des premier et second polypeptides étant liées par une liaison disulfure qui n'a pas d'équivalent dans les récepteurs de cellules T αβ natifs, entre les résidus cystéine remplaçant Thr 48 de l'exon 1 de TRAC*01 et Ser 57 de l'exon 1 de TRBC*01 ou de TRBC2*01 ou de l'équivalent non-humain de celui-ci.

**11.** TCR selon la revendication 1, constitué de la séquence d'acides aminés de la chaîne alpha de la SEQ ID n° : 71 et de la séquence d'acides aminés de la chaîne bêta de la SEQ ID n° : 73.

**12.** TCR selon l'une quelconque des revendications précédentes, dans lequel le TCR est associé avec au moins une chaîne polyalkylèneglycol.

**13.** TCR selon l'une quelconque des revendications précédentes, dans lequel un agent thérapeutique ou un motif détectable est lié de façon covalente à l'extrémité C-terminale de l'une ou des deux chaînes du TCR.

**14.** TCR selon la revendication 13, dans lequel l'agent thérapeutique est choisi parmi :

(i) une molécule d'effecteur immun ;
(ii) un agent cytotoxique ; et
(iii) un radionucléide.

**15.** TCR selon la revendication 13 ou 14, dans lequel l'agent thérapeutique est l'IL-2.

**16.** Complexe de TCR multivalent comprenant au moins deux TCR selon l'une quelconque des revendications précédentes.

**17.** Cellule isolée présentant un TCR selon l'une quelconque des revendications 1 à 10.

**18.** Composition pharmaceutique comprenant un TCR ou un complexe de TCR multivalent selon l'une quelconque des revendications 1 à 16, ou une pluralité de cellules selon la revendication 17, conjointement avec un support acceptable sur le plan pharmaceutique.

**19.** Utilisation d'un TCR ou d'un complexe de TCR multivalent selon l'une quelconque des revendications 1 à 16, ou d'une pluralité de cellules selon la revendication 17, dans la préparation d'une composition destinée au traitement du cancer.

**Figure 1a**

```
                  10                  20
                  *                   *
G I Q V E Q S P P D L I L Q E G A N S T L R C N F S D S V

30                  40                  50
*                   *                   *
N N L Q W F H Q N P W G Q L I N L F Y I P S G T K Q N G R

  60                  70                  80
  *                   *                   *
L S A T T V A T E R Y S L L Y I S S S Q T T D S G V Y F C

    90                  100                 110
    *                   *                   *
A V D S A T S G T Y K Y I F G T G T R L K V L A
```

(SEQ ID No: 1)

**Figure 1b**

```
                  10                  20
                  *                   *
N A G V T Q T P K F Q V L K T G Q S M T L Q C A Q D M N H

30                  40                  50
*                   *                   *
E Y M S W Y R Q D P G M G L R L I H Y S V G A G I T D Q G

 60                  70                  80
  *                   *                   *
E V P N G Y N V S R S T T E D F P L R L L S A A P S Q T S

    90                  100
    *                   *
V Y F C A S S Y Q G T E A F F G Q G T R L T V V
```

(SEQ ID No: 2)

## Figure 2a

ggaatacaagtggagcagagtcctccagacctgattctccaggagggagccaattccacgctgcggtgcaatttttctgact
ctgtgaacaatttgcagtggtttcatcaaaacccttggggacagctcatcaacctgttttacattccctcagggacaaaacaga
atggaagattaagcgccacgactgtcgctacggaacgctacagcttattgtacatttcctcttcccagaccacagactcaggc
gtttatttctgtgctgtggactctgctacctcaggaacctacaaatacatctttggaacaggcaccaggctgaaggttttagcaa
atatccagaaccctgaccctgccgtgtaccagctgagagactctaaatccagtgacaagtctgtctgcctattcaccgattttg
attctcaaacaaatgtgtcacaaagtaaggattctgatgtgtatatcacagacaaaactgtgctagacatgaggtctatggactt
caagagcaacagtgctgtggcctggagcaacaaatctgactttgcatgtgcaaacgccttcaacaacagcattattccagaa
gacaccttcttccccagcccagaaagttcctaa
(SEQ ID No: 3)

## Figure 2b

aacgctggtgtcactcagaccccaaaattccaggtcctgaagacaggacagagcatgacactgcagtgtgcccaggatatg
aaccatgaatacatgtcctggtatcgacaagacccaggcatggggctgaggctgattcattactcagttggtgctggtatcac
tgaccaaggagaagtccccaatggctacaatgtctccagatcaaccacagaggatttcccgctcaggctgctgtcggctgct
ccctcccagacatctgtgtacttctgtgccagcagttaccaaggcactgaagctttctttggacaaggcaccagactcacagtt
gtagaggacctgaaaaacgtgttcccacccgaggtcgctgtgtttgagccatcagaagcagagatctcccacacccaaaag
gccacactggtgtgcctggccacaggcttctaccccgaccacgtggagctgagctggtgggtgaatgggaaggaggtgc
acagtggggtcagcacagacccgcagcccctcaaggagcagcccgccctcaatgactccagatacgctctgagcagccg
cctgagggtctcggccaccttctggcaggacccccgcaaccacttccgctgtcaagtccagttctacgggctctcggagaat
gacgagtggacccaggatagggccaaacccgtcacccagatcgtcagcgccgaggcctggggtagagcagactaa
(SEQ ID No: 4)

**Figure 3a**

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T S G T Y K Y I F G T G T R L K V L A N I Q N P
D P A V Y Q L R D S K S S D K S V C L F T D F D S Q T N V
S Q S K D S D V Y I T D K T V L D M R S M D F K S N S A V
A W S N K S D F A C A N A F N N S I I P E D T F F P S P E
S S
(SEQ ID No: 5)

**Figure 3b**

N A G V T Q T P K F Q V L K T G Q S M T L Q C A Q D M N H
E Y M S W Y R Q D P G M G L R L I H Y S V G A G I T D Q G
E V P N G Y N V S R S T T E D F P L R L L S A A P S Q T S
V Y F C A S S Y Q G T E A F F G Q G T R L T V V E D L K N
V F P P E V A V F E P S E A E I S H T Q K A T L V C L A T
G F Y P D H V E L S W W V N G K E V H S G V S T D P Q P L
K E Q P A L N D S R Y A L S S R L R V S A T F W Q D P R N
H F R C Q V Q F Y G L S E N D E W T Q D R A K P V T Q I V
S A E A W G R A D
(SEQ ID No: 6)

## Figure 4a

ggaatacaagtggagcagagtcctccagacctgattctccaggagggagccaattccacgctgcggtgcaatttttctgact
ctgtgaacaatttgcagtggtttcatcaaaacccttggggacagctcatcaacctgttttacattccctcagggacaaaacaga
atggaagattaagcgccacgactgtcgctacggaacgctacagcttattgtacatttcctcttcccagaccacagactcaggc
gtttatttctgtgctgtggactctgctacctcaggaacctacaaatacatctttggaacaggcaccaggctgaaggttttagcaa
atatccagaaccctgaccctgccgtgtaccagctgagagactctaaatccagtgacaagtctgtctgcctattcaccgattttg
attctcaaacaaatgtgtcacaaagtaaggattctgatgtgtatatcacagacaaatgtgtgctagacatgaggtctatggactt
caagagcaacagtgctgtggcctggagcaacaaatctgactttgcatgtgcaaacgccttcaacaacagcattattccagaa
gacaccttcttccccagcccagaaagttcctaa
(SEQ ID No: 7)

## Figure 4b

aacgctggtgtcactcagaccccaaaattccaggtcctgaagacaggacagagcatgacactgcagtgtgcccaggatatg
aaccatgaatacatgtcctggtatcgacaagacccaggcatggggctgaggctgattcattactcagttggtgctggtatcac
tgaccaaggagaagtccccaatggctacaatgtctccagatcaaccacagaggatttcccgctcaggctgctgtcggctgct
ccctcccagacatctgtgtacttctgtgccagcagttaccaaggcactgaagctttctttggacaaggcaccagactcacagtt
gtagaggacctgaaaaacgtgttcccacccgaggtcgctgtgtttgagccatcagaagcagagatctcccacacccaaaag
gccacactggtgtgcctggccacaggcttctaccccgaccacgtggagctgagctggtgggtgaatgggaaggaggtgc
acagtggggtctgcacagacccgcagcccctcaaggagcagcccgccctcaatgactccagatacgctctgagcagccg
cctgagggtctcggccaccttctggcaggacccccgcaaccacttccgctgtcaagtccagttctacgggctctcggagaat
gacgagtggacccaggatagggccaaacccgtcacccagatcgtcagcgccgaggcctggggtagagcagactaa
(SEQ ID No: 8)

## Figure 5a

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T S G T Y K Y I F G T G T R L K V L A N I Q N P
D P A V Y Q L R D S K S S D K S V C L F T D F D S Q T N V
S Q S K D S D V Y I T D K C V L D M R S M D F K S N S A V
A W S N K S D F A C A N A F N N S I I P E D T F F P S P E
S S

(SEQ ID No: 9)

## Figure 5b

N A G V T Q T P K F Q V L K T G Q S M T L Q C A Q D M N H
E Y M S W Y R Q D P G M G L R L I H Y S V G A G I T D Q G
E V P N G Y N V S R S T T E D F P L R L L S A A P S Q T S
V Y F C A S S Y Q G T E A F F G Q G T R L T V V E D L K N
V F P P E V A V F E P S E A E I S H T Q K A T L V C L A T
G F Y P D H V E L S W W V N G K E V H S G V C T D P Q P L
K E Q P A L N D S R Y A L S S R L R V S A T F W Q D P R N
H F R C Q V Q F Y G L S E N D E W T Q D R A K P V T Q I V
S A E A W G R A D

(SEQ ID No: 10)

# Figure 6

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T A L P Y G Y I F G T G T R L K V L A
(SEQ ID No: 11)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T Q L P Y G Y I F G T G T R L K V L A
(SEQ ID NO: 12)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T T L P Y G Y I F G T G T R L K V L A
(SEQ ID NO: 13)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T G L P Y G Y I F G T G T R L K V L A
(SEQ ID NO: 14)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T T G M F G Y I F G T G T R L K V L A
(SEQ ID NO: 15)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T S L P Y G Y I F G T G T R L K V L A
(SEQ ID NO: 16)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T S P P Y G Y I F G T G T R L K V L A
(SEQ ID NO: 17)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T L P P Y G Y I F G T G T R L K V L A
(SEQ ID NO: 18)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T S I P Y G Y I F G T G T R L K V L A
(SEQ ID No: 19)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T S V P Y G Y I F G T G T R L K V L A
(SEQ ID No: 20)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T S G M F G Y I F G T G T R L K V L A
(SEQ ID No: 21)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T T M P Y G Y I F G T G T R L K V L A
(SEQ ID No: 22)

# Figure 7

N A G V T Q T P K F Q V L K T G Q S M T L Q C A Q D M N H
E Y M S W Y R Q D P G M G L R L I H Y S V G A G I T D Q G
E V P N G Y N V S R S T T E D F P L R L L S A A P S Q T S
V Y F C G I Q P Q G T E A F F G Q G T R L T V V
(SEQ ID No: 23)

N A G V T Q T P K F Q V L K T G Q S M T L Q C A Q D M N H
E Y M S W Y R Q D P G M G L R L I H Y S V G A G I T D Q G
E V P N G Y N V S R S T T E D F P L R L L S A A P S Q T S
V Y F C G L Q P Q G T E A F F G Q G T R L T V V
(SEQ ID No: 24)

N A G V T Q T P K F Q V L K T G Q S M T L Q C A Q D M N H
E Y M S W Y R Q D P G M G L R L I H Y S V G A G I T D Q G
E V P N G Y N V S R S T T E D F P L R L L S A A P S Q T S
V Y F C G V Q P Q G T E A F F G Q G T R L T V V
(SEQ ID No: 25)

N A G V T Q T P K F Q V L K T G Q S V T L Q C A Q D M N H
E Y M S W Y R Q D P G M G L R L I H Y S I H P E Y T D Q G
E V P N G Y N V S R S T T E D F P L R L L S A A P S Q T S
V Y F C A S S Y Q G T E A F F G Q G T R L T V V
(SEQ ID No: 26 )

N A G V T Q T P K F Q V L K T G Q S M T L Q C A Q D M N H
E Y M S W Y R Q D P G M G L R L I H Y S L H P S V T D Q G
E V P N G Y N V S R S T T E D F P L R L L S A A P S Q T S
V Y F C A S S Y Q G T E A F F G Q G T R L T V V
(SEQ ID No: 27)

N A G V T Q T P K F Q V L K T G Q S M T L Q C A Q D M N H
E Y M S W Y R Q D P G M G L R L I H Y S I C P S C T D Q G
E V P N G Y N V S R S T T E D F P L R L L S A A P S Q T S
V Y F C A S S Y Q G T E A F F G Q G T R L T V V
(SEQ ID No: 28)

N A G V T Q T P K F Q V L K T G Q S M T L Q C A Q D M N H
E Y M S W Y R Q D P G M G L R L I H Y S I C W G C T D Q G
E V P N G Y N V S R S T T E D F P L R L L S A A P S Q T S
V Y F C A S S Y Q G T E A F F G Q G T R L T V V
(SEQ ID No: 29)

N A G V T Q T P K F Q V L K T G Q S M T L Q C A Q D M N H
E Y M S W Y R Q D P G M G L R L I H Y S I W E F E T D Q G
E V P N G Y N V S R S T T E D F P L R L L S A A P S Q T S
V Y F C A S S Y Q G T E A F F G Q G T R L T V V
(SEQ ID No: 30)

N A G V T Q T P K F Q V L K T G Q S M T L Q C A Q D M N H
E Y M S W Y R Q D P G M G L R L I H Y S R W V G D T D Q G
E V P N G Y N V S R S T T E D F P L R L L S A A P S Q T S
V Y F C A S S Y Q G T E A F F G Q G T R L T V V
(SEQ ID No: 31)

## Figure 8a

N I Q N P D P A V Y Q L R D S K S S D K S V C L F T
D F D S Q T N V S Q S K D S D V Y I T D K
(SEQ ID NO: 32)

## Figure 8b

E D L N K V F P P E V A V F E P S E A E I S H T Q K A T
L V C L A T G F F P D H V E L S W W V N G K E V H S G V
(SEQ ID NO: 33)

## Figure 8c

E D L K N V F P P E V A V F E P S E A E I S H T Q K A T
L V C L A T G F Y P D H V E L S W W V N G K E V H S G V
(SEQ ID NO: 34)

Figure 9

**PEX954**

gatctcgatcccgcgaaattaatacgactcactatagggagaccacaacggtttccctctagaaataattttgtttaactttaaga
aggagatataatcgatgtctaactcgagtgacaagtctgtctgcctattcaccgattttgattctcaaacaaatgtgtcacaaagt
aaggattctgatgtgtatatcacagacaaatgtgtgctagacatgaggtctatggacttcaagagcaacagtgctgtggcctg
gagcaacaaatctgactttgcatgtgcaaacgccttcaacaacagcattattccagaagacaccttcttccccagcccagaaa
gttcctaagcttgaattccgatccggctgctaacaaagcccgaaaggaagctgagttggctgctgccaccgctgagcaataa
ctagcataaccccttggggcctctaaacgggtcttgaggggttttttgctgaaaggaggaactatatccggataattcttgaag
acgaaagggcctcgtgatacgcctatttttataggttaatgtcatgataataatggtttcttagacgtgaggtggcacttttcggg
gaaatgtgcgcggaacccctatttgtttatttttctaaatacattcaaatatgtatccgctcatgagacaataaccctgataaatgc
ttcaataatattttgttaaaattcgcgttaaatttttgttaaatcagctcattttttaaccaataggccgaaatcggcaaaatcccttat
aaatcaaaagaatagaccgagatagggttgagtgttgttccagtttggaacaagagtccactattaaagaacgtggactccaa
cgtcaaagggcgaaaaaccgtctatcagggcgatggcccactacgtgaaccatcaccctaatcaagttttttggggtcgagg
tgccgtaaagcactaaatcggaaccctaaagggagcccccgatttagagcttgacggggaaagccggcgaacgtggcga
gaaaggaagggaagaaagcgaaaggagcgggcgctagggcgctggcaagtgtagcggtcacgctgcgcgtaaccacc
acacccgccgcgcttaatgcgccgctacagggcgcgtcaggtggcacttttcggggaaatgtgcgcggaacccctatttgtt
tatttttctaaatacattcaaatatgtatccgctcatgagacaataaccctgataaatgcttcaataatattgaaaaaggaagagta
tgagtattcaacatttccgtgtcgcccttattcccttttttgcggcattttgccttcctgtttttgctcacccagaaacgctggtgaaa
gtaaaagatgctgaagatcagttgggtgcacgagtgggttacatcgaactggatctcaacagcggtaagatccttgagagttt
tcgccccgaagaacgttttccaatgatgagcacttttaaagttctgctatgtggcgcggtattatcccgtgttgacgccgggca
agagcaactcggtcgccgcatacactattctcagaatgacttggttgagtactcaccagtcacagaaaagcatcttacggatg
gcatgacagtaagagaattatgcagtgctgccataaccatgagtgataacactgcggccaacttacttctgacaacgatcgg
aggaccgaaggagctaaccgcttttttgcacaacatgggggatcatgtaactcgccttgatcgttgggaaccggagctgaat
gaagccataccaaacgacgagcgtgacaccacgatgcctgcagcaatggcaacaacgttgcgcaaactattaactggcga
actacttactctagcttcccggcaacaattaatagactggatggaggcggataaagttgcaggaccacttctgcgctcggccc
ttccggctggctggtttattgctgataaatctggagccggtgagcgtgggtctcgcggtatcattgcagcactggggccagat
ggtaagccctcccgtatcgtagttatctacacgacggggagtcaggcaactatggatgaacgaaatagacagatcgctgag

ataggtgcctcactgattaagcattggtaactgtcagaccaagtttactcatatatactttagattgatttaaaacttcatttttaattt
aaaaggatctaggtgaagatcctttttgataatctcatgaccaaaatcccttaacgtgagttttcgttccactgagcgtcagacc
ccgtagaaaagatcaaaggatcttcttgagatcctttttttctgcgcgtaatctgctgcttgcaaacaaaaaaaccaccgctacc
agcggtggtttgtttgccggatcaagagctaccaactctttttccgaaggtaactggcttcagcagagcgcagataccaaata
ctgtccttctagtgtagccgtagttaggccaccacttcaagaactctgtagcaccgcctacatacctcgctctgctaatcctgtt
accagtggctgctgccagtggcgataagtcgtgtcttaccgggttggactcaagacgatagttaccggataaggcgcagcg
gtcgggctgaacggggggttcgtgcacacagcccagcttggagcgaacgacctacaccgaactgagatacctacagcgt
gagctatgagaaagcgccacgcttcccgaagggagaaaggcggacaggtatccggtaagcggcagggtcggaacagg
agagcgcacgagggagcttccagggggaaacgcctggtatctttatagtcctgtcgggtttcgccacctctgacttgagcgt
cgatttttgtgatgctcgtcaggggggcggagcctatggaaaaacgccagcaacgcggcctttttacggttcctggccttttg
ctggccttttgctcacatgttctttcctgcgttatcccctgattctgtggataaccgtattaccgcctttgagtgagctgataccgct
cgccgcagccgaacgaccgagcgcagcgagtcagtgagcgaggaagcggaagagcgcctgatgcggtattttctcctta
cgcatctgtgcggtatttcacaccgcaatggtgcactctcagtacaatctgctctgatgccgcatagttaagccagtatacact
ccgctatcgctacgtgactgggtcatggctgcgccccgacacccgccaacacccgctgacgcgccctgacgggcttgtct
gctcccggcatccgcttacagacaagctgtgaccgtctccgggagctgcatgtgtcagaggttttcaccgtcatcaccgaaa
cgcgcgaggcag

(SEQ ID NO: 46)

## Figure 10

### PEX821

gatctcgatcccgcgaaattaatacgactcactatagggagaccacaacggtttccctctagaaataattttgtttaactttaaga
aggagatatacatatgaacgctggtgtcactcagaccccaaaattccaggtcctgaagacaggacagagcatgacactgca
gtgtgcccaggatatgaaccatgaatacatgtcctggtatcgacaagacccaggcatggggctgaggctgattcattactca
gttggtgctggtatcactgaccaaggagaagtccccaatggctacaatgtctccagatcaaccacagaggatttcccgctca
ggctgctgtcggctgctccctcccagacatctgtgtacttctgtgccagcaggccgggactagcgggagggcgaccagag
cagtacttcgggccgggcaccaggctcacggtcacagaggacctgaaaaacgtgttcccacccgaggtcgctgtgtttga
gccatcagaagcagagatctcccacacccaaaaggccacactggtgtgcctggccaccggtttctaccccgaccacgtgg
agctgagctggtgggtgaatgggaaggaggtgcacagtggggtctgcacagacccgcagcccctcaaggagcagcccg
ccctcaatgactccagatacgctctgagcagccgcctgagggtctcggccaccttctggcaggacccccgcaaccacttcc
gctgtcaagtccagttctacgggctctcggagaatgacgagtggacccaggatagggccaaacccgtcacccagatcgtc
agcgccgaggcctggggtagagcagactaagcttgaattccgatccggctgctaacaaagcccgaaaggaagctgagttg
gctgctgccaccgctgagcaataactagcataaccccttggggcctctaaacgggtcttgaggggttttttgctgaaaggag
gaactatatccggataattcttgaagacgaaagggcctcgtgatacgcctatttttataggttaatgtcatgataataatggtttct
tagacgtcaggtggcacttttcggggaaatgtgcgcggaacccctatttgtttatttttctaaatacattcaaatatgtatccgctc
atgagacaataaccctgataaatgcttcaataatattttgttaaaattcgcgttaaatttttgttaaatcagctcattttttaaccaata
ggccgaaatcggcaaaatcccttataaatcaaaagaatagaccgagatagggttgagtgttgttccagtttggaacaagagt
ccactattaaagaacgtggactccaacgtcaaagggcgaaaaaccgtctatcagggcgatggcccactacgtgaaccatca
ccctaatcaagttttttggggtcgaggtgccgtaaagcactaaatcggaaccctaaagggagcccccgatttagagcttgac
ggggaaagccggcgaacgtggcgagaaaggaagggaagaaagcgaaaggagcgggcgctagggcgctggcaagtg
tagcggtcacgctgcgcgtaaccaccacacccgccgcgcttaatgcgccgctacagggcgcgtcaggtggcacttttcgg
ggaaatgtgcgcggaacccctatttgtttatttttctaaatacattcaaatatgtatccgctcatgagacaataaccctgataaatg
cttcaataatattgaaaaaggaagagtatgagtattcaacatttccgtgtcgcccttattcccttttttgcggcattttgccttcctgt
ttttgctcacccagaaacgctggtgaaagtaaaagatgctgaagatcagttgggtgcacgagtgggttacatcgaactggat
ctcaacagcggtaagatccttgagagttttcgccccgaagaacgttttccaatgatgagcacttttaaagttctgctatgtggcg
cggtattatcccgtgttgacgccgggcaagagcaactcggtcgccgcatacactattctcagaatgacttggttgagtactca
ccagtcacagaaaagcatcttacggatggcatgacagtaagagaattatgcagtgctgccataaccatgagtgataacactg
cggccaacttacttctgacaacgatcggaggaccgaaggagctaaccgcttttttgcacaacatgggggatcatgtaactcg
ccttgatcgttgggaaccggagctgaatgaagccataccaaacgacgagcgtgacaccacgatgcctgcagcaatggcaa
caacgttgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgggtctcg
cggtatcattgcagcactggggccagatggtaagccctcccgtatcgtagttatctacacgacggggagtcaggcaactatg
gatgaacgaaatagacagatcgctgagataggtgcctcactgattaagcattggtaactgtcagaccaagtttactcatatata
ctttagattgatttaaaacttcatttttaatttaaaaggatctaggtgaagatcctttttgataatctcatgaccaaaatcccttaacgt
gagttttcgttccactgagcgtcagaccccgtagaaaagatcaaaggatcttcttgagatcctttttttctgcgcgtaatctgctg
cttgcaaacaaaaaaaccaccgctaccagcggtggtttgtttgccggatcaagagctaccaactctttttccgaaggtaactg
gcttcagcagagcgcagataccaaatactgtccttctagtgtagccgtagttaggccaccacttcaagaactctgtagcaccg
cctacatacctcgctctgctaatcctgttaccagtggctgctgccagtggcgataagtcgtgtcttaccgggttggactcaaga
cgatagttaccggataaggcgcagcggtcgggctgaacggggggttcgtgcacacagcccagcttggagcgaacgacct
acaccgaactgagatacctacagcgtgagctatgagaaagcgccacgcttcccgaagggagaaaggcggacaggtatcc

ggtaagcggcagggtcggaacaggagagcgcacgagggagcttccagggggaaacgcctggtatctttatagtcctgtc
gggtttcgccacctctgacttgagcgtcgatttttgtgatgctcgtcaggggggcggagcctatggaaaaacgccagcaacg
cggcctttttacggttcctggccttttgctggccttttgctcacatgttctttcctgcgttatcccctgattctgtggataaccgtatta
ccgcctttgagtgagctgataccgctcgccgcagccgaacgaccgagcgcagcgagtcagtgagcgaggaagcggaag
agcgcctgatgcggtattttctccttacgcatctgtgcggtatttcacaccgcaatggtgcactctcagtacaatctgctctgatg
ccgcatagttaagccagtatacactccgctatcgctacgtgactgggtcatggctgcgccccgacacccgccaacacccgc
tgacgcgccctgacgggcttgtctgctcccggcatccgcttacagacaagctgtgaccgtctccgggagctgcatgtgtcag
aggttttcaccgtcatcaccgaaacgcgcgaggcag
(SEQ ID NO: 47)

## Figure 11

**pEX202**

gatctcgatcccgcgaaattaatacgactcactatagggagaccacaacggtttccctctagaaataattttgtttaactttaaga
aggagatatacatatgcagaaggaagtggagcagaactctggacccctcagtgttccagagggagccattgcctctctcaa
ctgcacttacagtgaccgaggttcccagtccttcttctggtacagacaatattctgggaaaagccctgagttgataatgtccata
tactccaatggtgacaaagaagatggaaggtttacagcacagctcaataaagccagccagtatgtttctctgctcatcagaga
ctcccagcccagtgattcagccacctacctctgtgccgttacaactgacagctgggggaaattgcagtttggagcagggacc
caggttgtggtcaccccagatatccagaaccctgaccctgccgtgtaccagctgagagactctaaatccagtgacaagtctg
tctgcctattcaccgattttgattctcaaacaaatgtgtcacaaagtaaggattctgatgtgtatatcacagacaaaactgtgcta
gacatgaggtctatggacttcaagagcaacagtgctgtggcctggagcaacaaatctgactttgcatgtgcaaacgccttca
acaacagcattattccagaagacaccttcttccccagcccagaaagttcccccgggggtagaatcgcccggctggaggaa
aaagtgaaaaccttgaaagctcagaactcggagctggcgtccacggccaacatgctcagggaacaggtggcacagcttaa
acagaaagtcatgaactactaggatccatggtaagcttgaattccgatccggctgctaacaaagcccgaaaggaagctgag
ttggctgctgccaccgctgagcaataactagcataaccccttggggcctctaaacgggtcttgaggggttttttgctgaaagg
aggaactatatccggataattcttgaagacgaaagggcctcgtgatacgcctatttttataggttaatgtcatgataataatggtt
tcttagacgtcaggtggcacttttcggggaaatgtgcgcggaacccctatttgtttatttttctaaatacattcaaatatgtatccg
ctcatgagacaataaccctgataaatgcttcaataatattttgttaaaattcgcgttaaatttttgttaaatcagctcattttttaacca
ataggccgaaatcggcaaaatcccttataaatcaaaagaatagaccgagatagggttgagtgttgttccagtttggaacaaga
gtccactattaaagaacgtggactccaacgtcaaagggcgaaaaaccgtctatcagggcgatggcccactacgtgaaccat
caccctaatcaagttttttggggtcgaggtgccgtaaagcactaaatcggaaccctaaagggagcccccgatttagagcttg
acggggaaagccggcgaacgtggcgagaaaggaagggaagaaagcgaaaggagcgggcgctagggcgctggcaag
tgtagcggtcacgctgcgcgtaaccaccacacccgccgcgcttaatgcgccgctacagggcgcgtcaggtggcacttttcg
gggaaatgtgcgcggaacccctatttgtttatttttctaaatacattcaaatatgtatccgctcatgagacaataaccctgataaat
gcttcaataatattgaaaaaggaagagtatgagtattcaacatttccgtgtcgcccttattcccttttttgcggcattttgccttcct
gtttttgctcacccagaaacgctggtgaaagtaaaagatgctgaagatcagttgggtgcacgagtgggttacatcgaactgg
atctcaacagcggtaagatccttgagagttttcgccccgaagaacgttttccaatgatgagcacttttaaagttctgctatgtgg
cgcggtattatcccgtgttgacgccgggcaagagcaactcggtcgccgcatacactattctcagaatgacttggttgagtact
caccagtcacagaaaagcatcttacggatggcatgacagtaagagaattatgcagtgctgccataaccatgagtgataacac
tgcggccaacttacttctgacaacgatcggaggaccgaaggagctaaccgcttttttgcacaacatgggggatcatgtaact
cgccttgatcgttgggaaccggagctgaatgaagccataccaaacgacgagcgtgacaccacgatgcctgcagcaatggc
aacaacgttgcgcaaactattaactggcgaactacttactctagcttcccggcaacaattaatagactggatggaggcggata
aagttgcaggaccacttctgcgctcggcccttccggctggctggtttattgctgataaatctggagccggtgagcgtgggtct
cgcggtatcattgcagcactggggccagatggtaagccctcccgtatcgtagttatctacacgacggggagtcaggcaact
atggatgaacgaaatagacagatcgctgagataggtgcctcactgattaagcattggtaactgtcagaccaagtttactcatat
atactttagattgatttaaaacttcatttttaatttaaaaggatctaggtgaagatcctttttgataatctcatgaccaaaatcccttaa
cgtgagttttcgttccactgagcgtcagaccccgtagaaaagatcaaaggatcttcttgagatcctttttttctgcgcgtaatctg
ctgcttgcaaacaaaaaaaccaccgctaccagcggtggtttgtttgccggatcaagagctaccaactctttttccgaaggtaa
ctggcttcagcagagcgcagataccaaatactgtccttctagtgtagccgtagttaggccaccacttcaagaactctgtagca
ccgcctacatacctcgctctgctaatcctgttaccagtggctgctgccagtggcgataagtcgtgtcttaccgggttggactca
agacgatagttaccggataaggcgcagcggtcgggctgaacggggggttcgtgcacacagcccagcttggagcgaacg
acctacaccgaactgagatacctacagcgtgagctatgagaaagcgccacgcttcccgaagggagaaaggcggacaggt
atccggtaagcggcagggtcggaacaggagagcgcacgagggagcttccagggggaaacgcctggtatctttatagtcct

gtcgggtttcgccacctctgacttgagcgtcgatttttgtgatgctcgtcaggggggcggagcctatggaaaaacgccagca
acgcggcctttttacggttcctggccttttgctggccttttgctcacatgttctttcctgcgttatcccctgattctgtggataaccgt
attaccgcctttgagtgagctgataccgctcgccgcagccgaacgaccgagcgcagcgagtcagtgagcgaggaagcgg
aagagcgcctgatgcggtattttctccttacgcatctgtgcggtatttcacaccgcaatggtgcactctcagtacaatctgctct
gatgccgcatagttaagccagtatacactccgctatcgctacgtgactgggtcatggctgcgccccgacacccgccaacac
ccgctgacgcgccctgacgggcttgtctgctcccggcatccgcttacagacaagctgtgaccgtctccgggagctgcatgt
gtcagaggttttcaccgtcatcaccgaaacgcgcgaggcag
(SEQ ID NO: 48)

## Figure 12

**pEX205**

gatctcgatcccgcgaaattaatacgactcactatagggagaccacaacggtttccctctagaaataattttgtttaactttaagaaggagat
atacatatgaacgctggtgtcactcagaccccaaaattccaggtcctgaagacaggacagagcatgacactgcagtgtgcccaggatat
gaaccatgaatacatgtcctggtatcgacaagacccaggcatggggctgaggctgattcattactcagttggtgctggtatcactgacca
aggagaagtccccaatggctacaatgtctccagatcaaccacagaggatttcccgctcaggctgctgtcggctgctccctcccagacat
ctgtgtacttctgtgccagcaggccgggactagcgggagggcgaccagagcagtacttcgggccgggcaccaggctcacggtcaca
gaggacctgaaaaacgtgttcccacccgaggtcgctgtgtttgagccatcagaagcagagatctcccacacccaaaaggccacactgg
tgtgcctggccacaggcttctaccccgaccacgtggagctgagctggtgggtgaatgggaaggaggtgcacagtggggtcagcaca
gacccgcagcccctcaaggagcagcccgccctcaatgactccagatacgctctgagcagccgcctgagggtctcggccaccttctgg
caggacccccgcaaccacttccgctgtcaagtccagttctacgggctctcggagaatgacgagtggacccaggatagggccaaaccc
gtcacccagatcgtcagcgccgaggcctggggtagagcagaccccggggggtctgactgatacactccaagcggagacagatcaactt
gaagacaagaagtctgcgttgcagaccgagattgccaatctactgaaagagaaggaaaaactagagttcatcctggcagcttacggatc
cggtggtggtctgaacgatatttttgaagctcagaaaatcgaatggcattaagcttgaattccgatccggctgctaacaaagcccgaaag
gaagctgagttggctgctgccaccgctgagcaataactagcataaccccttggggcctctaaacgggtcttgaggggttttttgctgaaa
ggaggaactatatccggataattcttgaagacgaaagggcctcgtgatacgcctatttttataggttaatgtcatgataataatggtttcttag
acgtcaggtggcacttttcggggaaatgtgcgcggaacccctatttgtttatttttctaaatacattcaaatatgtatccgctcatgagacaat
aaccctgataaatgcttcaataatattttgttaaaattcgcgttaaatttttgttaaatcagctcattttttaaccaataggccgaaatcggcaaa
atcccttataaatcaaaagaatagaccgagatagggttgagtgttgttccagtttggaacaagagtccactattaaagaacgtggactcca
acgtcaaagggcgaaaaaccgtctatcagggcgatggcccactacgtgaaccatcaccctaatcaagttttttggggtcgaggtgccgt
aaagcactaaatcggaaccctaaagggagcccccgatttagagcttgacggggaaagccggcgaacgtggcgagaaaggaaggga
agaaagcgaaaggagcgggcgctagggcgctggcaagtgtagcggtcacgctgcgcgtaaccaccacacccgccgcgcttaatgc
gccgctacagggcgcgtcaggtggcacttttcggggaaatgtgcgcggaacccctatttgtttatttttctaaatacattcaaatatgtatcc
gctcatgagacaataaccctgataaatgcttcaataatattgaaaaaggaagagtatgagtattcaacatttccgtgtcgcccttattcccttt
tttgcggcattttgccttcctgtttttgctcacccagaaacgctggtgaaagtaaaagatgctgaagatcagttgggtgcacgagtgggtta
catcgaactggatctcaacagcggtaagatccttgagagttttcgccccgaagaacgttttccaatgatgagcacttttaaagttctgctatg
tggcgcggtattatcccgtgttgacgccgggcaagagcaactcggtcgccgcatacactattctcagaatgacttggttgagtactcacc
agtcacagaaaagcatcttacggatggcatgacagtaagagaattatgcagtgctgccataaccatgagtgataacactgcggccaactt
acttctgacaacgatcggaggaccgaaggagctaaccgcttttttgcacaacatgggggatcatgtaactcgccttgatcgttgggaacc
ggagctgaatgaagccataccaaacgacgagcgtgacaccacgatgcctgcagcaatggcaacaacgttgcgcaaactattaactgg
cgaactacttactctagcttcccggcaacaattaatagactggatggaggcggataaagttgcaggaccacttctgcgctcggcccttcc
ggctggctggtttattgctgataaatctggagccggtgagcgtgggtctcgcggtatcattgcagcactggggccagatggtaagccctc
ccgtatcgtagttatctacacgacggggagtcaggcaactatggatgaacgaaatagacagatcgctgagataggtgcctcactgattaa
gcattggtaactgtcagaccaagtttactcatatatactttagattgatttaaaacttcatttttaatttaaaaggatctaggtgaagatcctttttg
ataatctcatgaccaaaatcccttaacgtgagttttcgttccactgagcgtcagaccccgtagaaaagatcaaaggatcttcttgagatcctt
tttttctgcgcgtaatctgctgcttgcaaacaaaaaaaccaccgctaccagcggtggtttgtttgccggatcaagagctaccaactctttttc
cgaaggtaactggcttcagcagagcgcagataccaaatactgtccttctagtgtagccgtagttaggccaccacttcaagaactctgtag
caccgcctacatacctcgctctgctaatcctgttaccagtggctgctgccagtggcgataagtcgtgtcttaccgggttggactcaagacg
atagttaccggataaggcgcagcggtcgggctgaacggggggttcgtgcacacagcccagcttggagcgaacgacctacaccgaac
tgagatacctacagcgtgagctatgagaaagcgccacgcttcccgaagggagaaaggcggacaggtatccggtaagcggcagggtc
ggaacaggagagcgcacgagggagcttccagggggaaacgcctggtatctttatagtcctgtcgggtttcgccacctctgacttgagc
gtcgatttttgtgatgctcgtcaggggggcggagcctatggaaaaacgccagcaacgcggcctttttacggttcctggccttttgctggcc
ttttgctcacatgttctttcctgcgttatcccctgattctgtggataaccgtattaccgcctttgagtgagctgataccgctcgccgcagccga
acgaccgagcgcagcgagtcagtgagcgaggaagcggaagagcgcctgatgcggtattttctccttacgcatctgtgcggtatttcaca
ccgcaatggtgcactctcagtacaatctgctctgatgccgcatagttaagccagtatacactccgctatcgctacgtgactgggtcatggc
tgcgccccgacacccgccaacacccgctgacgcgccctgacgggcttgtctgctcccggcatccgcttacagacaagctgtgaccgtc
tccgggagctgcatgtgtcagaggttttcaccgtcatcaccgaaacgcgcgaggcag (SEQ ID NO: 49)

## Figure 13

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T M P T G K Y I F G T G T R L K V L A
(SEQ ID NO: 50)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T R L P F L Y I F G T G T R L K V L A
(SEQ ID NO: 51)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T Q L P F L Y I F G T G T R L K V L A
(SEQ ID NO: 52)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T M L P Y G Y I F G T G T R L K V L A
(SEQ ID NO: 53)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T A L P F G Y I F G T G T R L K V L A
(SEQ ID NO: 54)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T A L P Y A Y I F G T G T R L K V L A
(SEQ ID NO: 55)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T H L P Y G Y I F G T G T R L K V L A
(SEQ ID NO: 56)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T T P P Y G Y I F G T G T R L K V L A
(SEQ ID NO: 57)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T E M P Y G Y I F G T G T R L K V L A
(SEQ ID NO: 58)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T N L P Y G Y I F G T G T R L K V L A
(SEQ ID NO: 59)

G I Q V E Q S P P D L I L Q E G A N S T L R C N F S D S V
N N L Q W F H Q N P W G Q L I N L F Y I P S G T K Q N G R
L S A T T V A T E R Y S L L Y I S S S Q T T D S G V Y F C
A V D S A T K L P Y G Y I F G T G T R L K V L A
(SEQ ID NO: 60)

# Figure 14

N A G V T Q T P K F Q V L K T G Q S M T L Q C A Q D M N H
E Y M S W Y R Q D P G M G L R L I H Y S I H P E Y T D Q G
E V P N G Y N V S R S T T E D F P L R L L S A A P S Q T S
V Y F C G I Q P Q G T E A F F G Q G T R L T V V
(SEQ ID No: 61)

N A G V T Q T P K F Q V L K T G Q S M T L Q C A Q D M N H
E Y M S W Y R Q D P G M G L R L I H Y S I C P S C T D Q G
E V P N G Y N V S R S T T E D F P L R L L S A A P S Q T S
V Y F C G I Q P Q G T E A F F G Q G T R L T V V
(SEQ ID No: 62)

N A G V T Q T P K F Q V L K T G Q S M T L Q C A Q D M N H
E Y M S W Y R Q D P G M G L R L I H Y S I H P E Y T D Q G
E V P N G Y N V S R S T T E D F P L R L L S A A P S Q T S
V Y F C G V Q P Q G T E A F F G Q G T R L T V V
(SEQ ID No: 63)

N A G V T Q T P K F Q V L K T G Q S M T L Q C A Q D M N H
E Y M S W Y R Q D P G M G L R L I H Y S I C P S C T D Q G
E V P N G Y N V S R S T T E D F P L R L L S A A P S Q T S
V Y F C G V Q P Q G T E A F F G Q G T R L T V V
(SEQ ID No: 64)

N A G V T Q T P K F Q V L K T G Q S M T L Q C A Q D M N H
E Y M S W Y R Q D P G M G L R L I H Y S R W V G D T D Q G
E V P N G Y N V S R S T T E D F P L R L L S A A P S Q T S
V Y F C G V Q P Q G T E A F F G Q G T R L T V V
(SEQ ID No: 65)

N A G V T Q T P K F Q V L K T G Q S M T L Q C A Q D M N H
E Y M S W Y R Q D P G M G L R L I H Y S R W V G D T D Q G
E V P N G Y N V S R S T T E D F P L R L L S A A P S Q T S
V Y F C G I Q P Q G T E A F F G Q G T R L T V V
(SEQ ID No: 66)

N A G V T Q T P K F Q V L K T G Q S M T L Q C A Q D M N H
E Y M S W Y R Q D P G M G L R L I H Y S I C W G C T D Q G
E V P N G Y N V S R S T T E D F P L R L L S A A P S Q T S
V Y F C G I Q P Q G T E A F F G Q G T R L T V V
(SEQ ID No: 67)

N A G V T Q T P K F Q V L K T G Q S M T L Q C A Q D M N H
E Y M S W Y R Q D P G M G L R L I H Y S I W E F E T D Q G
E V P N G Y N V S R S T T E D F P L R L L S A A P S Q T S
V Y F C G I Q P Q G T E A F F G Q G T R L T V V
(SEQ ID No: 68)

N A G V T Q T P K F Q V L K T G Q S M T L Q C A Q D M N H
E Y M S W Y R Q D P G M G L R L I H Y S I W E F E T D Q G
E V P N G Y N V S R S T T E D F P L R L L S A A P S Q T S
V Y F C G V Q P Q G T E A F F G Q G T R L T V V
(SEQ ID No: 69)

N A G V T Q T P K F Q V L K T G Q S M T L Q C A Q D M N H
E Y M S W Y R Q D P G M G L R L I H Y S I C W G C T D Q G
E V P N G Y N V S R S T T E D F P L R L L S A A P S Q T S
V Y F C G V Q P Q G T E A F F G Q G T R L T V V
(SEQ ID No: 70)

## Figure 15a

M G I Q V E Q S P P D L I L Q E G A N S T L R C N F S D S
V N N L Q W F H Q N P W G Q L I N L F Y I P S G T K Q N G
R L S A T T V A T E R Y S L L Y I S S S Q T T D S G V Y F
C A V D S A T A L P Y G Y I F G T G T R L K V L A N I Q N
P D P A V Y Q L R D S K S S D K S V C L F T D F D S Q T N
V S Q S K D S D V Y I T D K C V L D M R S M D F K S N S A
V A W S N K S D F A C A N A F N N S I I P E D T F F P S P
E S S

(SEQ ID No: 71)

## Figure 15b

M N A G V T Q T P K F Q V L K T G Q S M T L Q C A Q D M N
H E Y M S W Y R Q D P G M G L R L I H Y S V G A G I T D Q
G E V P N G Y N V S R S T T E D F P L R L L S A A P S Q T
S V Y F C G I Q P Y Q G T E A F F G Q G T R L T V V E D L
K N V F P P E V A V F E P S E A E I S H T Q K A T L V C L
A T G F Y P D H V E L S W W V N G K E V H S G V C T D P Q
P L K E Q P A L N D S R Y A L S S R L R V S A T F W Q D P
R N H F R C Q V Q F Y G L S E N D E W T Q D R A K P V T Q
I V S A E A W G R A D *P G A P T S S S T K K T Q L Q L E H*
*L L L D L Q M I L N G I N N Y K N P K L T R M L T F K F Y*
*M P K K A T E L K H L Q C L E E E L K P L E E V L N L A Q*
*S K N F H L R P R D L I S N I N V I V L E L K G S E T T F*
*M C E Y A D E T A T I V E F L N R W I T F C Q S I I S T L*
*T*

(SEQ ID No: 72)

Figure 16

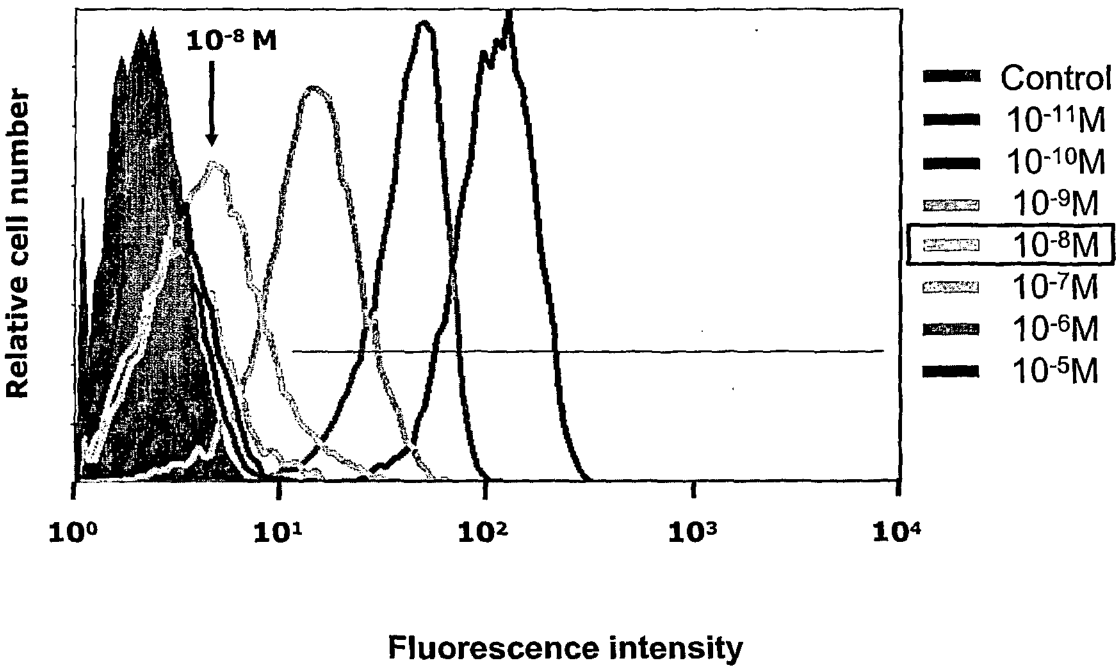
10-8 M
Relative cell number
Control
10-11M
10-10M
10-9M
10-8M
10-7M
10-6M
10-5M
10⁰
10¹
10²
10³
10⁴
Fluorescence intensity

Figure 17

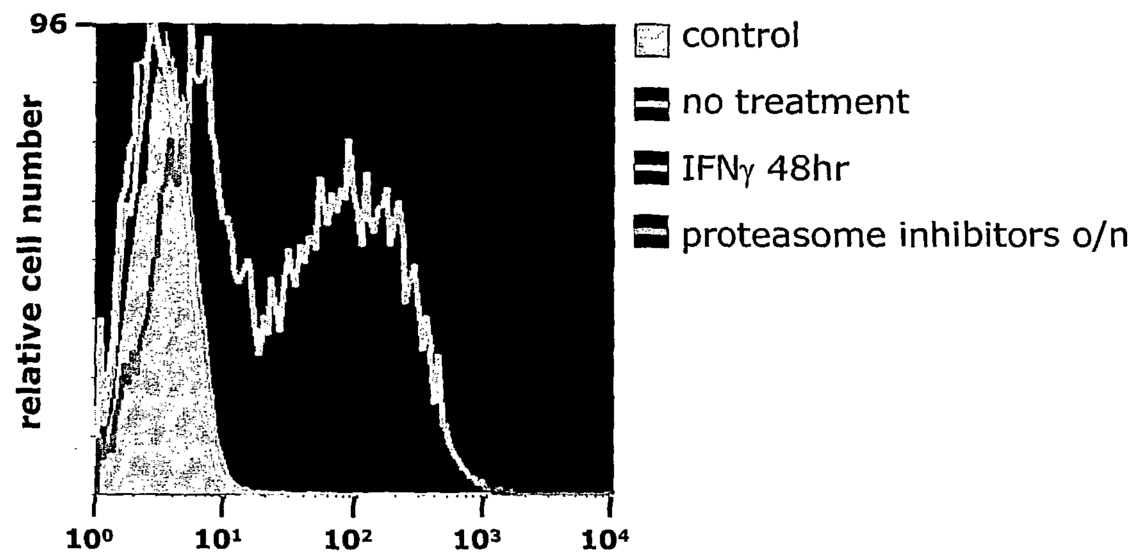
96
relative cell number
10^0
10^1
10^2
10^3
10^4
control
no treatment
IFNγ 48hr
proteasome inhibitors o/n

**Figure 18**

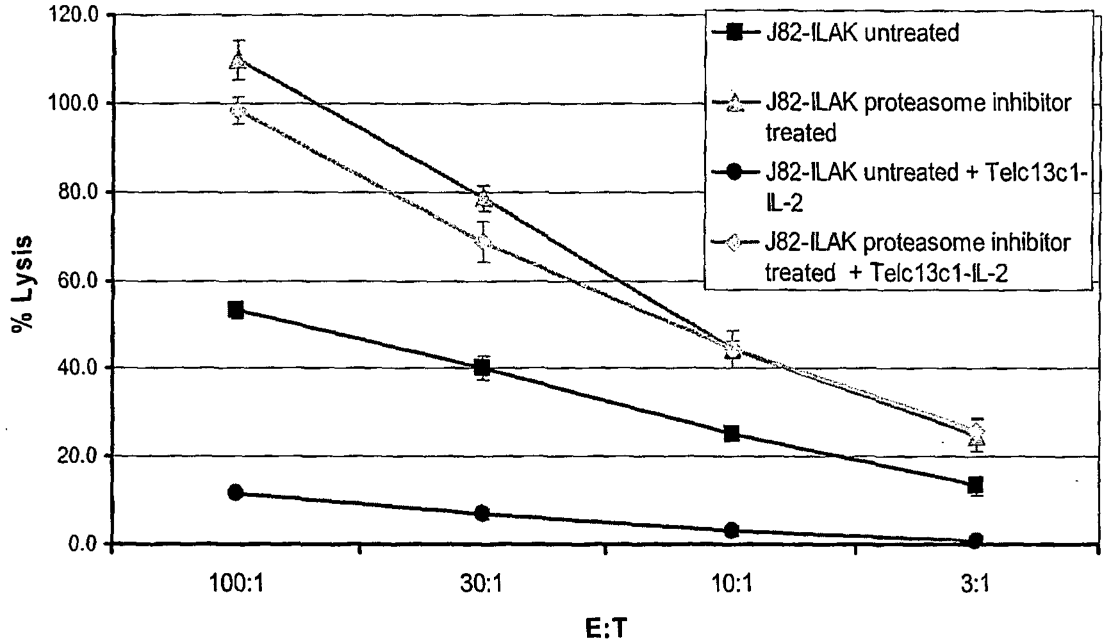
120.0
100.0
80.0
60.0
40.0
20.0
0.0
% Lysis
J82-ILAK untreated
J82-ILAK proteasome inhibitor treated
J82-ILAK untreated + Telc13c1-IL-2
J82-ILAK proteasome inhibitor treated + Telc13c1-IL-2
100:1
30:1
10:1
3:1
E:T

# Figure 19

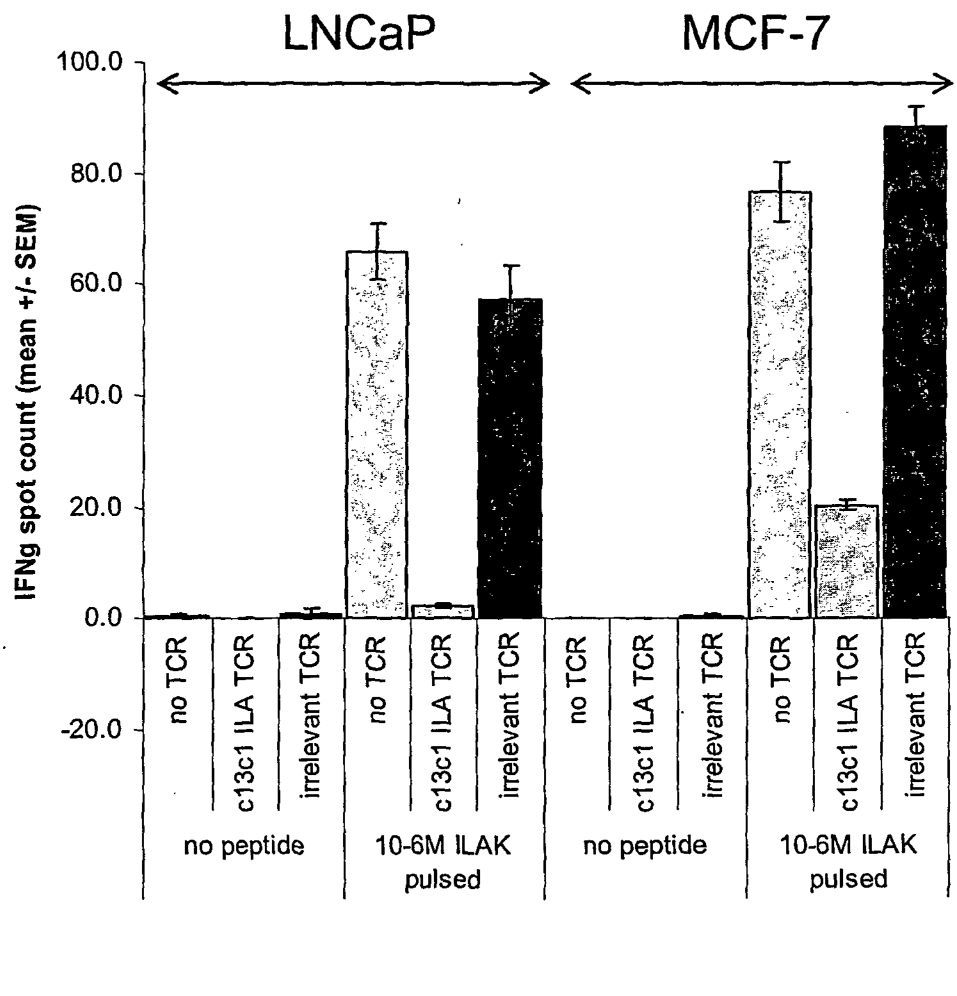
LNCaP
MCF-7
IFNg spot count (mean +/- SEM)
100.0
80.0
60.0
40.0
20.0
0.0
-20.0
no TCR
c13c1 ILA TCR
irrelevant TCR
no TCR
c13c1 ILA TCR
irrelevant TCR
no TCR
c13c1 ILA TCR
irrelevant TCR
no TCR
c13c1 ILA TCR
irrelevant TCR
no peptide
10-6M ILAK pulsed
no peptide
10-6M ILAK pulsed

## Figure 20a

M N A G V T Q T P K F Q V L K T G Q S M T L Q C A Q D M N
H E Y M S W Y R Q D P G M G L R L I H Y S V G A G I T D Q
G E V P N G Y N V S R S T T E D F P L R L L S A A P S Q T
S V Y F C G I Q P Y Q G T E A F F G Q G T R L T V V E D L
K N V F P P E V A V F E P S E A E I S H T Q K A T L V C L
A T G F Y P D H V E L S W W V N G K E V H S G V C T D P Q
P L K E Q P A L N D S R Y A L S S R L R V S A T F W Q D P
R N H F R C Q V Q F Y G L S E N D E W T Q D R A K P V T Q
I V S A E A W G R A D
(SEQ ID NO: 73)

## Figure 20b

M N A G V T Q T P K F Q V L K T G Q S M T L Q C A Q D M N
H E Y M S W Y R Q D P G M G L R L I H Y S I C P S C T D Q
G E V P N G Y N V S R S T T E D F P L R L L S A A P S Q T
S V Y F C G V Q P Y Q G T E A F F G Q G T R L T V V E D L
K N V F P P E V A V F E P S E A E I S H T Q K A T L V C L
A T G F Y P D H V E L S W W V N G K E V H S G V C T D P Q
P L K E Q P A L N D S R Y A L S S R L R V S A T F W Q D P
R N H F R C Q V Q F Y G L S E N D E W T Q D R A K P V T Q
I V S A E A W G R A D
(SEQ ID NO: 74)

## Figure 20c

M N A G V T Q T P K F Q V L K T G Q S M T L Q C A Q D M N
H E Y M S W Y R Q D P G M G L R L I H Y S R W V G D T D Q
G E V P N G Y N V S R S T T E D F P L R L L S A A P S Q T
S V Y F C G V Q P Y Q G T E A F F G Q G T R L T V V E D L
K N V F P P E V A V F E P S E A E I S H T Q K A T L V C L
A T G F Y P D H V E L S W W V N G K E V H S G V C T D P Q
P L K E Q P A L N D S R Y A L S S R L R V S A T F W Q D P
R N H F R C Q V Q F Y G L S E N D E W T Q D R A K P V T Q
I V S A E A W G R A D
(SEQ ID NO: 75)

## Figure 21a

M N A G V T Q T P K F Q V L K T G Q S M T L Q C A Q D M N
H E Y M S W Y R Q D P G M G L R L I H Y S V G A G I T D Q
G E V P N G Y N V S R S T T E D F P L R L L S A A P S Q T
S V Y F C G I Q P Y Q G T E A F F G Q G T R L T V V E D L
N K V F P P E V A V F E P S E A E I S H T Q K A T L V C L
A T G F F P D H V E L S W W V N G K E V H S G V C T D P Q
P L K E Q P A L N D S R Y A L S S R L R V S A T F W Q D P
R N H F R C Q V Q F Y G L S E N D E W T Q D R A K P V T Q
I V S A E A W G R A D
(SEQ ID NO: 76)

## Figure 21b

M N A G V T Q T P K F Q V L K T G Q S M T L Q C A Q D M N
H E Y M S W Y R Q D P G M G L R L I H Y S I C P S C T D Q
G E V P N G Y N V S R S T T E D F P L R L L S A A P S Q T
S V Y F C G V Q P Y Q G T E A F F G Q G T R L T V V E D L
N K V F P P E V A V F E P S E A E I S H T Q K A T L V C L
A T G F F P D H V E L S W W V N G K E V H S G V C T D P Q
P L K E Q P A L N D S R Y A L S S R L R V S A T F W Q D P
R N H F R C Q V Q F Y G L S E N D E W T Q D R A K P V T Q
I V S A E A W G R A D
(SEQ ID NO: 77)

## Figure 21c

M N A G V T Q T P K F Q V L K T G Q S M T L Q C A Q D M N
H E Y M S W Y R Q D P G M G L R L I H Y S R W V G D T D Q
G E V P N G Y N V S R S T T E D F P L R L L S A A P S Q T
S V Y F C G V Q P Y Q G T E A F F G Q G T R L T V V E D L
N K V F P P E V A V F E P S E A E I S H T Q K A T L V C L
A T G F F P D H V E L S W W V N G K E V H S G V C T D P Q
P L K E Q P A L N D S R Y A L S S R L R V S A T F W Q D P
R N H F R C Q V Q F Y G L S E N D E W T Q D R A K P V T Q
I V S A E A W G R A D
(SEQ ID NO: 78)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03070752 A
- WO 9960119 A
- GB 0411772 A
- GB 0419644 A

### Non-patent literature cited in the description


- **MEYERSON et al.** *Cell,* 1997, vol. 90, 785-795
- **NAKAMURA et al.** *Science,* 1997, vol. 277, 955-9
- **HOLLER et al.** *PNAS USA,* 2000, vol. 97 (10), 5387-5392
- **SAMBROOK ; RUSSELL.** Molecular Cloning - A Laboratory Manual. CSHL Press, 2001
- **RASHTCHIAN.** *Curr Opin Biotechnol,* 1995, vol. 6 (1), 30-6
- **FACTSBOOK.** LeFranc and LeFranc. Academic Press, 2001
- **CASEY et al.** *Tumor Targetting,* 2000, vol. 4, 235-244
- **HARRIS.** Polyethylene Glycol Chemistry - Biotechnical and Biomedical Applications. Plenum, 1992
- **HARRIS ; ZALIPSKY.** Chemistry and Biological Applications of Polyethylene Glycol ACS Books. 1997
- **WILLUDA et al.** *J. Biol. Chem.,* 2001, vol. 276 (17), 14385-14392
- **PAN et al.** *Biotechniques,* 2000, vol. 29 (6), 1234-8
- **O'CALLAGHAN et al.** *Anal. Biochem.,* 1999, vol. 266, 9-15
- **PRICE ; DWEK.** Principles and Problems in Physical Chemistry for Biochemists. Clarendon Press, 1979
- **RIMOLDI et al.** *J. Immunol.,* 2000, vol. 165, 7253-7261

SEQUENCE LISTING

<110> Avidex Ltd
Li, Yi
Jakobsen, Bent K

<120> High Affinity Telomerase TCRs

<130> 55/MG

<150> GB 0411772.7
<151> 2004-05-26

<150> GB 0419644.0
<151> 2004-09-03

<160> 78

<170> PatentIn version 3.1

<210> 1
<211> 111
<212> PRT
<213> Homo sapiens

<400> 1

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60
```



```
Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Ser Gly Thr
                85                  90                  95

Tyr Lys Tyr Ile Phe Gly Thr Gly Thr Arg Leu Lys Val Leu Ala
            100                 105                 110
```

<210> 2
<211> 111
<212> PRT
<213> Homo sapiens

<400> 2

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Val Gly Ala Gly Ile Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Ala Ser Ser Tyr Gln
                85                  90                  95

Gly Thr Glu Ala Phe Phe Gly Gln Gly Thr Arg Leu Thr Val Val
            100                 105                 110

<210> 3
<211> 618
<212> DNA
<213> Artificial Sequence

<220>
<223> DNA encoding soluble fragment of ILA TCR alpha chain

<400> 3

ggaatacaag tggagcagag tcctccagac ctgattctcc aggagggagc caattccacg 60

ctgcggtgca atttttctga ctctgtgaac aatttgcagt ggtttcatca aaacccttgg 120
ggacagctca tcaacctgtt ttacattccc tcagggacaa aacagaatgg aagattaagc 180
gccacgactg tcgctacgga acgctacagc ttattgtaca tttcctcttc ccagaccaca 240
gactcaggcg tttatttctg tgctgtggac tctgctacct caggaaccta caaatacatc 300
tttggaacag gcaccaggct gaaggtttta gcaaatatcc agaaccctga ccctgccgtg 360
taccagctga gagactctaa atccagtgac aagtctgtct gcctattcac cgattttgat 420
tctcaaacaa atgtgtcaca aagtaaggat tctgatgtgt atatcacaga caaaactgtg 480
ctagacatga ggtctatgga cttcaagagc aacagtgctg tggcctggag caacaaatct 540
gactttgcat gtgcaaacgc cttcaacaac agcattattc cagaagacac cttcttcccc 600
agcccagaaa gttcctaa 618

<210> 4
<211> 726
<212> DNA
<213> Artificial Sequence

<220>
<223> DNA encoding soluble form of ILA TCR beta chain

<400> 4

```
aacgctggtg tcactcagac cccaaaattc caggtcctga agacaggaca gagcatgaca     60
ctgcagtgtg cccaggatat gaaccatgaa tacatgtcct ggtatcgaca agacccaggc    120
atggggctga ggctgattca ttactcagtt ggtgctggta tcactgacca aggagaagtc    180
cccaatggct acaatgtctc cagatcaacc acagaggatt tcccgctcag gctgctgtcg    240
gctgctccct cccagacatc tgtgtacttc tgtgccagca gttaccaagg cactgaagct    300
ttctttggac aaggcaccag actcacagtt gtagaggacc tgaaaaacgt gttcccaccc    360
gaggtcgctg tgtttgagcc atcagaagca gagatctccc acacccaaaa ggccacactg    420
gtgtgcctgg ccacaggctt ctaccccgac cacgtggagc tgagctggtg ggtgaatggg    480
aaggaggtgc acagtggggt cagcacagac ccgcagcccc tcaaggagca gcccgccctc    540
aatgactcca gatacgctct gagcagccgc ctgagggtct cggccacctt ctggcaggac    600
ccccgcaacc acttccgctg tcaagtccag ttctacgggc tctcggagaa tgacgagtgg    660
acccaggata gggccaaacc cgtcacccag atcgtcagcg ccgaggcctg gggtagagca    720
gactaa                                                               726
```

<210> 5
<211> 205
<212> PRT
<213> Artificial Sequence

<220>
<223> Soluble fragment of ILA TCR alpha chain

<400> 5

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Ser Gly Thr
                85                  90                  95

Tyr Lys Tyr Ile Phe Gly Thr Gly Thr Arg Leu Lys Val Leu Ala Asn
            100                 105                 110

Ile Gln Asn Pro Asp Pro Ala Val Tyr Gln Leu Arg Asp Ser Lys Ser
        115                 120                 125

Ser Asp Lys Ser Val Cys Leu Phe Thr Asp Phe Asp Ser Gln Thr Asn
    130                 135                 140

Val Ser Gln Ser Lys Asp Ser Asp Val Tyr Ile Thr Asp Lys Thr Val
145                 150                 155                 160

Leu Asp Met Arg Ser Met Asp Phe Lys Ser Asn Ser Ala Val Ala Trp
                165                 170                 175

Ser Asn Lys Ser Asp Phe Ala Cys Ala Asn Ala Phe Asn Asn Ser Ile
            180                 185                 190

Ile Pro Glu Asp Thr Phe Phe Pro Ser Pro Glu Ser Ser
        195                 200                 205
```

<210> 6
<211> 241
<212> PRT
<213> Artificial Sequence

<220>
<223> Soluble fragment of ILA TCR beta chain

<400> 6

```
Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Val Gly Ala Gly Ile Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Ala Ser Ser Tyr Gln
                85                  90                  95

Gly Thr Glu Ala Phe Phe Gly Gln Gly Thr Arg Leu Thr Val Val Glu
            100                 105                 110

Asp Leu Lys Asn Val Phe Pro Pro Glu Val Ala Val Phe Glu Pro Ser
        115                 120                 125

Glu Ala Glu Ile Ser His Thr Gln Lys Ala Thr Leu Val Cys Leu Ala
    130                 135                 140

Thr Gly Phe Tyr Pro Asp His Val Glu Leu Ser Trp Trp Val Asn Gly
145                 150                 155                 160

Lys Glu Val His Ser Gly Val Ser Thr Asp Pro Gln Pro Leu Lys Glu
                165                 170                 175

Gln Pro Ala Leu Asn Asp Ser Arg Tyr Ala Leu Ser Ser Arg Leu Arg
            180                 185                 190

Val Ser Ala Thr Phe Trp Gln Asp Pro Arg Asn His Phe Arg Cys Gln
        195                 200                 205

Val Gln Phe Tyr Gly Leu Ser Glu Asn Asp Glu Trp Thr Gln Asp Arg
    210                 215                 220

Ala Lys Pro Val Thr Gln Ile Val Ser Ala Glu Ala Trp Gly Arg Ala
225                 230                 235                 240

Asp
```

<210> 7

<211> 618
<212> DNA
<213> Artificial Sequence

<220>
<223> DNA encoding soluble fragment of ILA TCR alpha chain with introdu ced cysteine

<400> 7

```
ggaatacaag tggagcagag tcctccagac ctgattctcc aggagggagc caattccacg    60
ctgcggtgca atttttctga ctctgtgaac aatttgcagt ggtttcatca aaacccttgg   120
ggacagctca tcaacctgtt ttacattccc tcagggacaa aacagaatgg aagattaagc   180
gccacgactg tcgctacgga acgctacagc ttattgtaca tttcctcttc ccagaccaca   240
gactcaggcg tttatttctg tgctgtggac tctgctacct caggaaccta caaatacatc   300
tttggaacag gcaccaggct gaaggtttta gcaaatatcc agaaccctga ccctgccgtg   360
taccagctga gagactctaa atccagtgac aagtctgtct gcctattcac cgattttgat   420
tctcaaacaa atgtgtcaca aagtaaggat tctgatgtgt atatcacaga caaatgtgtg   480
ctagacatga ggtctatgga cttcaagagc aacagtgctg tggcctggag caacaaatct   540
gactttgcat gtgcaaacgc cttcaacaac agcattattc cagaagacac cttcttcccc   600
agcccagaaa gttcctaa                                                 618
```

<210> 8
<211> 726
<212> DNA
<213> Artificial Sequence

<220>
<223> DNA encoding soluble fragment of ILA TCR beta chain with introduc ed cysteine

<400> 8

```
aacgctggtg tcactcagac cccaaaattc caggtcctga agacaggaca gagcatgaca    60
ctgcagtgtg cccaggatat gaaccatgaa tacatgtcct ggtatcgaca agacccaggc   120
atggggctga ggctgattca ttactcagtt ggtgctggta tcactgacca aggagaagtc   180
```

```
cccaatggct acaatgtctc cagatcaacc acagaggatt tcccgctcag gctgctgtcg     240
gctgctccct cccagacatc tgtgtacttc tgtgccagca gttaccaagg cactgaagct     300
ttctttggac aaggcaccag actcacagtt gtagaggacc tgaaaaacgt gttcccaccc     360
gaggtcgctg tgtttgagcc atcagaagca gagatctccc acacccaaaa ggccacactg     420
gtgtgcctgg ccacaggctt ctaccccgac cacgtggagc tgagctggtg ggtgaatggg     480
aaggaggtgc acagtggggt ctgcacagac ccgcagcccc tcaaggagca gcccgccctc     540
aatgactcca gatacgctct gagcagccgc ctgagggtct cggccacctt ctggcaggac     600
ccccgcaacc acttccgctg tcaagtccag ttctacgggc tctcggagaa tgacgagtgg     660
acccaggata gggccaaacc cgtcacccag atcgtcagcg ccgaggcctg gggtagagca     720
gactaa                                                                726
```

<210> 9
<211> 205
<212> PRT
<213> Artificial Sequence

<220>
<223> Soluble fragment of ILA TCR alpha chain with introduced cysteine

<400> 9

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Ser Gly Thr
                85                  90                  95

Tyr Lys Tyr Ile Phe Gly Thr Gly Thr Arg Leu Lys Val Leu Ala Asn
            100                 105                 110

Ile Gln Asn Pro Asp Pro Ala Val Tyr Gln Leu Arg Asp Ser Lys Ser
        115                 120                 125
```

Ser Asp Lys Ser Val Cys Leu Phe Thr Asp Phe Asp Ser Gln Thr Asn
130 135 140

Val Ser Gln Ser Lys Asp Ser Asp Val Tyr Ile Thr Asp Lys Cys Val
145 150 155 160

Leu Asp Met Arg Ser Met Asp Phe Lys Ser Asn Ser Ala Val Ala Trp
165 170 175

Ser Asn Lys Ser Asp Phe Ala Cys Ala Asn Ala Phe Asn Asn Ser Ile
180 185 190

Ile Pro Glu Asp Thr Phe Phe Pro Ser Pro Glu Ser Ser
195 200 205

<210> 10
<211> 241
<212> PRT
<213> Artificial Sequence

<220>
<223> Soluble fragment of ILA TCR with introduced cysteine

<400> 10

Asn Ala Gly Val Thr Gln Thr Pro Lys Phe Gln Val Leu Lys Thr Gly
1 5 10 15

Gln Ser Met Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Val Gly Ala Gly Ile Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Ala Ser Ser Tyr Gln
85 90 95

Gly Thr Glu Ala Phe Phe Gly Gln Gly Thr Arg Leu Thr Val Val Glu
100 105 110

Asp Leu Lys Asn Val Phe Pro Pro Glu Val Ala Val Phe Glu Pro Ser
115 120 125

Glu Ala Glu Ile Ser His Thr Gln Lys Ala Thr Leu Val Cys Leu Ala
130 135 140

Thr Gly Phe Tyr Pro Asp His Val Glu Leu Ser Trp Trp Val Asn Gly
145 150 155 160

Lys Glu Val His Ser Gly Val Cys Thr Asp Pro Gln Pro Leu Lys Glu
165 170 175

Gln Pro Ala Leu Asn Asp Ser Arg Tyr Ala Leu Ser Ser Arg Leu Arg
180 185 190

Val Ser Ala Thr Phe Trp Gln Asp Pro Arg Asn His Phe Arg Cys Gln
195 200 205

Val Gln Phe Tyr Gly Leu Ser Glu Asn Asp Glu Trp Thr Gln Asp Arg
210 215 220

Ala Lys Pro Val Thr Gln Ile Val Ser Ala Glu Ala Trp Gly Arg Ala
225 230 235 240

Asp

<210> 11
<211> 111
<212> PRT
<213> Artificial Sequence

<220>
<223> High affinity variant of ILA TCR alpha chain variable domain

<400> 11

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Ala Leu Pro
85 90 95

Tyr Gly Tyr Ile Phe Gly Thr Gly Thr Arg Leu Lys Val Leu Ala
100 105 110

<210> 12
<211> 111
<212> PRT
<213> Artificial Sequence

<220>
<223> High affinity variant of ILA TCR alpha chain variable domain

<400> 12

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Gln Leu Pro
85 90 95

Tyr Gly Tyr Ile Phe Gly Thr Gly Thr Arg Leu Lys Val Leu Ala
100 105 110

<210> 13
<211> 111
<212> PRT
<213> Artificial Sequence

<220>
<223> High affinity variant of ILA TCR alpha chain variable domain

<400> 13

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Thr Leu Pro
85 90 95

Tyr Gly Tyr Ile Phe Gly Thr Gly Thr Arg Leu Lys Val Leu Ala
100 105 110

<210> 14
<211> 111
<212> PRT
<213> Artificial Sequence

<220>
<223> High affinity variant of ILA TCR alpha chain variable domain

<400> 14

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Gly Leu Pro
85 90 95

Tyr Gly Tyr Ile Phe Gly Thr Gly Thr Arg Leu Lys Val Leu Ala
100 105 110

<210> 15
<211> 111
<212> PRT
<213> Artificial Sequence

<220>
<223> High affinity variant of ILA TCR alpha chain variable domain

<400> 15

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Thr Gly Met
85 90 95

Phe Gly Tyr Ile Phe Gly Thr Gly Thr Arg Leu Lys Val Leu Ala
100 105 110

<210> 16
<211> 111
<212> PRT
<213> Artificial Sequence

<220>
<223> High affinity variant of ILA TCR alpha chain variable domain

<400> 16

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Ser Leu Pro
85 90 95

Tyr Gly Tyr Ile Phe Gly Thr Gly Thr Arg Leu Lys Val Leu Ala
100 105 110

<210> 17
<211> 111
<212> PRT
<213> Artificial Sequence

<220>
<223> High affinity variant of ILA TCR alpha chain variable domain

<400> 17

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Ser Pro Pro
85 90 95

Tyr Gly Tyr Ile Phe Gly Thr Gly Thr Arg Leu Lys Val Leu Ala
100 105 110

<210> 18

<211> 111
<212> PRT
<213> Artificial Sequence

<220>
<223> High affinity variant of ILA TCR alpha chain variable domain

<400> 18

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Leu Pro Pro
                85                  90                  95

Tyr Gly Tyr Ile Phe Gly Thr Gly Thr Arg Leu Lys Val Leu Ala
            100                 105                 110
```

<210> 19
<211> 111
<212> PRT
<213> Artificial Sequence

<220>
<223> High affinity variant of ILA TCR alpha chain variable domain

<400> 19

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30
```

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Ser Ile Pro
85 90 95

Tyr Gly Tyr Ile Phe Gly Thr Gly Thr Arg Leu Lys Val Leu Ala
100 105 110

<210> 20
<211> 111
<212> PRT
<213> Artificial Sequence

<220>
<223> High affinity variant of ILA TCR alpha chain variable domain

<400> 20

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Ser Val Pro
85 90 95

Tyr Gly Tyr Ile Phe Gly Thr Gly Thr Arg Leu Lys Val Leu Ala
100 105 110

<210> 21
<211> 111
<212> PRT
<213> Artificial Sequence

<220>
<223> High affinity variant of ILA TCR alpha chain variable domain

<400> 21

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Ser Gly Met
                85                  90                  95

Phe Gly Tyr Ile Phe Gly Thr Gly Thr Arg Leu Lys Val Leu Ala
            100                 105                 110
```

<210> 22
<211> 111
<212> PRT
<213> Artificial Sequence

<220>
<223> High affinity variant of ILA TCR alpha chain variable domain

<400> 22

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45
```

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Thr Met Pro
85 90 95

Tyr Gly Tyr Ile Phe Gly Thr Gly Thr Arg Leu Lys Val Leu Ala
100 105 110

<210> 23
<211> 111
<212> PRT
<213> Artificial Sequence

<220>
<223> High affinity variant of ILA TCR beta chain variable domain

<400> 23

Asn Ala Gly Val Thr Gln Thr Pro Lys Phe Gln Val Leu Lys Thr Gly
1 5 10 15

Gln Ser Met Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Val Gly Ala Gly Ile Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Gly Ile Gln Pro Gln
85 90 95

Gly Thr Glu Ala Phe Phe Gly Gln Gly Thr Arg Leu Thr Val Val
100 105 110

<210> 24
<211> 111
<212> PRT
<213> Artificial Sequence

<220>
<223> High affinity variant of ILA TCR beta chain variable domain

<400> 24

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Val Gly Ala Gly Ile Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Gly Leu Gln Pro Gln
                85                  90                  95

Gly Thr Glu Ala Phe Phe Gly Gln Gly Thr Arg Leu Thr Val Val
            100                 105                 110

<210> 25
<211> 111
<212> PRT
<213> Artificial Sequence

<220>
<223> High affinity variant of ILA TCR beta chain variable domain

<400> 25

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Val Gly Ala Gly Ile Thr Asp Gln Gly Glu Val Pro Asn Gly Tyr
    50                  55                  60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Gly Val Gln Pro Gln
85 90 95

Gly Thr Glu Ala Phe Phe Gly Gln Gly Thr Arg Leu Thr Val Val
100 105 110

<210> 26
<211> 111
<212> PRT
<213> Artificial Sequence

<220>
<223> High affinity variant of ILA TCR beta chain variable domain

<400> 26

Asn Ala Gly Val Thr Gln Thr Pro Lys Phe Gln Val Leu Lys Thr Gly
1 5 10 15

Gln Ser Val Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Ile His Pro Glu Tyr Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Ala Ser Ser Tyr Gln
85 90 95

Gly Thr Glu Ala Phe Phe Gly Gln Gly Thr Arg Leu Thr Val Val
100 105 110

<210> 27
<211> 111
<212> PRT
<213> Artificial Sequence

<220>
<223> High affinity variant of ILA TCR beta chain variable domain

<400> 27

```
Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Leu His Pro Ser Val Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                      80

Ala Ala Pro Ser Gln Thr Ser Val Tyr Phe Cys Ala Ser Ser Tyr Gln
                85                  90                  95

Gly Thr Glu Ala Phe Phe Gly Gln Gly Thr Arg Leu Thr Val Val
            100                 105                 110
```

<210> 28
<211> 111
<212> PRT
<213> Artificial Sequence

<220>
<223> High affinity variant of ILA TCR beta chain variable domain

<400> 28

```
Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Ile Cys Pro Ser Cys Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                      80
```

Ala Ala Pro Ser Gln Thr Ser Val Tyr Phe Cys Ala Ser Ser Tyr Gln
85 90 95

Gly Thr Glu Ala Phe Phe Gly Gln Gly Thr Arg Leu Thr Val Val
100 105 110

<210> 29
<211> 111
<212> PRT
<213> Artificial Sequence

<220>
<223> High affinity variant of ILA TCR beta chain variable domain

<400> 29

Asn Ala Gly Val Thr Gln Thr Pro Lys Phe Gln Val Leu Lys Thr Gly
1 5 10 15

Gln Ser Met Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Ile Cys Trp Gly Cys Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Ala Ser Ser Tyr Gln
85 90 95

Gly Thr Glu Ala Phe Phe Gly Gln Gly Thr Arg Leu Thr Val Val
100 105 110

<210> 30
<211> 111
<212> PRT
<213> Artificial Sequence

<220>
<223> High affinity variant of ILA TCR beta chain variable domain

<400> 30

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Ile Trp Glu Phe Glu Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Ala Ser Ser Tyr Gln
                85                  90                  95

Gly Thr Glu Ala Phe Phe Gly Gln Gly Thr Arg Leu Thr Val Val
            100                 105                 110

<210> 31
<211> 111
<212> PRT
<213> Artificial Sequence

<220>
<223> High affinity variant of ILA TCR beta chain variable domain

<400> 31

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Arg Trp Val Gly Asp Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Ala Ser Ser Tyr Gln
                85                  90                  95

Gly Thr Glu Ala Phe Phe Gly Gln Gly Thr Arg Leu Thr Val Val
100 105 110

<210> 32
<211> 47
<212> PRT
<213> Homo sapiens

<400> 32

Asn Ile Gln Asn Pro Asp Pro Ala Val Tyr Gln Leu Arg Asp Ser Lys
1 5 10 15

Ser Ser Asp Lys Ser Val Cys Leu Phe Thr Asp Phe Asp Ser Gln Thr
20 25 30

Asn Val Ser Gln Ser Lys Asp Ser Asp Val Tyr Ile Thr Asp Lys
35 40 45

<210> 33
<211> 56
<212> PRT
<213> Homo sapiens

<400> 33

Glu Asp Leu Asn Lys Val Phe Pro Pro Glu Val Ala Val Phe Glu Pro
1 5 10 15

Ser Glu Ala Glu Ile Ser His Thr Gln Lys Ala Thr Leu Val Cys Leu
20 25 30

Ala Thr Gly Phe Phe Pro Asp His Val Glu Leu Ser Trp Trp Val Asn
35 40 45

Gly Lys Glu Val His Ser Gly Val
50 55

<210> 34
<211> 56
<212> PRT
<213> Homo sapiens

<400> 34

Glu Asp Leu Lys Asn Val Phe Pro Pro Glu Val Ala Val Phe Glu Pro
1               5                   10                  15

Ser Glu Ala Glu Ile Ser His Thr Gln Lys Ala Thr Leu Val Cys Leu
            20                  25                  30

Ala Thr Gly Phe Tyr Pro Asp His Val Glu Leu Ser Trp Trp Val Asn
        35                  40                  45

Gly Lys Glu Val His Ser Gly Val
    50                  55

<210> 35
<211> 30
<212> PRT
<213> Artificial Sequence

<220>
<223> Short scTCR interchain linker

<400> 35

Pro Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Pro
            20                  25                  30

<210> 36
<211> 35
<212> PRT
<213> Artificial Sequence

<220>
<223> Long TCR interchain linker

<400> 36

Pro Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Pro
        35

<210> 37
<211> 9
<212> PRT
<213> Homo sapiens

<400> 37

Ile Leu Ala Lys Phe Leu His Trp Leu
1 5

<210> 38
<211> 37
<212> DNA
<213> Artificial Sequence

<220>
<223> primer

<400> 38
tctctcatta atgggaatac aagtggagca gagtcct 37

<210> 39
<211> 20
<212> DNA
<213> Artificial Sequence

<220>
<223> Primer

<400> 39
cggcagggtc agggttctgg 20

<210> 40
<211> 20
<212> DNA
<213> artificial sequence

<220>
<223> Primer

<400> 40
ccagaaccct gaccctgccg 20

<210> 41
<211> 30
<212> DNA
<213> Artificial Sequence

<220>
<223> Primer

<400> 41
aagcttcccg ggggaacttt ctgggctggg 30

<210> 42
<211> 36
<212> DNA
<213> Artificial Sequence

<220>
<223> Primer

<400> 42
tctctcatta atgaatgctg gtgtcactca gacccc 36

<210> 43
<211> 22
<212> DNA
<213> Artificial Sequence

<220>
<223> Primer

<400> 43
cttctgatgg ctcaaacaca gc 22

<210> 44
<211> 22
<212> DNA
<213> Artificial Sequence

<220>
<223> Primer

<400> 44
gctgtgtttg agccatcaga ag 22

<210> 45
<211> 30
<212> DNA
<213> Artificial Sequence

<220>
<223> Primer

<400> 45
aagcttcccg gggtctgctc taccccaggc 30

<210> 46
<211> 3342
<212> DNA
<213> Artificial Sequence

<220>
<223> pEX954 plasmid

<400> 46

gatctcgatc ccgcgaaatt aatacgactc actataggga gaccacaacg gtttccctct 60
agaaataatt ttgtttaact ttaagaagga gatataatcg atgtctaact cgagtgacaa 120
gtctgtctgc ctattcaccg attttgattc tcaaacaaat gtgtcacaaa gtaaggattc 180
tgatgtgtat atcacagaca aatgtgtgct agacatgagg tctatggact tcaagagcaa 240
cagtgctgtg gcctggagca acaaatctga ctttgcatgt gcaaacgcct tcaacaacag 300
cattattcca gaagacacct tcttccccag cccagaaagt tcctaagctt gaattccgat 360
ccggctgcta acaaagcccg aaaggaagct gagttggctg ctgccaccgc tgagcaataa 420
ctagcataac cccttggggc ctctaaacgg gtcttgaggg gttttttgct gaaaggagga 480
actatatccg gataattctt gaagacgaaa gggcctcgtg atacgcctat ttttataggt 540
taatgtcatg ataataatgg tttcttagac gtgaggtggc acttttcggg gaaatgtgcg 600
cggaacccct atttgtttat ttttctaaat acattcaaat atgtatccgc tcatgagaca 660
ataaccctga taaatgcttc aataatattt tgttaaaatt cgcgttaaat ttttgttaaa 720
tcagctcatt ttttaaccaa taggccgaaa tcggcaaaat cccttataaa tcaaaagaat 780
agaccgagat agggttgagt gttgttccag tttggaacaa gagtccacta ttaaagaacg 840

```
tggactccaa cgtcaaaggg cgaaaaaccg tctatcaggg cgatggccca ctacgtgaac     900
catcacccta atcaagtttt ttggggtcga ggtgccgtaa agcactaaat cggaacccta     960
aagggagccc ccgatttaga gcttgacggg gaaagccggc gaacgtggcg agaaaggaag    1020
ggaagaaagc gaaaggagcg ggcgctaggg cgctggcaag tgtagcggtc acgctgcgcg    1080
taaccaccac acccgccgcg cttaatgcgc cgctacaggg cgcgtcaggt ggcacttttc    1140
ggggaaatgt gcgcggaacc cctatttgtt tatttttcta aatacattca aatatgtatc    1200
cgctcatgag acaataaccc tgataaatgc ttcaataata ttgaaaaagg aagagtatga    1260
gtattcaaca tttccgtgtc gcccttattc ccttttttgc ggcattttgc cttcctgttt    1320
ttgctcaccc agaaacgctg gtgaaagtaa aagatgctga agatcagttg ggtgcacgag    1380
tgggttacat cgaactggat ctcaacagcg gtaagatcct tgagagtttt cgccccgaag    1440
aacgttttcc aatgatgagc acttttaaag ttctgctatg tggcgcggta ttatcccgtg    1500
ttgacgccgg gcaagagcaa ctcggtcgcc gcatacacta ttctcagaat gacttggttg    1560
agtactcacc agtcacagaa aagcatctta cggatggcat gacagtaaga gaattatgca    1620
gtgctgccat aaccatgagt gataacactg cggccaactt acttctgaca acgatcggag    1680
gaccgaagga gctaaccgct tttttgcaca acatggggga tcatgtaact cgccttgatc    1740
gttgggaacc ggagctgaat gaagccatac caaacgacga gcgtgacacc acgatgcctg    1800
cagcaatggc aacaacgttg cgcaaactat taactggcga actacttact ctagcttccc    1860
ggcaacaatt aatagactgg atggaggcgg ataaagttgc aggaccactt ctgcgctcgg    1920
cccttccggc tggctggttt attgctgata aatctggagc cggtgagcgt gggtctcgcg    1980
gtatcattgc agcactgggg ccagatggta agccctcccg tatcgtagtt atctacacga    2040
cggggagtca ggcaactatg gatgaacgaa atagacagat cgctgagata ggtgcctcac    2100
tgattaagca ttggtaactg tcagaccaag tttactcata tatactttag attgatttaa    2160
aacttcattt ttaatttaaa aggatctagg tgaagatcct ttttgataat ctcatgacca    2220
aaatccctta acgtgagttt tcgttccact gagcgtcaga ccccgtagaa aagatcaaag    2280
gatcttcttg agatcctttt tttctgcgcg taatctgctg cttgcaaaca aaaaaaccac    2340
cgctaccagc ggtggtttgt ttgccggatc aagagctacc aactcttttt ccgaaggtaa    2400
ctggcttcag cagagcgcag ataccaaata ctgtccttct agtgtagccg tagttaggcc    2460
accacttcaa gaactctgta gcaccgccta catacctcgc tctgctaatc ctgttaccag    2520
tggctgctgc cagtggcgat aagtcgtgtc ttaccgggtt ggactcaaga cgatagttac    2580
cggataaggc gcagcggtcg ggctgaacgg ggggttcgtg cacacagccc agcttggagc    2640
gaacgaccta caccgaactg agatacctac agcgtgagct atgagaaagc gccacgcttc    2700
ccgaagggag aaaggcggac aggtatccgg taagcggcag ggtcggaaca ggagagcgca    2760
cgagggagct tccaggggga aacgcctggt atctttatag tcctgtcggg tttcgccacc    2820
tctgacttga gcgtcgattt ttgtgatgct cgtcaggggg gcggagccta tggaaaaacg    2880
```

ccagcaacgc ggccttttta cggttcctgg ccttttgctg gccttttgct cacatgttct 2940
ttcctgcgtt atcccctgat tctgtggata accgtattac cgcctttgag tgagctgata 3000
ccgctcgccg cagccgaacg accgagcgca gcgagtcagt gagcgaggaa gcggaagagc 3060
gcctgatgcg gtattttctc cttacgcatc tgtgcggtat ttcacaccgc aatggtgcac 3120
tctcagtaca atctgctctg atgccgcata gttaagccag tatacactcc gctatcgcta 3180
cgtgactggg tcatggctgc gccccgacac ccgccaacac ccgctgacgc gccctgacgg 3240
gcttgtctgc tcccggcatc cgcttacaga caagctgtga ccgtctccgg gagctgcatg 3300
tgtcagaggt tttcaccgtc atcaccgaaa cgcgcgaggc ag 3342

<210> 47
<211> 3836
<212> DNA
<213> Artificial Sequence

<220>
<223> pEX821 plasmid

<400> 47

gatctcgatc ccgcgaaatt aatacgactc actataggga gaccacaacg gtttccctct 60
agaaataatt ttgtttaact ttaagaagga gatatacata tgaacgctgg tgtcactcag 120
accccaaaat tccaggtcct gaagacagga cagagcatga cactgcagtg tgcccaggat 180
atgaaccatg aatacatgtc ctggtatcga caagacccag gcatggggct gaggctgatt 240
cattactcag ttggtgctgg tatcactgac caaggagaag tccccaatgg ctacaatgtc 300
tccagatcaa ccacagagga tttcccgctc aggctgctgt cggctgctcc ctcccagaca 360
tctgtgtact tctgtgccag caggccggga ctagcgggag ggcgaccaga gcagtacttc 420
gggccgggca ccaggctcac ggtcacagag gacctgaaaa acgtgttccc acccgaggtc 480
gctgtgtttg agccatcaga agcagagatc tcccacaccc aaaaggccac actggtgtgc 540
ctggccaccg gtttctaccc cgaccacgtg gagctgagct ggtgggtgaa tgggaaggag 600
gtgcacagtg ggtctgcac agacccgcag cccctcaagg agcagcccgc cctcaatgac 660
tccagatacg ctctgagcag ccgcctgagg gtctcggcca ccttctggca ggacccccgc 720
aaccacttcc gctgtcaagt ccagttctac gggctctcgg agaatgacga gtggacccag 780
gatagggcca aacccgtcac ccagatcgtc agcgccgagg cctggggtag agcagactaa 840
gcttgaattc cgatccggct gctaacaaag cccgaaagga agctgagttg gctgctgcca 900
ccgctgagca ataactagca taaccccttg gggcctctaa acgggtcttg aggggttttt 960
tgctgaaagg aggaactata tccggataat tcttgaagac gaaagggcct cgtgatacgc 1020
ctatttttat aggttaatgt catgataata atggtttctt agacgtcagg tggcactttt 1080

cggggaaatg tgcgcggaac ccctatttgt ttatttttct aaatacattc aaatatgtat 1140
ccgctcatga gacaataacc ctgataaatg cttcaataat attttgttaa aattcgcgtt 1200
aaatttttgt taaatcagct cattttttaa ccaataggcc gaaatcggca aaatccctta 1260
taaatcaaaa gaatagaccg agatagggtt gagtgttgtt ccagtttgga acaagagtcc 1320
actattaaag aacgtggact ccaacgtcaa agggcgaaaa accgtctatc agggcgatgg 1380
cccactacgt gaaccatcac cctaatcaag ttttttgggg tcgaggtgcc gtaaagcact 1440
aaatcggaac cctaaaggga gcccccgatt tagagcttga cggggaaagc cggcgaacgt 1500
ggcgagaaag gaagggaaga aagcgaaagg agcgggcgct agggcgctgg caagtgtagc 1560
ggtcacgctg cgcgtaacca ccacacccgc cgcgcttaat gcgccgctac agggcgcgtc 1620
aggtggcact tttcggggaa atgtgcgcgg aacccctatt tgtttatttt tctaaataca 1680
ttcaaatatg tatccgctca tgagacaata accctgataa atgcttcaat aatattgaaa 1740
aaggaagagt atgagtattc aacatttccg tgtcgccctt attccctttt ttgcggcatt 1800
ttgccttcct gtttttgctc acccagaaac gctggtgaaa gtaaaagatg ctgaagatca 1860
gttgggtgca cgagtgggtt acatcgaact ggatctcaac agcggtaaga tccttgagag 1920
ttttcgcccc gaagaacgtt ttccaatgat gagcactttt aaagttctgc tatgtggcgc 1980
ggtattatcc cgtgttgacg ccgggcaaga gcaactcggt cgccgcatac actattctca 2040
gaatgacttg gttgagtact caccagtcac agaaaagcat cttacggatg gcatgacagt 2100
aagagaatta tgcagtgctg ccataaccat gagtgataac actgcggcca acttacttct 2160
gacaacgatc ggaggaccga aggagctaac cgcttttttg cacaacatgg gggatcatgt 2220
aactcgcctt gatcgttggg aaccggagct gaatgaagcc ataccaaacg acgagcgtga 2280
caccacgatg cctgcagcaa tggcaacaac gttgcgcaaa ctattaactg gcgaactact 2340
tactctagct tcccggcaac aattaataga ctggatggag gcggataaag ttgcaggacc 2400
acttctgcgc tcggcccttc cggctggctg gtttattgct gataaatctg gagccggtga 2460
gcgtgggtct cgcggtatca ttgcagcact ggggccagat ggtaagccct cccgtatcgt 2520
agttatctac acgacgggga gtcaggcaac tatggatgaa cgaaatagac agatcgctga 2580
gataggtgcc tcactgatta agcattggta actgtcagac caagtttact catatatact 2640
ttagattgat ttaaaacttc atttttaatt taaaaggatc taggtgaaga tcctttttga 2700
taatctcatg accaaaatcc cttaacgtga gttttcgttc cactgagcgt cagaccccgt 2760
agaaaagatc aaaggatctt cttgagatcc tttttttctg cgcgtaatct gctgcttgca 2820
aacaaaaaaa ccaccgctac cagcggtggt ttgtttgccg gatcaagagc taccaactct 2880
ttttccgaag gtaactggct tcagcagagc gcagatacca aatactgtcc ttctagtgta 2940
gccgtagtta ggccaccact tcaagaactc tgtagcaccg cctacatacc tcgctctgct 3000
aatcctgtta ccagtggctg ctgccagtgg cgataagtcg tgtcttaccg ggttggactc 3060
aagacgatag ttaccggata aggcgcagcg gtcgggctga acggggggtt cgtgcacaca 3120

gcccagcttg gagcgaacga cctacaccga actgagatac ctacagcgtg agctatgaga 3180
aagcgccacg cttcccgaag ggagaaaggc ggacaggtat ccggtaagcg gcagggtcgg 3240
aacaggagag cgcacgaggg agcttccagg gggaaacgcc tggtatcttt atagtcctgt 3300
cgggtttcgc cacctctgac ttgagcgtcg atttttgtga tgctcgtcag gggggcggag 3360
cctatggaaa aacgccagca acgcggcctt tttacggttc ctggcctttt gctggccttt 3420
tgctcacatg ttctttcctg cgttatcccc tgattctgtg gataaccgta ttaccgcctt 3480
tgagtgagct gataccgctc gccgcagccg aacgaccgag cgcagcgagt cagtgagcga 3540
ggaagcggaa gagcgcctga tgcggtattt tctccttacg catctgtgcg gtatttcaca 3600
ccgcaatggt gcactctcag tacaatctgc tctgatgccg catagttaag ccagtataca 3660
ctccgctatc gctacgtgac tgggtcatgg ctgcgccccg acacccgcca acacccgctg 3720
acgcgccctg acgggcttgt ctgctcccgg catccgctta cagacaagct gtgaccgtct 3780
ccgggagctg catgtgtcag aggttttcac cgtcatcacc gaaacgcgcg aggcag 3836

<210> 48
<211> 3857
<212> DNA
<213> Artificial Sequence

<220>
<223> pEX202 plasmid

<400> 48

gatctcgatc ccgcgaaatt aatacgactc actataggga gaccacaacg gtttccctct 60
agaaataatt ttgtttaact ttaagaagga gatatacata tgcagaagga agtggagcag 120
aactctggac ccctcagtgt tccagaggga gccattgcct ctctcaactg cacttacagt 180
gaccgaggtt cccagtcctt cttctggtac agacaatatt ctgggaaaag ccctgagttg 240
ataatgtcca tatactccaa tggtgacaaa gaagatggaa ggtttacagc acagctcaat 300
aaagccagcc agtatgtttc tctgctcatc agagactccc agcccagtga ttcagccacc 360
tacctctgtg ccgttacaac tgacagctgg gggaaattgc agtttggagc agggacccag 420
gttgtggtca ccccagatat ccagaaccct gaccctgccg tgtaccagct gagagactct 480
aaatccagtg acaagtctgt ctgcctattc accgattttg attctcaaac aaatgtgtca 540
caaagtaagg attctgatgt gtatatcaca gacaaaactg tgctagacat gaggtctatg 600
gacttcaaga gcaacagtgc tgtggcctgg agcaacaaat ctgactttgc atgtgcaaac 660
gccttcaaca acagcattat tccagaagac accttcttcc ccagcccaga aagttccccc 720
gggggtagaa tcgcccggct ggaggaaaaa gtgaaaacct tgaaagctca gaactcggag 780
ctggcgtcca cggccaacat gctcagggaa caggtggcac agcttaaaca gaaagtcatg 840

aactactagg atccatggta agcttgaatt ccgatccggc tgctaacaaa gcccgaaagg 900
aagctgagtt ggctgctgcc accgctgagc aataactagc ataacccctt ggggcctcta 960
aacgggtctt gaggggtttt ttgctgaaag gaggaactat atccggataa ttcttgaaga 1020
cgaaagggcc tcgtgatacg cctattttta taggttaatg tcatgataat aatggtttct 1080
tagacgtcag gtggcacttt tcggggaaat gtgcgcggaa cccctatttg tttatttttc 1140
taaatacatt caaatatgta tccgctcatg agacaataac cctgataaat gcttcaataa 1200
tattttgtta aaattcgcgt taaatttttg ttaaatcagc tcatttttta accaataggc 1260
cgaaatcggc aaaatccctt ataaatcaaa agaatagacc gagatagggt tgagtgttgt 1320
tccagtttgg aacaagagtc cactattaaa gaacgtggac tccaacgtca aagggcgaaa 1380
aaccgtctat cagggcgatg gcccactacg tgaaccatca ccctaatcaa gttttttggg 1440
gtcgaggtgc cgtaaagcac taaatcggaa ccctaaaggg agcccccgat ttagagcttg 1500
acggggaaag ccggcgaacg tggcgagaaa ggaagggaag aaagcgaaag gagcgggcgc 1560
tagggcgctg gcaagtgtag cggtcacgct gcgcgtaacc accacacccg ccgcgcttaa 1620
tgcgccgcta cagggcgcgt caggtggcac ttttcgggga aatgtgcgcg gaacccctat 1680
ttgtttattt ttctaaatac attcaaatat gtatccgctc atgagacaat aaccctgata 1740
aatgcttcaa taatattgaa aaaggaagag tatgagtatt caacatttcc gtgtcgccct 1800
tattcccttt tttgcggcat tttgccttcc tgtttttgct cacccagaaa cgctggtgaa 1860
agtaaaagat gctgaagatc agttgggtgc acgagtgggt tacatcgaac tggatctcaa 1920
cagcggtaag atccttgaga gttttcgccc cgaagaacgt tttccaatga tgagcacttt 1980
taaagttctg ctatgtggcg cggtattatc ccgtgttgac gccgggcaag agcaactcgg 2040
tcgccgcata cactattctc agaatgactt ggttgagtac tcaccagtca cagaaaagca 2100
tcttacggat ggcatgacag taagagaatt atgcagtgct gccataacca tgagtgataa 2160
cactgcggcc aacttacttc tgacaacgat cggaggaccg aaggagctaa ccgctttttt 2220
gcacaacatg ggggatcatg taactcgcct tgatcgttgg gaaccggagc tgaatgaagc 2280
cataccaaac gacgagcgtg acaccacgat gcctgcagca atggcaacaa cgttgcgcaa 2340
actattaact ggcgaactac ttactctagc ttcccggcaa caattaatag actggatgga 2400
ggcggataaa gttgcaggac cacttctgcg ctcggccctt ccggctggct ggtttattgc 2460
tgataaatct ggagccggtg agcgtgggtc tcgcggtatc attgcagcac tggggccaga 2520
tggtaagccc tcccgtatcg tagttatcta cacgacgggg agtcaggcaa ctatggatga 2580
acgaaataga cagatcgctg agataggtgc ctcactgatt aagcattggt aactgtcaga 2640
ccaagtttac tcatatatac tttagattga tttaaaactt catttttaat ttaaaaggat 2700
ctaggtgaag atcctttttg ataatctcat gaccaaaatc ccttaacgtg agttttcgtt 2760
ccactgagcg tcagaccccg tagaaaagat caaaggatct tcttgagatc ctttttttct 2820
gcgcgtaatc tgctgcttgc aaacaaaaaa accaccgcta ccagcggtgg tttgtttgcc 2880

```
ggatcaagag ctaccaactc tttttccgaa ggtaactggc ttcagcagag cgcagatacc   2940
aaatactgtc cttctagtgt agccgtagtt aggccaccac ttcaagaact ctgtagcacc   3000
gcctacatac ctcgctctgc taatcctgtt accagtggct gctgccagtg gcgataagtc   3060
gtgtcttacc gggttggact caagacgata gttaccggat aaggcgcagc ggtcgggctg   3120
aacggggggt tcgtgcacac agcccagctt ggagcgaacg acctacaccg aactgagata   3180
cctacagcgt gagctatgag aaagcgccac gcttcccgaa gggagaaagg cggacaggta   3240
tccggtaagc ggcagggtcg gaacaggaga gcgcacgagg gagcttccag ggggaaacgc   3300
ctggtatctt tatagtcctg tcgggtttcg ccacctctga cttgagcgtc gatttttgtg   3360
atgctcgtca ggggggcgga gcctatggaa aaacgccagc aacgcggcct ttttacggtt   3420
cctggccttt tgctggcctt ttgctcacat gttctttcct gcgttatccc ctgattctgt   3480
ggataaccgt attaccgcct ttgagtgagc tgataccgct cgccgcagcc gaacgaccga   3540
gcgcagcgag tcagtgagcg aggaagcgga agagcgcctg atgcggtatt ttctccttac   3600
gcatctgtgc ggtatttcac accgcaatgg tgcactctca gtacaatctg ctctgatgcc   3660
gcatagttaa gccagtatac actccgctat cgctacgtga ctgggtcatg gctgcgcccc   3720
gacacccgcc aacacccgct gacgcgccct gacgggcttg tctgctcccg gcatccgctt   3780
acagacaagc tgtgaccgtc tccgggagct gcatgtgtca gaggttttca ccgtcatcac   3840
cgaaacgcgc gaggcag                                                  3857
```

<210> 49
<211> 4019
<212> DNA
<213> Artificial Sequence

<220>
<223> pEX205 plasmid

<400> 49

```
gatctcgatc ccgcgaaatt aatacgactc actataggga gaccacaacg gtttccctct     60
agaaataatt ttgtttaact ttaagaagga gatatacata tgaacgctgg tgtcactcag    120
accccaaaat tccaggtcct gaagacagga cagagcatga cactgcagtg tgcccaggat    180
atgaaccatg aatacatgtc ctggtatcga caagacccag gcatggggct gaggctgatt    240
cattactcag ttggtgctgg tatcactgac caaggagaag tccccaatgg ctacaatgtc    300
tccagatcaa ccacagagga tttcccgctc aggctgctgt cggctgctcc ctcccagaca    360
tctgtgtact tctgtgccag caggccggga ctagcgggag ggcgaccaga gcagtacttc    420
gggccgggca ccaggctcac ggtcacagag gacctgaaaa acgtgttccc acccgaggtc    480
gctgtgtttg agccatcaga agcagagatc tcccacaccc aaaaggccac actggtgtgc    540
```

ctggccacag gcttctaccc cgaccacgtg gagctgagct ggtgggtgaa tgggaaggag 600
gtgcacagtg ggtcagcac agacccgcag cccctcaagg agcagcccgc cctcaatgac 660
tccagatacg ctctgagcag ccgcctgagg gtctcggcca ccttctggca ggacccccgc 720
aaccacttcc gctgtcaagt ccagttctac gggctctcgg agaatgacga gtggacccag 780
gatagggcca aacccgtcac ccagatcgtc agcgccgagg cctggggtag agcagacccc 840
gggggtctga ctgatacact ccaagcggag acagatcaac ttgaagacaa gaagtctgcg 900
ttgcagaccg agattgccaa tctactgaaa gagaaggaaa aactagagtt catcctggca 960
gcttacggat ccggtggtgg tctgaacgat atttttgaag ctcagaaaat cgaatggcat 1020
taagcttgaa ttccgatccg gctgctaaca aagcccgaaa ggaagctgag ttggctgctg 1080
ccaccgctga gcaataacta gcataacccc ttggggcctc taaacgggtc ttgaggggtt 1140
ttttgctgaa aggaggaact atatccggat aattcttgaa gacgaaaggg cctcgtgata 1200
cgcctatttt tataggttaa tgtcatgata ataatggttt cttagacgtc aggtggcact 1260
tttcggggaa atgtgcgcgg aacccctatt tgtttatttt tctaaataca ttcaaatatg 1320
tatccgctca tgagacaata accctgataa atgcttcaat aatattttgt taaaattcgc 1380
gttaaatttt tgttaaatca gctcattttt taaccaatag gccgaaatcg gcaaaatccc 1440
ttataaatca aaagaataga ccgagatagg gttgagtgtt gttccagttt ggaacaagag 1500
tccactatta aagaacgtgg actccaacgt caaagggcga aaaaccgtct atcagggcga 1560
tggcccacta cgtgaaccat caccctaatc aagttttttg gggtcgaggt gccgtaaagc 1620
actaaatcgg aaccctaaag ggagcccccg atttagagct tgacggggaa agccggcgaa 1680
cgtggcgaga aaggaaggga agaaagcgaa aggagcgggc gctagggcgc tggcaagtgt 1740
agcggtcacg ctgcgcgtaa ccaccacacc cgccgcgctt aatgcgccgc tacagggcgc 1800
gtcaggtggc acttttcggg gaaatgtgcg cggaacccct atttgtttat ttttctaaat 1860
acattcaaat atgtatccgc tcatgagaca ataaccctga taaatgcttc aataatattg 1920
aaaaaggaag agtatgagta ttcaacattt ccgtgtcgcc cttattccct tttttgcggc 1980
attttgcctt cctgtttttg ctcacccaga aacgctggtg aaagtaaaag atgctgaaga 2040
tcagttgggt gcacgagtgg gttacatcga actggatctc aacagcggta agatccttga 2100
gagttttcgc cccgaagaac gttttccaat gatgagcact tttaaagttc tgctatgtgg 2160
cgcggtatta tcccgtgttg acgccgggca agagcaactc ggtcgccgca tacactattc 2220
tcagaatgac ttggttgagt actcaccagt cacagaaaag catcttacgg atggcatgac 2280
agtaagagaa ttatgcagtg ctgccataac catgagtgat aacactgcgg ccaacttact 2340
tctgacaacg atcggaggac cgaaggagct aaccgctttt ttgcacaaca tgggggatca 2400
tgtaactcgc cttgatcgtt gggaaccgga gctgaatgaa gccataccaa acgacgagcg 2460
tgacaccacg atgcctgcag caatggcaac aacgttgcgc aaactattaa ctggcgaact 2520
acttactcta gcttcccggc aacaattaat agactggatg gaggcggata aagttgcagg 2580

accacttctg cgctcggccc ttccggctgg ctggtttatt gctgataaat ctggagccgg 2640
tgagcgtggg tctcgcggta tcattgcagc actggggcca gatggtaagc cctcccgtat 2700
cgtagttatc tacacgacgg ggagtcaggc aactatggat gaacgaaata gacagatcgc 2760
tgagataggt gcctcactga ttaagcattg gtaactgtca gaccaagttt actcatatat 2820
actttagatt gatttaaaac ttcattttta atttaaaagg atctaggtga agatcctttt 2880
tgataatctc atgaccaaaa tcccttaacg tgagttttcg ttccactgag cgtcagaccc 2940
cgtagaaaag atcaaaggat cttcttgaga tccttttttt ctgcgcgtaa tctgctgctt 3000
gcaaacaaaa aaaccaccgc taccagcggt ggtttgtttg ccggatcaag agctaccaac 3060
tctttttccg aaggtaactg gcttcagcag agcgcagata ccaaatactg tccttctagt 3120
gtagccgtag ttaggccacc acttcaagaa ctctgtagca ccgcctacat acctcgctct 3180
gctaatcctg ttaccagtgg ctgctgccag tggcgataag tcgtgtctta ccgggttgga 3240
ctcaagacga tagttaccgg ataaggcgca gcggtcgggc tgaacggggg gttcgtgcac 3300
acagcccagc ttggagcgaa cgacctacac cgaactgaga tacctacagc gtgagctatg 3360
agaaagcgcc acgcttcccg aagggagaaa ggcggacagg tatccggtaa gcggcagggt 3420
cggaacagga gagcgcacga gggagcttcc agggggaaac gcctggtatc tttatagtcc 3480
tgtcgggttt cgccacctct gacttgagcg tcgatttttg tgatgctcgt caggggggcg 3540
gagcctatgg aaaaacgcca gcaacgcggc ctttttacgg ttcctggcct tttgctggcc 3600
ttttgctcac atgttctttc ctgcgttatc ccctgattct gtggataacc gtattaccgc 3660
ctttgagtga gctgataccg ctcgccgcag ccgaacgacc gagcgcagcg agtcagtgag 3720
cgaggaagcg gaagagcgcc tgatgcggta ttttctcctt acgcatctgt gcggtatttc 3780
acaccgcaat ggtgcactct cagtacaatc tgctctgatg ccgcatagtt aagccagtat 3840
acactccgct atcgctacgt gactgggtca tggctgcgcc ccgacacccg ccaacacccg 3900
ctgacgcgcc ctgacgggct tgtctgctcc cggcatccgc ttacagacaa gctgtgaccg 3960
tctccgggag ctgcatgtgt cagaggtttt caccgtcatc accgaaacgc gcgaggcag 4019

<210> 50
<211> 111
<212> PRT
<213> Artificial Sequence

<220>
<223> High affinity variant of ILA TCR alpha chain variable domain

<400> 50

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Met Pro Thr
85 90 95

Gly Lys Tyr Ile Phe Gly Thr Gly Thr Arg Leu Lys Val Leu Ala
100 105 110

<210> 51
<211> 111
<212> PRT
<213> Artificial Sequence

<220>
<223> High affinity variant of ILA TCR alpha chain variable domain

<400> 51

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Arg Leu Pro
85 90 95

Phe Leu Tyr Ile Phe Gly Thr Gly Thr Arg Leu Lys Val Leu Ala
100 105 110

<210> 52

<211> 111
<212> PRT
<213> Artificial Sequence

<220>
<223> High affinity variant of ILA TCR alpha chain variable domain

<400> 52

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Gln Leu Pro
                85                  90                  95

Phe Leu Tyr Ile Phe Gly Thr Gly Thr Arg Leu Lys Val Leu Ala
            100                 105                 110
```

<210> 53
<211> 111
<212> PRT
<213> Artificial Sequence

<220>
<223> High affinity variant of ILA TCR alpha chain variable domain

<400> 53

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30
```

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Met Leu Pro
85 90 95

Tyr Gly Tyr Ile Phe Gly Thr Gly Thr Arg Leu Lys Val Leu Ala
100 105 110

<210> 54
<211> 111
<212> PRT
<213> Artificial Sequence

<220>
<223> High affinity variant of ILA TCR alpha chain variable domain

<400> 54

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Ala Leu Pro
85 90 95

Phe Gly Tyr Ile Phe Gly Thr Gly Thr Arg Leu Lys Val Leu Ala
100 105 110

<210> 55
<211> 111
<212> PRT
<213> Artificial Sequence

<220>
<223> High affinity variant of ILA TCR alpha chain variable domain

<400> 55

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Ala Leu Pro
                85                  90                  95

Tyr Ala Tyr Ile Phe Gly Thr Gly Thr Arg Leu Lys Val Leu Ala
            100                 105                 110
```

<210> 56
<211> 111
<212> PRT
<213> Artificial Sequence

<220>
<223> High affinity variant of ILA TCR alpha chain variable domain

<400> 56

```
Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45
```

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr His Leu Pro
85 90 95

Tyr Gly Tyr Ile Phe Gly Thr Gly Thr Arg Leu Lys Val Leu Ala
100 105 110

<210> 57
<211> 111
<212> PRT
<213> Artificial Sequence

<220>
<223> High affinity variant of ILA TCR alpha chain variable domain

<400> 57

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Thr Pro Pro
85 90 95

Tyr Gly Tyr Ile Phe Gly Thr Gly Thr Arg Leu Lys Val Leu Ala
100 105 110

<210> 58
<211> 111
<212> PRT
<213> Artificial sequence

<220>
<223> High affinity variant of ILA TCR alpha chain variable domain

<400> 58

Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Asp Ser Ala Thr Glu Met Pro
                85                  90                  95

Tyr Gly Tyr Ile Phe Gly Thr Gly Thr Arg Leu Lys Val Leu Ala
            100                 105                 110

<210> 59
<211> 111
<212> PRT
<213> Artificial Sequence

<220>
<223> High affinity variant of ILA TCR alpha chain variable domain

<400> 59

Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Asn Leu Pro
85 90 95

Tyr Gly Tyr Ile Phe Gly Thr Gly Thr Arg Leu Lys Val Leu Ala
100 105 110

<210> 60
<211> 111
<212> PRT
<213> Artificial sequence

<220>
<223> High affinity variant of ILA TCR alpha chain variable domain

<400> 60

Gly Ile Gln Val Glu Gln Ser Pro Pro Asp Leu Ile Leu Gln Glu Gly
1 5 10 15

Ala Asn Ser Thr Leu Arg Cys Asn Phe Ser Asp Ser Val Asn Asn Leu
20 25 30

Gln Trp Phe His Gln Asn Pro Trp Gly Gln Leu Ile Asn Leu Phe Tyr
35 40 45

Ile Pro Ser Gly Thr Lys Gln Asn Gly Arg Leu Ser Ala Thr Thr Val
50 55 60

Ala Thr Glu Arg Tyr Ser Leu Leu Tyr Ile Ser Ser Ser Gln Thr Thr
65 70 75 80

Asp Ser Gly Val Tyr Phe Cys Ala Val Asp Ser Ala Thr Lys Leu Pro
85 90 95

Tyr Gly Tyr Ile Phe Gly Thr Gly Thr Arg Leu Lys Val Leu Ala
100 105 110

<210> 61
<211> 111
<212> PRT
<213> Artificial Sequence

<220>
<223> High affinity variant of ILA TCR beta chain variable domain

<400> 61

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Ile His Pro Glu Tyr Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Gly Ile Gln Pro Gln
                85                  90                  95

Gly Thr Glu Ala Phe Phe Gly Gln Gly Thr Arg Leu Thr Val Val
            100                 105                 110

<210> 62
<211> 111
<212> PRT
<213> Artificial sequence

<220>
<223> High affinity variant of ILA TCR beta chain variable domain

<400> 62

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Ile Cys Pro Ser Cys Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Gly Ile Gln Pro Gln
85 90 95

Gly Thr Glu Ala Phe Phe Gly Gln Gly Thr Arg Leu Thr Val Val
100 105 110

<210> 63
<211> 111
<212> PRT
<213> Artificial Sequence

<220>
<223> High affinity variant of ILA TCR beta chain variable domain

<400> 63

Asn Ala Gly Val Thr Gln Thr Pro Lys Phe Gln Val Leu Lys Thr Gly
1 5 10 15

Gln Ser Met Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Ile His Pro Glu Tyr Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Gly Val Gln Pro Gln
85 90 95

Gly Thr Glu Ala Phe Phe Gly Gln Gly Thr Arg Leu Thr Val Val
100 105 110

<210> 64
<211> 111
<212> PRT
<213> Artificial sequence

<220>
<223> High affinity variant of ILA TCR beta chain variable domain

<400> 64

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Ile Cys Pro Ser Cys Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Gly Val Gln Pro Gln
                85                  90                  95

Gly Thr Glu Ala Phe Phe Gly Gln Gly Thr Arg Leu Thr Val Val
            100                 105                 110

<210> 65
<211> 111
<212> PRT
<213> Artificial Sequence

<220>
<223> High affinity variant of ILA TCR beta chain variable domain

<400> 65

Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Arg Trp Val Gly Asp Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Gly Val Gln Pro Gln
                85                  90                  95

Gly Thr Glu Ala Phe Phe Gly Gln Gly Thr Arg Leu Thr Val Val
100 105 110

<210> 66
<211> 111
<212> PRT
<213> Artificial sequence

<220>
<223> High affinity variant of ILA TCR beta chain variable domain

<400> 66

Asn Ala Gly Val Thr Gln Thr Pro Lys Phe Gln Val Leu Lys Thr Gly
1 5 10 15

Gln Ser Met Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Arg Trp Val Gly Asp Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Gly Ile Gln Pro Gln
85 90 95

Gly Thr Glu Ala Phe Phe Gly Gln Gly Thr Arg Leu Thr Val Val
100 105 110

<210> 67
<211> 111
<212> PRT
<213> Artificial sequence

<220>
<223> High affinity variant of ILA TCR beta chain variable domain

<400> 67

Asn Ala Gly Val Thr Gln Thr Pro Lys Phe Gln Val Leu Lys Thr Gly
1 5 10 15

Gln Ser Met Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Ile Cys Trp Gly Cys Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Gly Ile Gln Pro Gln
85 90 95

Gly Thr Glu Ala Phe Phe Gly Gln Gly Thr Arg Leu Thr Val Val
100 105 110

<210> 68
<211> 111
<212> PRT
<213> Artificial sequence

<220>
<223> High affinity variant of ILA TCR beta chain variable domain

<400> 68

Asn Ala Gly Val Thr Gln Thr Pro Lys Phe Gln Val Leu Lys Thr Gly
1 5 10 15

Gln Ser Met Thr Leu Gln Cys Ala Gln Asp Met Asn His Glu Tyr Met
20 25 30

Ser Trp Tyr Arg Gln Asp Pro Gly Met Gly Leu Arg Leu Ile His Tyr
35 40 45

Ser Ile Trp Glu Phe Glu Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Gly Ile Gln Pro Gln
85 90 95

Gly Thr Glu Ala Phe Phe Gly Gln Gly Thr Arg Leu Thr Val Val
100 105 110

<210> 69

<211> 111
<212> PRT
<213> Artificial sequence

<220>
<223> High affinity variant of ILA TCR beta chain variable domain

<400> 69

```
Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45

Ser Ile Trp Glu Phe Glu Thr Asp Gln Gly Glu Val Pro Asn Gly Tyr
    50                  55                  60

Asn Val Ser Arg Ser Thr Thr Glu Asp Phe Pro Leu Arg Leu Leu Ser
65                  70                  75                  80

Ala Ala Pro Ser Gln Thr Ser Val Tyr Phe Cys Gly Val Gln Pro Gln
                85                  90                  95

Gly Thr Glu Ala Phe Phe Gly Gln Gly Thr Arg Leu Thr Val Val
            100                 105                 110
```

<210> 70
<211> 111
<212> PRT
<213> Artificial sequence

<400> 70

```
Asn Ala Gly Val Thr Gln Thr Pro Lys Phe Gln Val Leu Lys Thr Gly
1               5                   10                  15

Gln Ser Met Thr Leu Gln Cys Ala Gln Asp Met Asn His Glu Tyr Met
            20                  25                  30

Ser Trp Tyr Arg Gln Asp Pro Gly Met Gly Leu Arg Leu Ile His Tyr
        35                  40                  45
```

Ser Ile Cys Trp Gly Cys Thr Asp Gln Gly Glu Val Pro Asn Gly Tyr
50 55 60

Asn Val Ser Arg Ser Thr Thr Glu Asp Phe Pro Leu Arg Leu Leu Ser
65 70 75 80

Ala Ala Pro Ser Gln Thr Ser Val Tyr Phe Cys Gly Val Gln Pro Gln
85 90 95

Gly Thr Glu Ala Phe Phe Gly Gln Gly Thr Arg Leu Thr Val Val
100 105 110

<210> 71
<211> 205
<212> PRT
<213> Artificial sequence

<220>
<223> Soluble Dis-linked high affinity variant of-ILA TCR alpha chain

<400> 71

Met Gly Ile Gln Val Glu Gln Ser Pro Pro Asp Leu Ile Leu Gln Glu
1 5 10 15

Gly Ala Asn Ser Thr Leu Arg Cys Asn Phe Ser Asp Ser Val Asn Asn
20 25 30

Leu Gln Trp Phe His Gln Asn Pro Trp Gly Gln Leu Ile Asn Leu Phe
35 40 45

Tyr Ile Pro Ser Gly Thr Lys Gln Asn Gly Arg Leu Ser Ala Thr Thr
50 55 60

Val Ala Thr Glu Arg Tyr Ser Leu Leu Tyr Ile Ser Ser Ser Gln Thr
65 70 75 80

Thr Asp Ser Gly Val Tyr Phe Cys Ala Val Asp Ser Ala Thr Ala Leu
85 90 95

Pro Tyr Gly Tyr Ile Phe Gly Thr Gly Thr Arg Leu Lys Val Leu Ala
100 105 110

Ile Gln Asn Pro Asp Pro Ala Val Tyr Gln Leu Arg Asp Ser Lys Ser
115 120 125

Ser Asp Lys Ser Val Cys Leu Phe Thr Asp Phe Asp Ser Gln Thr Asn
130 135 140

Val Ser Gln Ser Lys Asp Ser Asp Val Tyr Ile Thr Asp Lys Cys Val
145 150 155 160

Leu Asp Met Arg Ser Met Asp Phe Lys Ser Asn Ser Ala Val Ala Trp
165 170 175

Ser Asn Lys Ser Asp Phe Ala Cys Ala Asn Ala Phe Asn Asn Ser Ile
180 185 190

Ile Pro Glu Asp Thr Phe Phe Pro Ser Pro Glu Ser Ser
195 200 205

<210> 72
<211> 378
<212> PRT
<213> Artificial sequence

<220>
<223> soluble DiS-linked high affinity variant of ILA TCR beta chain li nked to wild-type human IL-2

<400> 72

Met Asn Ala Gly Val Thr Gln Thr Pro Lys Phe Gln Val Leu Lys Thr
1 5 10 15

Gly Gln Ser Met Thr Leu Gln Cys Ala Gln Asp Met Asn His Glu Tyr
20 25 30

Met Ser Trp Tyr Arg Gln Asp Pro Gly Met Gly Leu Arg Leu Ile His
35 40 45

Tyr Ser Val Gly Ala Gly Ile Thr Asp Gln Gly Glu Val Pro Asn Gly
50 55 60

Tyr Asn Val Ser Arg Ser Thr Thr Glu Asp Phe Pro Leu Arg Leu Leu
65 70 75 80

Ser Ala Ala Pro Ser Gln Thr Ser Val Tyr Phe Cys Gly Ile Gln Pro
85 90 95

Tyr Gln Gly Thr Glu Ala Phe Phe Gly Gln Gly Thr Arg Leu Thr Val
100 105 110

Val Glu Asp Leu Lys Asn Val Phe Pro Pro Glu Val Ala Val Phe Glu
115 120 125

Pro Ser Glu Ala Glu Ile Ser His Thr Gln Lys Ala Thr Leu Val Cys

130 135 140

Leu Ala Thr Gly Phe Tyr Pro Asp His Val Glu Leu Ser Trp Trp Val
145 150 155 160

Asn Gly Lys Glu Val His Ser Gly Val Cys Thr Asp Pro Gln Pro Leu
165 170 175

Lys Glu Gln Pro Ala Leu Asn Asp Ser Arg Tyr Ala Leu Ser Ser Arg
180 185 190

Leu Arg Val Ser Ala Thr Phe Trp Gln Asp Pro Arg Asn His Phe Arg
195 200 205

Cys Gln Val Gln Phe Tyr Gly Leu Ser Glu Asn Asp Glu Trp Thr Gln
210 215 220

Asp Arg Ala Lys Pro Val Thr Gln Ile Val Ser Ala Glu Ala Trp Gly
225 230 235 240

Arg Ala Asp Pro Gly Ala Pro Thr Ser Ser Ser Thr Lys Lys Thr Gln
245 250 255

Leu Gln Leu Glu His Leu Leu Leu Asp Leu Gln Met Ile Leu Asn Gly
260 265 270

Ile Asn Asn Tyr Lys Asn Pro Lys Leu Thr Arg Met Leu Thr Phe Lys
275 280 285

Phe Tyr Met Pro Lys Lys Ala Thr Glu Leu Lys His Leu Gln Cys Leu
290 295 300

Glu Glu Glu Leu Lys Pro Leu Glu Glu Val Leu Asn Leu Ala Gln Ser
305 310 315 320

Lys Asn Phe His Leu Arg Pro Arg Asp Leu Ile Ser Asn Ile Asn Val
325 330 335

Ile Val Leu Glu Leu Lys Gly Ser Glu Thr Thr Phe Met Cys Glu Tyr
340 345 350

Ala Asp Glu Thr Ala Thr Ile Val Glu Phe Leu Asn Arg Trp Ile Thr
355 360 365

Phe Cys Gln Ser Ile Ile Ser Thr Leu Thr
370 375

<210> 73
<211> 243
<212> PRT

<213> Artificial Sequence

<220>
<223> soluble Dis-linked high affinity variant of ILA TCR beta chain

<400> 73

```
Met Asn Ala Gly Val Thr Gln Thr Pro Lys Phe Gln Val Leu Lys Thr
1               5                   10                  15

Gly Gln Ser Met Thr Leu Gln Cys Ala Gln Asp Met Asn His Glu Tyr
            20                  25                  30

Met Ser Trp Tyr Arg Gln Asp Pro Gly Met Gly Leu Arg Leu Ile His
        35                  40                  45

Tyr Ser Val Gly Ala Gly Ile Thr Asp Gln Gly Glu Val Pro Asn Gly
    50                  55                  60

Tyr Asn Val Ser Arg Ser Thr Thr Glu Asp Phe Pro Leu Arg Leu Leu
65                  70                  75                  80

Ser Ala Ala Pro Ser Gln Thr Ser Val Tyr Phe Cys Gly Ile Gln Pro
                85                  90                  95

Tyr Gln Gly Thr Glu Ala Phe Phe Gly Gln Gly Thr Arg Leu Thr Val
            100                 105                 110

Val Glu Asp Leu Lys Asn Val Phe Pro Pro Glu Val Ala Val Phe Glu
        115                 120                 125

Pro Ser Glu Ala Glu Ile Ser His Thr Gln Lys Ala Thr Leu Val Cys
    130                 135                 140

Leu Ala Thr Gly Phe Tyr Pro Asp His Val Glu Leu Ser Trp Trp Val
145                 150                 155                 160

Asn Gly Lys Glu Val His Ser Gly Val Cys Thr Asp Pro Gln Pro Leu
                165                 170                 175

Lys Glu Gln Pro Ala Leu Asn Asp Ser Arg Tyr Ala Leu Ser Ser Arg
            180                 185                 190

Leu Arg Val Ser Ala Thr Phe Trp Gln Asp Pro Arg Asn His Phe Arg
        195                 200                 205

Cys Gln Val Gln Phe Tyr Gly Leu Ser Glu Asn Asp Glu Trp Thr Gln
    210                 215                 220

Asp Arg Ala Lys Pro Val Thr Gln Ile Val Ser Ala Glu Ala Trp Gly
```

225 230 235 240

Arg Ala Asp

<210> 74
<211> 243
<212> PRT
<213> Artificial sequence

<220>
<223> soluble Dis-linked high affinity variant of ILA TCR beta chain

<400> 74

Met Asn Ala Gly Val Thr Gln Thr Pro Lys Phe Gln Val Leu Lys Thr
1 5 10 15

Gly Gln Ser Met Thr Leu Gln Cys Ala Gln Asp Met Asn His Glu Tyr
20 25 30

Met Ser Trp Tyr Arg Gln Asp Pro Gly Met Gly Leu Arg Leu Ile His
35 40 45

Tyr Ser Ile Cys Pro Ser Cys Thr Asp Gln Gly Glu Val Pro Asn Gly
50 55 60

Tyr Asn Val Ser Arg Ser Thr Thr Glu Asp Phe Pro Leu Arg Leu Leu
65 70 75 80

Ser Ala Ala Pro Ser Gln Thr Ser Val Tyr Phe Cys Gly Val Gln Pro
85 90 95

Tyr Gln Gly Thr Glu Ala Phe Phe Gly Gln Gly Thr Arg Leu Thr Val
100 105 110

Val Glu Asp Leu Lys Asn Val Phe Pro Pro Glu Val Ala Val Phe Glu
115 120 125

Pro Ser Glu Ala Glu Ile Ser His Thr Gln Lys Ala Thr Leu Val Cys
130 135 140

Leu Ala Thr Gly Phe Tyr Pro Asp His Val Glu Leu Ser Trp Trp Val
145 150 155 160

Asn Gly Lys Glu Val His Ser Gly Val Cys Thr Asp Pro Gln Pro Leu
165 170 175

Lys Glu Gln Pro Ala Leu Asn Asp Ser Arg Tyr Ala Leu Ser Ser Arg

180 185 190

Leu Arg Val Ser Ala Thr Phe Trp Gln Asp Pro Arg Asn His Phe Arg
195 200 205

Cys Gln Val Gln Phe Tyr Gly Leu Ser Glu Asn Asp Glu Trp Thr Gln
210 215 220

Asp Arg Ala Lys Pro Val Thr Gln Ile Val Ser Ala Glu Ala Trp Gly
225 230 235 240

Arg Ala Asp

<210> 75
<211> 243
<212> PRT
<213> Artificial Sequence

<220>
<223> soluble Dis-linked high affinity variant of ILA TCR beta chain

<400> 75

Met Asn Ala Gly Val Thr Gln Thr Pro Lys Phe Gln Val Leu Lys Thr
1 5 10 15

Gly Gln Ser Met Thr Leu Gln Cys Ala Gln Asp Met Asn His Glu Tyr
20 25 30

Met Ser Trp Tyr Arg Gln Asp Pro Gly Met Gly Leu Arg Leu Ile His
35 40 45

Tyr Ser Arg Trp Val Gly Asp Thr Asp Gln Gly Glu Val Pro Asn Gly
50 55 60

Tyr Asn Val Ser Arg Ser Thr Thr Glu Asp Phe Pro Leu Arg Leu Leu
65 70 75 80

Ser Ala Ala Pro Ser Gln Thr Ser Val Tyr Phe Cys Gly Val Gln Pro
85 90 95

Tyr Gln Gly Thr Glu Ala Phe Phe Gly Gln Gly Thr Arg Leu Thr Val
100 105 110

Val Glu Asp Leu Lys Asn Val Phe Pro Pro Glu Val Ala Val Phe Glu
115 120 125

Pro Ser Glu Ala Glu Ile Ser His Thr Gln Lys Ala Thr Leu Val Cys

130 135 140

Leu Ala Thr Gly Phe Tyr Pro Asp His Val Glu Leu Ser Trp Trp Val
145 150 155 160

Asn Gly Lys Glu Val His Ser Gly Val Cys Thr Asp Pro Gln Pro Leu
165 170 175

Lys Glu Gln Pro Ala Leu Asn Asp Ser Arg Tyr Ala Leu Ser Ser Arg
180 185 190

Leu Arg Val Ser Ala Thr Phe Trp Gln Asp Pro Arg Asn His Phe Arg
195 200 205

Cys Gln Val Gln Phe Tyr Gly Leu Ser Glu Asn Asp Glu Trp Thr Gln
210 215 220

Asp Arg Ala Lys Pro Val Thr Gln Ile Val Ser Ala Glu Ala Trp Gly
225 230 235 240

Arg Ala Asp

<210> 76
<211> 243
<212> PRT
<213> Artificial sequence

<220>
<223> soluble Dis-linked high affinity variant of ILA TCR beta chain

<400> 76

Met Asn Ala Gly Val Thr Gln Thr Pro Lys Phe Gln Val Leu Lys Thr
1 5 10 15

Gly Gln Ser Met Thr Leu Gln Cys Ala Gln Asp Met Asn His Glu Tyr
20 25 30

Met Ser Trp Tyr Arg Gln Asp Pro Gly Met Gly Leu Arg Leu Ile His
35 40 45

Tyr Ser Val Gly Ala Gly Ile Thr Asp Gln Gly Glu Val Pro Asn Gly
50 55 60

Tyr Asn Val Ser Arg Ser Thr Thr Glu Asp Phe Pro Leu Arg Leu Leu
65 70 75 80

Ser Ala Ala Pro Ser Gln Thr Ser Val Tyr Phe Cys Gly Ile Gln Pro

85 90 95

Tyr Gln Gly Thr Glu Ala Phe Phe Gly Gln Gly Thr Arg Leu Thr Val
100 105 110

Val Glu Asp Leu Asn Lys Val Phe Pro Pro Glu Val Ala Val Phe Glu
115 120 125

Pro Ser Glu Ala Glu Ile Ser His Thr Gln Lys Ala Thr Leu Val Cys
130 135 140

Leu Ala Thr Gly Phe Phe Pro Asp His Val Glu Leu Ser Trp Trp Val
145 150 155 160

Asn Gly Lys Glu Val His Ser Gly Val Cys Thr Asp Pro Gln Pro Leu
165 170 175

Lys Glu Gln Pro Ala Leu Asn Asp Ser Arg Tyr Ala Leu Ser Ser Arg
180 185 190

Leu Arg Val Ser Ala Thr Phe Trp Gln Asp Pro Arg Asn His Phe Arg
195 200 205

Cys Gln Val Gln Phe Tyr Gly Leu Ser Glu Asn Asp Glu Trp Thr Gln
210 215 220

Asp Arg Ala Lys Pro Val Thr Gln Ile Val Ser Ala Glu Ala Trp Gly
225 230 235 240

Arg Ala Asp

<210> 77
<211> 243
<212> PRT
<213> Artificial sequence

<220>
<223> soluble Dis-linked high affinity variant of ILA TCR beta chain

<400> 77

Met Asn Ala Gly Val Thr Gln Thr Pro Lys Phe Gln Val Leu Lys Thr
1 5 10 15

Gly Gln Ser Met Thr Leu Gln Cys Ala Gln Asp Met Asn His Glu Tyr
20 25 30

Met Ser Trp Tyr Arg Gln Asp Pro Gly Met Gly Leu Arg Leu Ile His

35 40 45

Tyr Ser Ile Cys Pro Ser Cys Thr Asp Gln Gly Glu Val Pro Asn Gly
50 55 60

Tyr Asn Val Ser Arg Ser Thr Thr Glu Asp Phe Pro Leu Arg Leu Leu
65 70 75 80

Ser Ala Ala Pro Ser Gln Thr Ser Val Tyr Phe Cys Gly Val Gln Pro
85 90 95

Tyr Gln Gly Thr Glu Ala Phe Phe Gly Gln Gly Thr Arg Leu Thr Val
100 105 110

Val Glu Asp Leu Asn Lys Val Phe Pro Pro Glu Val Ala Val Phe Glu
115 120 125

Pro Ser Glu Ala Glu Ile Ser His Thr Gln Lys Ala Thr Leu Val Cys
130 135 140

Leu Ala Thr Gly Phe Phe Pro Asp His Val Glu Leu Ser Trp Trp Val
145 150 155 160

Asn Gly Lys Glu Val His Ser Gly Val Cys Thr Asp Pro Gln Pro Leu
165 170 175

Lys Glu Gln Pro Ala Leu Asn Asp Ser Arg Tyr Ala Leu Ser Ser Arg
180 185 190

Leu Arg Val Ser Ala Thr Phe Trp Gln Asp Pro Arg Asn His Phe Arg
195 200 205

Cys Gln Val Gln Phe Tyr Gly Leu Ser Glu Asn Asp Glu Trp Thr Gln
210 215 220

Asp Arg Ala Lys Pro Val Thr Gln Ile Val Ser Ala Glu Ala Trp Gly
225 230 235 240

Arg Ala Asp

<210> 78
<211> 243
<212> PRT
<213> Artificial Sequence

<220>
<223> soluble Dis-linked high affinity variant of ILA TCR beta chain

<400> 78

```
Met Asn Ala Gly Val Thr Gln Thr Pro Lys Phe Gln Val Leu Lys Thr
1               5                   10                  15

Gly Gln Ser Met Thr Leu Gln Cys Ala Gln Asp Met Asn His Glu Tyr
            20                  25                  30

Met Ser Trp Tyr Arg Gln Asp Pro Gly Met Gly Leu Arg Leu Ile His
        35                  40                  45

Tyr Ser Arg Trp Val Gly Asp Thr Asp Gln Gly Glu Val Pro Asn Gly
    50                  55                  60

Tyr Asn Val Ser Arg Ser Thr Thr Glu Asp Phe Pro Leu Arg Leu Leu
65                  70                  75                  80

Ser Ala Ala Pro Ser Gln Thr Ser Val Tyr Phe Cys Gly Val Gln Pro
                85                  90                  95

Tyr Gln Gly Thr Glu Ala Phe Phe Gly Gln Gly Thr Arg Leu Thr Val
            100                 105                 110

Val Glu Asp Leu Asn Lys Val Phe Pro Pro Glu Val Ala Val Phe Glu
        115                 120                 125

Pro Ser Glu Ala Glu Ile Ser His Thr Gln Lys Ala Thr Leu Val Cys
    130                 135                 140

Leu Ala Thr Gly Phe Phe Pro Asp His Val Glu Leu Ser Trp Trp Val
145                 150                 155                 160

Asn Gly Lys Glu Val His Ser Gly Val Cys Thr Asp Pro Gln Pro Leu
                165                 170                 175

Lys Glu Gln Pro Ala Leu Asn Asp Ser Arg Tyr Ala Leu Ser Ser Arg
            180                 185                 190

Leu Arg Val Ser Ala Thr Phe Trp Gln Asp Pro Arg Asn His Phe Arg
        195                 200                 205

Cys Gln Val Gln Phe Tyr Gly Leu Ser Glu Asn Asp Glu Trp Thr Gln
    210                 215                 220

Asp Arg Ala Lys Pro Val Thr Gln Ile Val Ser Ala Glu Ala Trp Gly
225                 230                 235                 240

Arg Ala Asp
```